# EUROPEAN PATENT APPLICATION

(11) **EP 4 537 904 A2**
(43) Date of publication of application: **16.04.2025**
(21) Application number: 24215746.9
(22) Date of filing: 28.05.2019
(51) Int. Cl.: A61P 35/00

(54) **MULTI-SPECIFIC BINDING PROTEINS AND IMPROVEMENTS THEREON**

(30) Priority: 28.05.2018 US 201862677137 P
(62) Divisional of application: 19812582.5
(71) Applicant: Dragonfly Therapeutics, Inc., Waltham, MA 02451 (US)
(72) Inventor: CHANG, Gregory, P., Medford, 02155 (US); CHEUNG, Ann, F., Lincoln, 01773 (US); DU, Jinyan, Waltham, 02452 (US); FALLON, Daniel, Winchester, 01890 (US); GRINBERG, Asya, Lexington, 02421 (US); HANEY, William, Wayland, 01778 (US); O'NEIL, Steven, Wayland, 01778 (US); WEI, Ronnie, Needham, 02494 (US); LUNDE, Bradley, M., Lebanon, 03766 (US); PRINZ, Bianka, Lebanon, 03766 (US)
(74) Representative: Haseltine Lake Kempner LLP

(57) **Abstract**

The invention provides improvements on single-chain variable fragment (scFv) antibodies, multi-specific binding proteins, pharmaceutical compositions comprising such proteins, and therapeutic methods using such proteins and pharmaceutical compositions, including for the treatment of cancer.

## Description

### CROSS-REFERENCE TO RELATED APPLICATIONS

This application claims the benefit of and priority to U.S. Provisional Patent Application No. 62/677,137, filed May 28, 2018, the disclosure of which is hereby incorporated by reference in its entirety for all purposes.

### SEQUENCE LISTING

The instant application contains a Sequence Listing which has been submitted electronically in ASCII format and is hereby incorporated by reference in its entirety. Said ASCII copy, created on May 28, 2019, is named DFY-049WO_SL_ST25.txt and is 340,101 bytes in size.

### FIELD OF THE INVENTION

The invention provides improvements on single-chain variable fragment (scFv) antibodies and a multi-specific binding protein, pharmaceutical compositions comprising such proteins, and therapeutic methods using such proteins and pharmaceutical compositions, including for the treatment of cancer.

### BACKGROUND

Cancer continues to be a significant health problem despite the substantial research efforts and scientific advances reported in the literature for treating this disease. Some of the most frequently diagnosed cancers include prostate cancer, breast cancer, and lung cancer. Prostate cancer is the most common form of cancer in men. Breast cancer remains a leading cause of death in women. Current treatment options for these cancers are not effective for all patients and/or can have substantial adverse side effects. Other types of cancers also remain challenging to treat using existing therapeutic options.

Cancer immunotherapies are desirable because they are highly specific and can facilitate destruction of cancer cells using the patient's own immune system. Fusion proteins such as bi-specific T-cell engagers are cancer immunotherapies described in the literature that bind to tumor cells and T-cells to facilitate destruction of tumor cells. Antibodies that bind to certain tumor-associated antigens and to certain immune cells have been described in the literature. *See, e.g.,* WO 2016/134371 and WO 2015/095412.

Natural killer (NK) cells are a component of the innate immune system and make up approximately 15% of circulating lymphocytes. NK cells infiltrate virtually all tissues and were originally characterized by their ability to kill tumor cells effectively without the need for prior sensitization. Activated NK cells kill target cells by means similar to cytotoxic T cells - *i.e.,* via cytolytic granules that contain perforin and granzymes as well as via death receptor pathways. Activated NK cells also secrete inflammatory cytokines such as IFN-y and chemokines that promote the recruitment of other leukocytes to the target tissue.

NK cells respond to signals through a variety of activating and inhibitory receptors on their surface. For example, when NK cells encounter healthy self-cells, their activity is inhibited through activation of the killer-cell immunoglobulin-like receptors (KIRs). Alternatively, when NK cells encounter foreign cells or cancer cells, they are activated via their activating receptors (e.g., NKG2D, NCRs, DNAM1). NK cells are also activated by the constant region of some immunoglobulins through CD16 receptors on their surface. The overall sensitivity of NK cells to activation depends on the sum of stimulatory and inhibitory signals. NKG2D is a type-II transmembrane protein that is expressed by essentially all natural killer cells where NKG2D serves as an activating receptor. NKG2D is also be found on T cells where it acts as a costimulatory receptor. The ability to modulate NK cell function via NKG2D is useful in various therapeutic contexts including malignancy.

### SUMMARY

In one aspect, the current invention provides an improvement on a single-chain variable fragment (scFv) that is linked to an antibody constant domain via a hinge sequence. In some embodiments, the hinge comprises amino acids Ala-Ser. In some other embodiments, the hinge comprises amino acids Ala-Ser and Thr-Lys-Gly. The scFv may include a heavy chain variable domain and a light chain variable domain. In some embodiments, the scFv binds NKG2D or a tumor-associated antigen. The hinge sequence provides flexibility of binding to the target antigen.

In some embodiments of the scFv, the heavy chain variable domain forms a disulfide bridge with the light chain variable domain. For example, a disulfide bridge can be formed between the C44 residue of the heavy chain variable domain and the C100 residue of the light chain variable domain. In some embodiments, the heavy chain variable domain is linked to the light chain variable domain via a flexible linker, such as (G₄S)₄ (SEQ ID NO:427). In some embodiments of the scFv, the heavy chain variable domain is positioned at the N terminus of the light chain variable domain. In some embodiments of the scFv, the heavy chain variable domain is positioned at the C terminus of the light chain variable domain.

In some embodiments, the antibody constant domain linked to the scFv is able to bind to CD16. In some embodiments, the antibody constant domain comprises a CH2 domain and a CH3 domain of an IgG antibody, for example, a human IgG1 antibody. In some embodiments, mutations are introduced in the antibody constant domain to enable heterodimerization with another antibody constant domain. For example, if the antibody constant domain is derived from the constant domain of a human IgG1, the antibody constant domain can comprise an amino acid sequence at least 90% identical to amino acids 234-332 of a human IgG1 antibody, and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439. In some embodiments, the antibody constant domain can comprise an amino acid sequence at least 90% identical to amino acids 234-332 of a human IgG1 antibody, and differs by one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E.

In another aspect, the current invention provides a protein that contains the scFv linked to an antibody constant region described above. In some embodiments, the protein includes a first antigen-binding site, which comprises the scFv linked to an antibody constant domain; a second antigen-binding site which may take the Fab or the scFv format described here; and a second antibody constant domain linked to the second antigen-binding site. In some embodiments, the protein is multi-specific, wherein the first antigen binding site binds NKG2D, the second antigen binding sites bind a tumor-associated antigen, and the antibody constant regions bind CD16.

In some other embodiments, the protein is multi-specific, wherein the first antigen binding site binds a tumor-associated antigen, the second antigen binding site binds NKG2D, and the antibody constant regions bind CD16. The antibody constant region linked to the scFv can heterodimerize with a second antibody constant region. The multi-specific binding proteins in these embodiments bind to the NKG2D receptor and CD16 receptor on natural killer cells, and to a tumor-associated antigen on cancer cells. Such proteins can engage more than one kind of NK-activating receptor, and may block the binding of natural ligands to NKG2D. In certain embodiments, the proteins can agonize NK cells in humans. In some embodiments, the proteins can agonize NK cells in humans, and/or in other species such as rodents and/or cynomolgus monkeys.

In another aspect, the current invention provides a multi-specific binding protein, and the protein contains a first antigen-binding site that binds a tumor-associated antigen; a second antigen-binding site that binds the same tumor-associated antigen as the first antigen-binding site; a third antigen-binding site that binds NKG2D; and an antibody constant region or a portion thereof sufficient to bind CD16, or a fourth antigen-binding site that binds CD16. Any one of the antigen binding sites can either take the form of an Fab or an scFv, such as those described above. The multi-specific binding protein provided here provides bivalent engagement of tumor-associated antigen, thereby stabilizing the tumor-associated antigen on cancer cell surface, and enhance cytotoxicity towards the cancer cells by NK cells.

In some embodiments, bivalent engagement of tumor-associated antigens by the multi-specific binding proteins confer stronger binding of the multi-specific binding proteins to the cancer cells, thereby facilitating stronger cytotoxic response of NK cells towards the cancer cells, especially towards cancer cells expressing a low level of tumor-associated antigen.

In some embodiments, the multi-specific binding proteins incorporate a portion of an antibody Fc domain sufficient to bind CD16, wherein the antibody Fc domain comprises a hinge and a CH2 domain and/or amino acid sequences at least 90% identical to amino acid sequence 234-332 of a human IgG antibody. Mutations can be introduced into the antibody constant domain to enable heterodimerization with another antibody constant domain. For example, if the antibody constant domain is derived from the constant domain of a human IgG1, the antibody constant domain can comprise an amino acid sequence at least 90% identical to amino acids 234-332 of a human IgG1 antibody, and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.

In some embodiments, the antibody constant domain can comprise an amino acid sequence at least 90% identical to amino acids 234-332 of a human IgG1 antibody, and differs by one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E.

The tumor-associated antigen binding site described above can be a site that bind to any tumor-associated antigen, for example, ANO1, BCMA, EpCAM, CAIX, CEA, CCR4, CD2, CD123, CD133, CD19, CD20, CD22, CD25, CD30, CD33, CD37, CD38, CD40, CD52, CD70, CLAUDIN-18.2, DLL3, EGFR/ERBB1, GD2, IGF1R, HER2, HER3/ERBB3, HER4/ERBB4, MUC1, cMET, SLAMF7, PSMA, mesothelin, MICA, MICB, TRAILR1, TRAILR2, TROP2, MAGE-A3, B7.1, B7.2, CTLA4, PD1, 5T4, GPNMB, FR-alpha, PAPP-A, FLT3, GPC3, CXCR4, ROR1, ROR2, HLA-E, PD-L1, VLA4, CD44, CD13, CD15, CD47, CLL1, CD81, CD23, CD79a, CD79b, CD80, CRLF2, SLAMF7, CD138, CA125, NaPi2b, Nectin4, ADAM8, ADAM9, SLC44A4, CA19-9, LILRBl, LILRB2, LILRB3, LILRB4, LILRB5, ULRA 1, LILRA2, LILRA3, ULRA4, LILRA5, and ULRA6, CCR8, CD7, CTLA4, CX3CR1, ENTPD1, HAVCR2, IL-1R2, PDCD1LG2, TIGIT, TNFRSF4, TNFRSF8, TNFRSF9, GEM, NT5E, TNFRSF18, MUC1, P-cadherin, Plexin-A1, TNFRSF10B, STEAP1, CDCP1, PTK7, Axl, erbB-3, EDNRB, Tyrp1, CD14, CD163, CSF3R, Siglec-9, ITGAM, VISTA, B7-H4 (VTCN1), CCR1, LRRC25, PTAFR, SIRPB1, TLR2, TLR4, CD300LB, ATP1A3, CCR5, MUC1 (or MUC1-C), Plexin-A1, TNFRSF10B, STEAP1, CDCP1, PTK7, AXL, EDNRB, OLR1, and TYRP1.

In another aspect, the present invention provides a protein comprising: (a) an antigen-binding site that binds NKG2D; (b) an antigen-binding T cell receptor (TCR) fragment; and (c) an antibody constant region or a portion thereof sufficient to bind CD16, or an additional antigen-binding site that binds CD16. In certain embodiments, the protein is a multi-specific binding protein comprising an antigen-binding TCR fragment that binds a tumor-associated antigen (TAA).

In certain embodiments, the antigen-binding site is an Fab fragment, and the antigen-binding TCR fragment is a single-chain TCR (scTCR) fragment. In certain embodiments, the scTCR fragment is linked to a polypeptide chain of the antibody constant region via a hinge comprising Ala-Ser. In certain embodiments, the hinge further comprises amino acid sequence Thr-Lys-Gly.

In certain embodiments, the antigen-binding site is an scFv, and the antigen-binding TCR fragment is an extracellular TCR fragment. In certain embodiments, the scFv is linked to a polypeptide chain of the antibody constant region via a hinge comprising Ala-Ser. In certain embodiments, the hinge further comprises amino acid sequence Thr-Lys-Gly.

In certain embodiments, the antigen-binding site is an Fab fragment, and the antigen-binding TCR fragment is an extracellular TCR fragment.

In certain embodiments, the multi-specific binding protein further comprises an additional antigen-binding TCR fragment that binds the same antigen as the antigen-binding TCR fragment. In certain embodiments, the antigen-binding site is an scFv, and the antigen-binding TCR fragment and the additional antigen-binding TCR fragment are extracellular TCR fragments. In certain embodiments, the antigen-binding site is an scFv, and the antigen-binding TCR fragment and the additional antigen-binding TCR fragment are scTCR fragments.

In certain embodiments of the multi-specific binding protein that contains an scFv, the scFv comprises a heavy chain variable domain linked to a light chain variable domain via a flexible linker. In certain embodiments, the flexible linker comprises (G₄S)₄. In certain embodiments, the scFv comprises a heavy chain variable domain positioned at the N-terminus or the C-terminus of a light chain variable domain.

In certain embodiments, the antigen-binding site comprises a heavy chain variable domain and a light chain variable domain, and wherein the heavy chain variable domain forms a disulfide bridge with the light chain variable domain. In certain embodiments, the disulfide bridge is formed between Cys at position 44 in the heavy chain variable domain and Cys at position 100 in the light chain variable domain, the positions defined under the Kabat numbering. In certain embodiments, the antigen-binding site that contains such a disulfide brideg is an scFv.

In certain embodiments, the antigen-binding site binds to NKG2D in humans.

In certain embodiments, the antigen-binding TCR fragment binds a peptide from a tumor-associated antigen presented by a major histocompatibility complex (MHC).

In certain embodiments, the antigen-binding TCR fragment binds an ELAVL4 peptide having the amino acid sequence of SEQ ID NO:425 presented by HLA-A*02:01:48. In certain embodiments, the antigen-binding TCR fragment comprises an alpha chain variable domain related to SEQ ID NO:351 and a beta chain variable domain related to SEQ ID NO:352. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:351 and/or incorporate an amino acid sequence identical to the CDR3α sequence (SEQ ID NO:353) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:352 and/or incorporate an amino acid sequence identical to the CDR3β sequence (SEQ ID NO:354) of the beta chain variable domain. In certain embodiments, the antigen-binding TCR fragment comprises an alpha chain amino acid sequence set forth in SEQ ID NO:349 and a beta chain amino acid sequence set forth in SEQ ID NO:350.

In certain embodiments, the antigen-binding TCR fragment binds an Insulin peptide having the amino acid sequence of SEQ ID NO:426 presented by HLA-A*02:01:48. In certain embodiments, the antigen-binding TCR fragment comprises an alpha chain variable domain related to SEQ ID NO:357 and a beta chain variable domain related to SEQ ID NO:358. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:357 and/or incorporate an amino acid sequence identical to the CDR3α sequence (SEQ ID NO:359) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:358 and/or incorporate an amino acid sequence identical to the CDR3β sequence (SEQ ID NO:360) of the beta chain variable domain. In certain embodiments, the antigen-binding TCR fragment comprises an alpha chain amino acid sequence set forth in SEQ ID NO:355 and a beta chain amino acid sequence set forth in SEQ ID NO:356.

In certain embodiments, the antigen-binding TCR fragment binds a TERT peptide having the amino acid sequence of SEQ ID NO:340 presented by HLA-A*02:01:48. In certain embodiments, the antigen-binding TCR fragment comprises an alpha chain variable domain related to SEQ ID NO:363 and a beta chain variable domain related to SEQ ID NO:364. For example, in certain embodiments, the antigen-binding TCR fragment comprises an alpha chain variable domain at least 90% *(e.g.,* 90%, 91%, 92%, 93%, 94%, 95%, 96%, 97%, 98%, 99%, or 100%) identical to SEQ ID NO:363 and/or incorporate an amino acid sequence identical to the CDR3α sequence (SEQ ID NO:365) of the alpha chain variable domain. Similarly, in certain embodiments, the antigen-binding TCR fragment comprises a beta chain variable domain at least 90% *(e.g.,* 90%, 91%, 92%, 93%, 94%, 95%, 96%, 97%, 98%, 99%, or 100%) identical to SEQ ID NO:364 and/or incorporate an amino acid sequence identical to the CDR3β sequence (SEQ ID NO:366) of the beta chain variable domain. In certain embodiments, the antigen-binding TCR fragment comprises an alpha chain amino acid sequence set forth in SEQ ID NO:361 and a beta chain amino acid sequence set forth in SEQ ID NO:362.

In certain embodiments, the antigen-binding TCR fragment binds an ERBB2 peptide having the amino acid sequence of SEQ ID NO:341 presented by HLA-A*02. In certain embodiments, the antigen-binding TCR fragment comprises an alpha chain variable domain related to SEQ ID NO:430 and a beta chain variable domain related to SEQ ID NO:431. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:430 and/or incorporate an amino acid sequence identical to the CDR3α sequence (SEQ ID NO:367) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:431 and/or incorporate an amino acid sequence identical to the CDR3β sequence (SEQ ID NO:368) of the beta chain variable domain. In certain embodiments, the antigen-binding TCR fragment comprises an alpha chain amino acid sequence set forth in SEQ ID NO:428 and a beta chain amino acid sequence set forth in SEQ ID NO:429.

In certain embodiments, the antigen-binding TCR fragment binds a WT1 peptide having the amino acid sequence of SEQ ID NO:342 presented by HLA-A*02. In certain embodiments, the antigen-binding TCR fragment comprises an alpha chain variable domain related to SEQ ID NO:434 and a beta chain variable domain related to SEQ ID NO:435. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:434 and/or incorporate an amino acid sequence identical to the CDR3α sequence (SEQ ID NO:369) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:435 and/or incorporate an amino acid sequence identical to the CDR3β sequence (SEQ ID NO:370) of the beta chain variable domain. In certain embodiments, the antigen-binding TCR fragment comprises an alpha chain amino acid sequence set forth in SEQ ID NO:432 and a beta chain amino acid sequence set forth in SEQ ID NO:433.

In certain embodiments, the antigen-binding TCR fragment binds a WT1 peptide having the amino acid sequence of SEQ ID NO:342 presented by HLA-A*02. In certain embodiments, the antigen-binding TCR fragment comprises an alpha chain variable domain related to SEQ ID NO:438 and a beta chain variable domain related to SEQ ID NO:439. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:438 and/or incorporate an amino acid sequence identical to the CDR3α sequence (SEQ ID NO:371) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:439 and/or incorporate an amino acid sequence identical to the CDR3β sequence (SEQ ID NO:372) of the beta chain variable domain. In certain embodiments, the antigen-binding TCR fragment comprises an alpha chain amino acid sequence set forth in SEQ ID NO:436 and a beta chain amino acid sequence set forth in SEQ ID NO:437.

In certain embodiments, the antigen-binding TCR fragment binds a MAGE-A3 peptide having the amino acid sequence of SEQ ID NO:343 presented by HLA-A1. In certain embodiments, the antigen-binding TCR fragment comprises an alpha chain variable domain related to SEQ ID NO:375 and a beta chain variable domain related to SEQ ID NO:376. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:375 and/or incorporate an amino acid sequence identical to the CDR3α sequence (SEQ ID NO:377) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:376 and/or incorporate an amino acid sequence identical to the CDR3β sequence (SEQ ID NO:378) of the beta chain variable domain. In certain embodiments, the antigen-binding TCR fragment comprises an alpha chain amino acid sequence set forth in SEQ ID NO:373 and a beta chain amino acid sequence set forth in SEQ ID NO:374.

In certain embodiments, the antigen-binding TCR fragment binds a MART1 peptide having the amino acid sequence of SEQ ID NO:344 presented by HLA-A2. In certain embodiments, the antigen-binding TCR fragment comprises an alpha chain variable domain related to SEQ ID NO:381 and a beta chain variable domain related to SEQ ID NO:382. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:381 and/or incorporate an amino acid sequence identical to the CDR3α sequence (SEQ ID NO:383) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:382 and/or incorporate an amino acid sequence identical to the CDR3β sequence (SEQ ID NO:384) of the beta chain variable domain. In certain embodiments, the antigen-binding TCR fragment comprises an alpha chain amino acid sequence set forth in SEQ ID NO:379 and a beta chain amino acid sequence set forth in SEQ ID NO:380.

In certain embodiments, the antigen-binding TCR fragment binds a BIRC5 peptide having the amino acid sequence of SEQ ID NO:346 presented by HLA-A2. In certain embodiments, the antigen-binding TCR fragment comprises an alpha chain variable domain related to SEQ ID NO:442 and a beta chain variable domain related to SEQ ID NO:443. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:442 and/or incorporate an amino acid sequence identical to the CDR3α sequence (SEQ ID NO:389) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:443 and/or incorporate an amino acid sequence identical to the CDR3β sequence (SEQ ID NO:390) of the beta chain variable domain. In certain embodiments, the antigen-binding TCR fragment comprises an alpha chain amino acid sequence set forth in SEQ ID NO:440 and a beta chain amino acid sequence set forth in SEQ ID NO:441.

In certain embodiments, the antigen-binding TCR fragment binds a BIRC5 peptide having the amino acid sequence of SEQ ID NO:346 presented by HLA-A2. In certain embodiments, the antigen-binding TCR fragment comprises an alpha chain variable domain related to SEQ ID NO:444 and a beta chain variable domain related to SEQ ID NO:445. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:444 and/or incorporate an amino acid sequence identical to the CDR3α sequence (SEQ ID NO:391) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:445 and/or incorporate an amino acid sequence identical to the CDR3β sequence (SEQ ID NO:392) of the beta chain variable domain. In certain embodiments, the antigen-binding TCR fragment comprises an alpha chain amino acid sequence set forth in SEQ ID NO:385 and a beta chain amino acid sequence set forth in SEQ ID NO:386.

In certain embodiments, the antigen-binding TCR fragment binds a PRAME peptide having the amino acid sequence of SEQ ID NO:347 presented by HLA-A2. In certain embodiments, the antigen-binding TCR fragment comprises an alpha chain variable domain related to SEQ ID NO:395 and a beta chain variable domain related to SEQ ID NO:396. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:395 and/or incorporate an amino acid sequence identical to the CDR3α sequence (SEQ ID NO:397) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:396 and/or incorporate an amino acid sequence identical to the CDR3β sequence (SEQ ID NO:398) of the beta chain variable domain. In certain embodiments, the antigen-binding TCR fragment comprises an alpha chain amino acid sequence set forth in SEQ ID NO:393 and a beta chain amino acid sequence set forth in SEQ ID NO:394.

In certain embodiments, the antigen-binding TCR fragment binds a PRAME peptide having the amino acid sequence of SEQ ID NO:347 presented by HLA-A2. In certain embodiments, the antigen-binding TCR fragment comprises an alpha chain variable domain related to SEQ ID NO:401 and a beta chain variable domain related to SEQ ID NO:402. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:401 and/or incorporate an amino acid sequence identical to the CDR3α sequence (SEQ ID NO:403) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:402 and/or incorporate an amino acid sequence identical to the CDR3β sequence (SEQ ID NO:404) of the beta chain variable domain. In certain embodiments, the antigen-binding TCR fragment comprises an alpha chain amino acid sequence set forth in SEQ ID NO:399 and a beta chain amino acid sequence set forth in SEQ ID NO:400.

In certain embodiments, the antigen-binding TCR fragment binds a PRAME peptide having the amino acid sequence of SEQ ID NO:347 presented by HLA-A2. In certain embodiments, the antigen-binding TCR fragment comprises an alpha chain variable domain related to SEQ ID NO:407 and a beta chain variable domain related to SEQ ID NO:408. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:407 and/or incorporate an amino acid sequence identical to the CDR3α sequence (SEQ ID NO:409) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:408 and/or incorporate an amino acid sequence identical to the CDR3β sequence (SEQ ID NO:410) of the beta chain variable domain. In certain embodiments, the antigen-binding TCR fragment comprises an alpha chain amino acid sequence set forth in SEQ ID NO:405 and a beta chain amino acid sequence set forth in SEQ ID NO:406.

In certain embodiments, the antigen-binding TCR fragment binds an NY-ESO-1 peptide having the amino acid sequence of SEQ ID NO:348 presented by HLA-A2. In certain embodiments, the antigen-binding TCR fragment comprises an alpha chain variable domain related to SEQ ID NO:413 and a beta chain variable domain related to SEQ ID NO:414. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:413 and/or incorporate an amino acid sequence identical to the CDR3α sequence (SEQ ID NO:415) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:414 and/or incorporate an amino acid sequence identical to the CDR3β sequence (SEQ ID NO:416) of the beta chain variable domain. In certain embodiments, the antigen-binding TCR fragment comprises an alpha chain amino acid sequence set forth in SEQ ID NO:411 and a beta chain amino acid sequence set forth in SEQ ID NO:412.

In certain embodiments, the antigen-binding TCR fragment binds an NY-ESO-1 peptide having the amino acid sequence of SEQ ID NO:348 presented by HLA-A2. In certain embodiments, the antigen-binding TCR fragment comprises an alpha chain variable domain related to SEQ ID NO:418 and a beta chain variable domain related to SEQ ID NO:414. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:418 and/or incorporate an amino acid sequence identical to the CDR3α sequence (SEQ ID NO:415) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:414 and/or incorporate an amino acid sequence identical to the CDR3β sequence (SEQ ID NO:416) of the beta chain variable domain. In certain embodiments, the antigen-binding TCR fragment comprises an alpha chain amino acid sequence set forth in SEQ ID NO:417 and a beta chain amino acid sequence set forth in SEQ ID NO:412.

In certain embodiments, the antigen-binding TCR fragment binds an NY-ESO-1 peptide having the amino acid sequence of SEQ ID NO:348 presented by HLA-A2. In certain embodiments, the antigen-binding TCR fragment comprises an alpha chain variable domain related to SEQ ID NO:421 and a beta chain variable domain related to SEQ ID NO:422. For example, in certain embodiments, the antigen-binding TCR fragment comprises an alpha chain variable domain at least 90% (*e.g.,* 90%, 91%, 92%, 93%, 94%, 95%, 96%, 97%, 98%, 99%, or 100%) identical to SEQ ID NO:421 and/or incorporate an amino acid sequence identical to the CDR3α sequence (SEQ ID NO:423) of the alpha chain variable domain. Similarly, in certain embodiments, the antigen-binding TCR fragment comprises a beta chain variable domain at least 90% (*e.g.,* 90%, 91%, 92%, 93%, 94%, 95%, 96%, 97%, 98%, 99%, or 100%) identical to SEQ ID NO:422 and/or incorporate an amino acid sequence identical to the CDR3β sequence (SEQ ID NO:424) of the beta chain variable domain. In certain embodiments, the antigen-binding TCR fragment comprises an alpha chain amino acid sequence set forth in SEQ ID NO:419 and a beta chain amino acid sequence set forth in SEQ ID NO:420.

In certain embodiments, the antigen-binding TCR fragment binds an SSX2 peptide having the amino acid sequence of SEQ ID NO:345 presented by HLA-A2. In certain embodiments, the antigen-binding TCR fragment comprises an alpha chain variable domain incorporating a CDR3α sequence set forth in SEQ ID NO:387. In certain embodiments, the antigen-binding TCR fragment comprises a beta chain variable domain incorporating a CDR3β sequence set forth in SEQ ID NO:388.

In certain embodiments, the multi-specific binding protein comprises an antibody constant region or a portion thereof sufficient to bind CD16, wherein the antibody constant region or the portion thereof sufficient to bind CD16 comprises hinge and CH2 domain of a human IgG1 antibody. In certain embodiments, the antibody constant region or the portion thereof sufficient to bind CD16 comprises an amino acid sequence at least 90% identical to amino acids 234-332 of a human IgG1 antibody. Mutations can be introduced into the antibody constant domain to enable heterodimerization with another antibody constant domain. For example, if the antibody constant domain is derived from the constant domain of a human IgG1, the antibody constant domain can comprise an amino acid sequence at least 90% identical to amino acids 234-332 of a human IgG1 antibody, and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439. In some embodiments, the antibody constant domain can comprise an amino acid sequence at least 90% identical to amino acids 234-332 of a human IgG1 antibody, and differs by one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E.

In certain embodiments of any one of the foregoing polypeptides or proteins that contain an antigen-binding site that binds NKG2D (also called "NKG2D binding site"), the NKG2D binding site can incorporate a heavy chain variable domain related to SEQ ID NO:1, such as by having an amino acid sequence at least 90% (*e.g.,* 90%, 91%, 92%, 93%, 94%, 95%, 96%, 97%, 98%, 99%, or 100%) identical to SEQ ID NO:1, and/or incorporating amino acid sequences identical to the CDR1 (SEQ ID NO:3 or SEQ ID NO:307), CDR2 (SEQ ID NO:4), and CDR3 (SEQ ID NO:5 or SEQ ID NO:308) sequences of SEQ ID NO:1. The heavy chain variable domain related to SEQ ID NO:1 can be coupled with a variety of light chain variable domains to form a NKG2D binding site. For example, the NKG2D binding site that incorporates a heavy chain variable domain related to SEQ ID NO:1 can further incorporate a light chain variable domain selected from any one of the sequences related to SEQ ID NOs:2, 7, 9, 11, 13, 15, 17, 19, 21, 23, 25, 27, 29, 31, 33, 35, 37, 39, 41, and 43. For example, the NKG2D binding site incorporates a heavy chain variable domain with amino acid sequences at least 90% (*e.g.,* 90%, 91%, 92%, 93%, 94%, 95%, 96%, 97%, 98%, 99%, or 100%) identical to SEQ ID NO:1 and a light chain variable domain with amino acid sequences at least 90% (*e.g.,* 90%, 91%, 92%, 93%, 94%, 95%, 96%, 97%, 98%, 99%, or 100%) identical to any one of the sequences selected from SEQ ID NOs: 2, 7, 9, 11, 13, 15, 17, 19, 21, 23, 25, 27, 29, 31, 33, 35, 37, 39, 41, and 43.

Alternatively, the NKG2D binding site can incorporate a heavy chain variable domain related to SEQ ID NO:44 and a light chain variable domain related to SEQ ID NO:48. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:44, and/or incorporate amino acid sequences identical to the CDR1 (SEQ ID NO:45 or SEQ ID NO:309), CDR2 (SEQ ID NO:46), and CDR3 (SEQ ID NO:47 or SEQ ID NO:310) sequences of SEQ ID NO:44. Similarly, the light chain variable domain of the second antigen-binding site can be at least 90% (*e.g.,* 90%, 91%, 92%, 93%, 94%, 95%, 96%, 97%, 98%, 99%, or 100%) identical to SEQ ID NO:48, and/or incorporate amino acid sequences identical to the CDR1 (SEQ ID NO:49), CDR2 (SEQ ID NO:50), and CDR3 (SEQ ID NO:51) sequences of SEQ ID NO:48.

In other embodiments, the NKG2D binding site can incorporate a heavy chain variable domain related to SEQ ID NO:52 and a light chain variable domain related to SEQ ID NO:56. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:52, and/or incorporate amino acid sequences identical to the CDR1 (SEQ ID NO:53 or SEQ ID NO:311), CDR2 (SEQ ID NO:54), and CDR3 (SEQ ID NO:55 or SEQ ID NO:312) sequences of SEQ ID NO:52. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:56, and/or incorporate amino acid sequences identical to the CDR1 (SEQ ID NO:57), CDR2 (SEQ ID NO:58), and CDR3 (SEQ ID NO:59) sequences of SEQ ID NO:56.

Alternatively, the NKG2D binding site can incorporate a heavy chain variable domain related to SEQ ID NO:60 and a light chain variable domain related to SEQ ID NO:61, such as by having amino acid sequences at least 90% (*e.g.,* 90%, 91%, 92%, 93%, 94%, 95%, 96%, 97%, 98%, 99%, or 100%) identical to SEQ ID NO:60 and at least 90% (*e.g.,* 90%, 91%, 92%, 93%, 94%, 95%, 96%, 97%, 98%, 99%, or 100%) identical to SEQ ID NO:61 respectively.

In another embodiment, the NKG2D binding site can incorporate a heavy chain variable domain related to SEQ ID NO:62 and a light chain variable domain related to SEQ ID NO:66, For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:62, and/or incorporate amino acid sequences identical to the CDR1 (SEQ ID NO:63), CDR2 (SEQ ID NO:64), and CDR3 (SEQ ID NO:65) sequences of SEQ ID NO:62. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:66, and/or incorporate amino acid sequences identical to the CDR1 (SEQ ID NO:67), CDR2 (SEQ ID NO:68), and CDR3 (SEQ ID NO:69) sequences of SEQ ID NO:66.

The NKG2D binding site, in some embodiments, can incorporate a heavy chain variable domain related to SEQ ID NO:70 and a light chain variable domain related to SEQ ID NO:74. For example, the heavy chain variable domain of the NKG2D binding site can be at least 90% *(e.g.,* 90%, 91%, 92%, 93%, 94%, 95%, 96%, 97%, 98%, 99%, or 100%) identical to SEQ ID NO:70, and/or incorporate amino acid sequences identical to the CDR1 (SEQ ID NO:71 or SEQ ID NO:313), CDR2 (SEQ ID NO:72), and CDR3 (SEQ ID NO:73 or SEQ ID NO:314) sequences of SEQ ID NO:70. Similarly, the light chain variable domain of the NKG2D binding site can be at least 90% *(e.g.,* 90%, 91%, 92%, 93%, 94%, 95%, 96%, 97%, 98%, 99%, or 100%) identical to SEQ ID NO:74, and/or incorporate amino acid sequences identical to the CDR1 (SEQ ID NO:75), CDR2 (SEQ ID NO:76), and CDR3 (SEQ ID NO:77) sequences of SEQ ID NO:74.

In some embodiments the NKG2D binding site can incorporate a heavy chain variable domain related to SEQ ID NO:78 and a light chain variable domain related to SEQ ID NO:82. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:78, and/or incorporate amino acid sequences identical to the CDR1 (SEQ ID NO:79 or SEQ ID NO:315), CDR2 (SEQ ID NO:80), and CDR3 (SEQ ID NO:81 or SEQ ID NO:316) sequences of SEQ ID NO:78. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:82, and/or incorporate amino acid sequences identical to the CDR1 (SEQ ID NO:83), CDR2 (SEQ ID NO:84), and CDR3 (SEQ ID NO:85) sequences of SEQ ID NO:82.

In some embodiments, the NKG2D binding site can incorporate a heavy chain variable domain related to SEQ ID NO:86 and a light chain variable domain related to SEQ ID NO:90. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:86, and/or incorporate amino acid sequences identical to the CDR1 (SEQ ID NO:87 or SEQ ID NO:317), CDR2 (SEQ ID NO:88), and CDR3 (SEQ ID NO:89 or SEQ ID NO:318) sequences of SEQ ID NO:86. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:90, and/or incorporate amino acid sequences identical to the CDR1 (SEQ ID NO:91), CDR2 (SEQ ID NO:92), and CDR3 (SEQ ID NO:93) sequences of SEQ ID NO:90.

In some embodiments, the NKG2D binding site can incorporate a heavy chain variable domain related to SEQ ID NO:94 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:94, and/or incorporate amino acid sequences identical to the CDR1 (SEQ ID NO:95 or SEQ ID NO:319), CDR2 (SEQ ID NO:96), and CDR3 (SEQ ID NO:97 or SEQ ID NO:320) sequences of SEQ ID NO:94. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO: 102 and a light chain variable domain related to SEQ ID NO:106. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:102, and/or incorporate amino acid sequences identical to the CDR1 (SEQ ID NO:103 or SEQ ID NO:313), CDR2 (SEQ ID NO:104), and CDR3 (SEQ ID NO:105 or SEQ ID NO:321) sequences of SEQ ID NO:102. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:106, and/or incorporate amino acid sequences identical to the CDR1 (SEQ ID NO:107), CDR2 (SEQ ID NO:108), and CDR3 (SEQ ID NO:109) sequences of SEQ ID NO:106.

In some embodiments, the NKG2D binding site can incorporate a heavy chain variable domain related to SEQ ID NO:322 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:322, and/or incorporate amino acid sequences identical to the CDR1 (SEQ ID NO:95 or SEQ ID NO:319), CDR2 (SEQ ID NO:96), and CDR3 (SEQ ID NO:323 or SEQ ID NO:324) sequences of SEQ ID NO:322. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:325 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:325, and/or incorporate amino acid sequences identical to the CDR1 (SEQ ID NO:95 or SEQ ID NO:319), CDR2 (SEQ ID NO:96), and CDR3 (SEQ ID NO:326 or SEQ ID NO:327) sequences of SEQ ID NO:325. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:328 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:328, and/or incorporate amino acid sequences identical to the CDR1 (SEQ ID NO:95 or SEQ ID NO:319), CDR2 (SEQ ID NO:96), and CDR3 (SEQ ID NO:329 or SEQ ID NO:330) sequences of SEQ ID NO:328. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:331 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:331, and/or incorporate amino acid sequences identical to the CDR1 (SEQ ID NO:95 or SEQ ID NO:319), CDR2 (SEQ ID NO:96), and CDR3 (SEQ ID NO:332 or SEQ ID NO:333) sequences of SEQ ID NO:331. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:334 and a light chain variable domain related to SEQ ID NO:98. For example, the heavy chain variable domain of the NKG2D binding site can be at least 90% (*e.g*., 90%, 91%, 92%, 93%, 94%, 95%, 96%, 97%, 98%, 99%, or 100%) identical to SEQ ID NO:334, and/or incorporate amino acid sequences identical to the CDR1 (SEQ ID NO:95 or SEQ ID NO:319), CDR2 (SEQ ID NO:96), and CDR3 (SEQ ID NO:335 or SEQ ID NO:336) sequences of SEQ ID NO:334. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:337 and a light chain variable domain related to SEQ ID NO:98. For example, the heavy chain variable domain of the NKG2D binding site can be at least 90% (*e.g.,* 90%, 91%, 92%, 93%, 94%, 95%, 96%, 97%, 98%, 99%, or 100%) identical to SEQ ID NO:337, and/or incorporate amino acid sequences identical to the CDR1 (SEQ ID NO:95 or SEQ ID NO:319), CDR2 (SEQ ID NO:96), and CDR3 (SEQ ID NO:338 or SEQ ID NO:339) sequences of SEQ ID NO:337. Similarly, the light chain variable domain of the NKG2D binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO:99), CDR2 (SEQ ID NO:100), and CDR3 (SEQ ID NO:101) sequences of SEQ ID NO:98.

In some embodiments, the NKG2D binding site can incorporate a heavy chain variable domain related to SEQ ID NO:110 and a light chain variable domain related to SEQ ID NO:111, such as by having amino acid sequences at least 90% (*e.g.,* 90%, 91%, 92%, 93%, 94%, 95%, 96%, 97%, 98%, 99%, or 100%) identical to SEQ ID NO:110 and at least 90% (*e.g.,* 90%, 91%, 92%, 93%, 94%, 95%, 96%, 97%, 98%, 99%, or 100%) identical to SEQ ID NO:111 respectively. In some embodiments, the NKG2D binding site can incorporate a heavy chain variable domain related to SEQ ID NO:112 and a light chain variable domain related to SEQ ID NO:113, such as by having amino acid sequences at least *90%* (*e.g.,* 90%, 91%, 92%, 93%, 94%, 95%, 96%, 97%, 98%, 99%, or 100%) identical to SEQ ID NO:112 and at least 90% (*e.g.,* 90%, 91%, 92%, 93%, 94%, 95%, 96%, 97%, 98%, 99%, or 100%) identical to SEQ ID NO:113 respectively.

Formulations containing any one of the proteins described herein; cells containing one or more nucleic acids expressing the proteins, and methods of enhancing tumor cell death using the proteins are also provided.

Another aspect of the invention provides a method of treating cancer in a patient. The method comprises administering to a patient in need thereof a therapeutically effective amount of the multi-specific binding proteins described herein. Cancers to be treated may include acute myeloid leukemia, acute myelomonocytic leukemia, B cell lymphoma, bladder cancer, breast cancer, colorectal cancer, diffuse large B cell lymphoma esophageal cancer, Ewing's sarcoma, follicular lymphoma, gastric cancer, gastrointestinal cancer, gastrointestinal stromal tumors, glioblastoma, head and neck cancer, melanoma, mesothelioma, multiple myeloma, myelodysplastic syndrome, renal cell carcinoma, neuroblastoma, non-small cell lung cancer, neuroendocrine tumors, ovarian cancer, and pancreatic cancer, prostate cancer, sarcomas, small cell lung cancer, T cell lymphoma, testis cancer, thymic carcinoma, thyroid cancer, urothelial cancer, cancers infiltrated by myeloid-derived suppressor cells, cancers infiltrated by T regulatory cells, cancers with extracellular matrix deposition, cancers with high levels of reactive stroma, and cancers with neoangiogenesis.

### BRIEF DESCRIPTION OF THE DRAWINGS

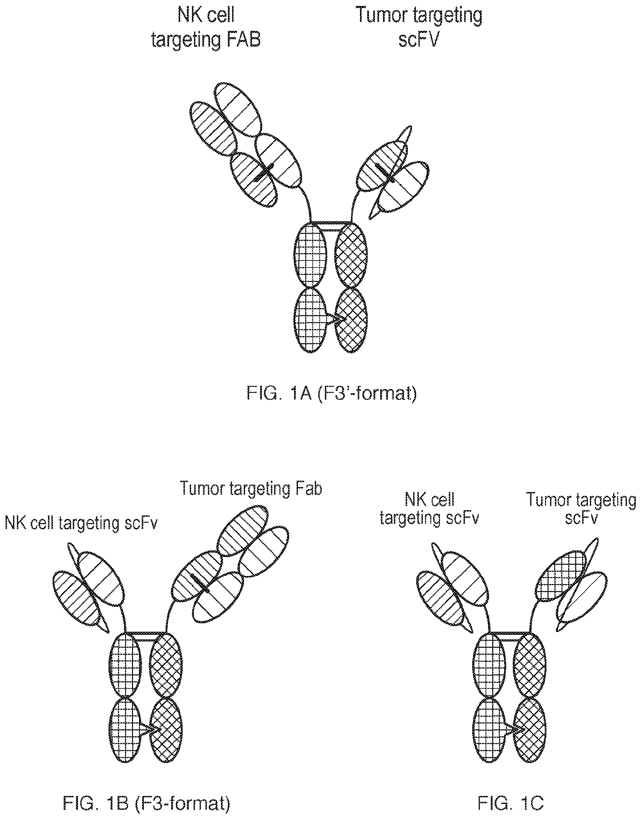

**FIG. 1A-1C** illustrate three exemplary formats of a multi-specific binding protein that includes the scFv linked to an antibody constant region/domain via a hinge. **FIG. 1A** represents a trispecific antibody (TriNKET) that contains a tumor-targeting scFv or single-chain TCR (scTCR) fragment, a NKG2D-targeting Fab, and a heterodimerized antibody constant region/domain ("CD domain") that binds CD16. The antibody format is referred herein as F3'-TriNKET. **FIG. 1B** resents an antibody that contains a NKG2D-targeting scFv, a tumor-targeting Fab or an extracellular TCR fragment, and a heterodimerized antibody constant region. The antibody format is referred herein as F3-TriNKET.**FIG. 1C** represents an antibody that contains a NKG2D-targeting scFv, a tumor-targeting scFv or scTCR fragment, and a heterodimerized antibody constant region. In certain exemplary TriNKETs, heterodimerization mutations on the CD domain linked to the antigen-binding site that binds NKG2D include K360E and K409W; heterodimerization mutations on the opposite CD include Q347R, D399V and F405T.

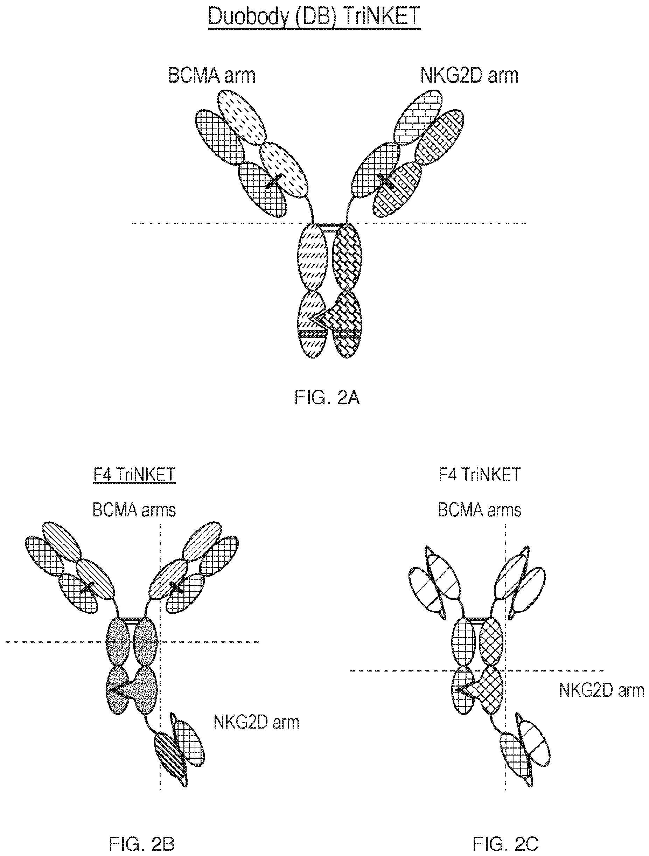

**FIG. 2A** illustrate a trispecific antibody (TriNKET), wherein the first antigen-binding site or antigen-binding TCR fragment binds a tumor-associated antigen (such as BCMA shown here) or a peptide thereof presented by a major histocompatibility complex (MHC); the second antigen-binding site binds NKG2D; and a heterodimerized antibody constant region binds CD16. In certain exemplary TriNKETs, heterodimerization mutations on the CD domain linked to the antigen-binding site that binds NKG2D include K360E and K409W; heterodimerization mutations on the opposite CD include Q347R, D399V and F405T.
**FIGs. 2B-2C** illustrate a trispecific antibody (TriNKET), wherein the first antigen binding site and the second antigen binding sites, or the first antigen-binding TCR fragment and the second antigen-binding TCR fragment, bind the same tumor-associated antigen (such as BCMA shown here) or the same peptide from a tumor-associated antigen presented by the same MHC; the third antigen binding site binds NKG2D; and a heterodimerized antibody constant region binds CD16. These antibody formats are referred herein as F4-TriNKET. In certain exemplary TriNKETs, heterodimerization mutations on the CD domain linked to the antigen-binding site that binds NKG2D include K360E and K409W; heterodimerization mutations on the opposite CD include Q347R, D399V and F405T. **FIG. 2B** illustrates that the first two antigen-binding sites in the Fab format. **FIG. 2C** illustrates that the first two antigen-binding sites in the scFv format.
**FIGs. 3A-3C** show results of an accelerated stability study carried out at 37 °C, in which F3'-TriNKET was found to be stable over 4 weeks.
**FIG. 4** shows that the purified F3'-TriNKET was stable during low pH hold.
**FIG. 5A** and **FIG. 5B** shows that the purified F3'-TriNKET was stable after 5 cycles of freeze-thaw, irrespective of the pH (FIG. 5A shows freeze-thaw cycles in PBS, and FIG. 5B shows freeze-thaw cycles in citrate at pH 5.5).
**FIG. 6** shows bar graphs of the forced degradation conditions in which the F3'-TriNKET remained stable.
**FIG. 7** shows binding of F3'-TriNKET-HER2 or Trastuzumab to HER2+ Colo-201 cells.
**FIG. 8** shows binding of F3'-TriNKET-CD33 or CD33 monoclonal antibody to CD33 expressed on Molm-13 cells.
**FIG. 9** and **FIG. 10** show F3'-TriNKET-HER2-mediated cytotoxicity against the HER2-low cell line 786-O, and HER2-high cell line SkBr-3, respectively.
**FIG. 11** and **FIG. 12** show F3'-TriNKET-CD33-mediated cytotoxicity towards two CD33 positive human cell lines, EOL-1 and THP-1, respectively.

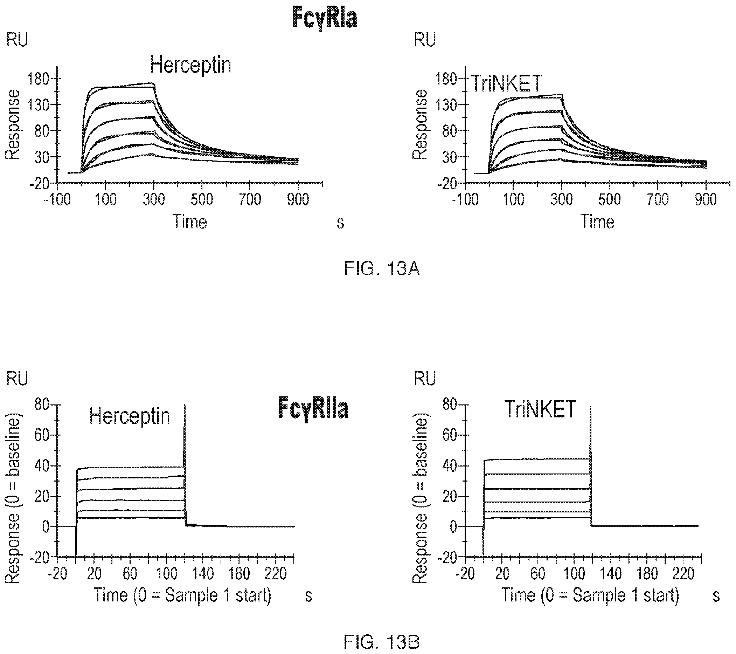

**FIG. 13A** shows that F3'-TriNKET-HER2 binding to FcyRIa is similar to Herceptin.

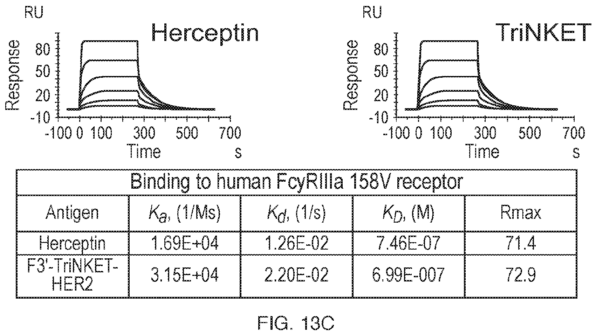

**FIG. 13B** shows that F3'-TriNKET-HER2 binding to FcγRIIa is similar to Herceptin. **FIG. 13C** shows that F3'-TriNKET-HER2 binding to FcγRIIIa 158V is similar to Herceptin.

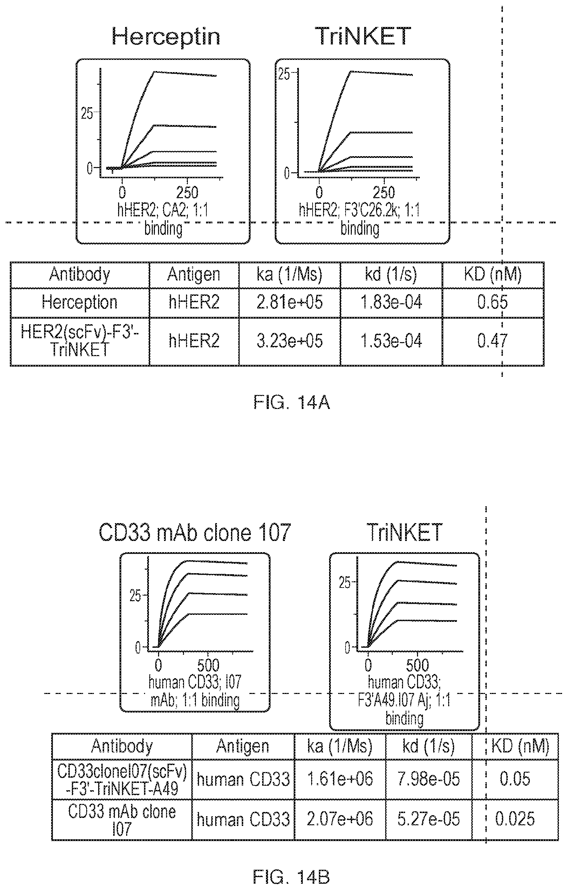

**FIG. 14A** shows that F3'-TriNKET-HER2, where HER2 binder is an scFv, binding to human HER2 is similar to Herceptin in which HER2 binders are Fabs. **FIG 14B** shows that F3'TriNKET-CD33, where CD33 binder is an scFv, binding to human CD33 is similar to CD33 monoclonal antibody in which CD33 binders are Fabs.

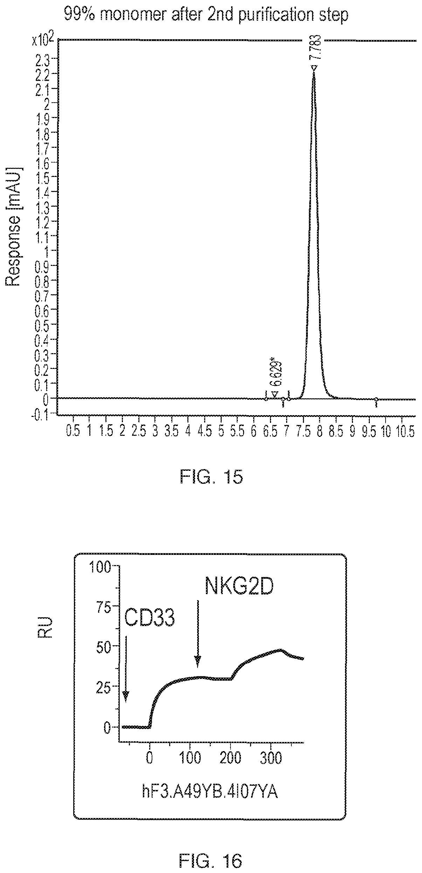

**FIG. 15** shows that two-step purification of F3-TriNKET-BCMA achieves 99% purity.
**FIG. 16** shows simultaneous engagement of NKG2D and CD33 targets with high potency by F3-TriNKET-CD33.

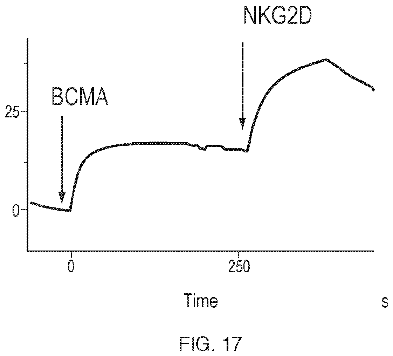

**FIG. 17** shows simultaneous engagement of NKG2D and BCMA targets with high potency by F3-TriNKET-BCMA.

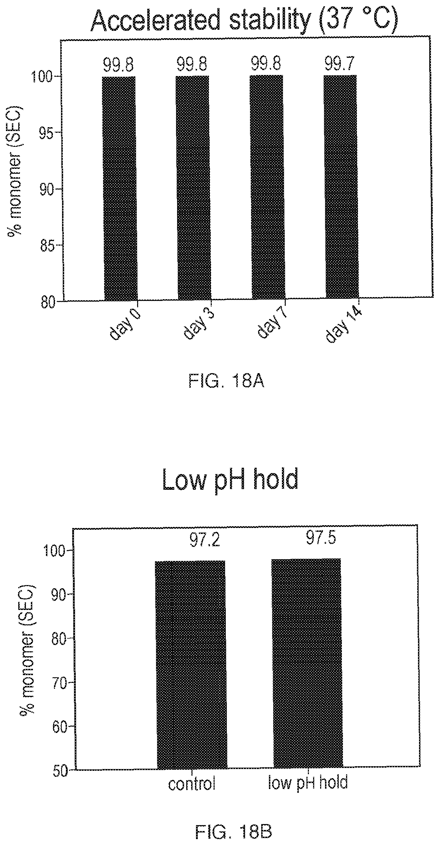

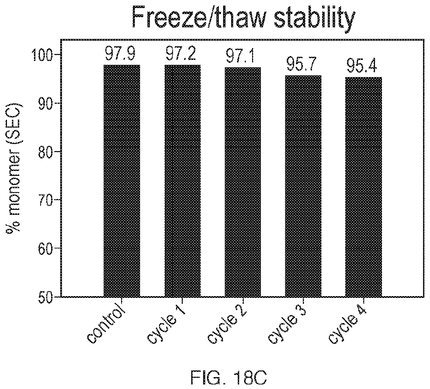

**FIG. 18A** shows that F3-TriNKET format is stable for at least up to 14 days. **FIG. 18B** shows that F3-TriNKET format is stable after a low pH hold. **FIG. 18C** shows that F3-TriNKET format is stable after at least up to 5 cycles of freeze-thaw.
**FIG. 19** are line graphs showing that BCMA targeting F4-TriNKETs with different NKG2D-binding domains enhance human NK cell lysis of KMS 12-PE myeloma cells.
**FIG. 20** are line graphs showing that BCMA targeting F4-TriNKETs with different NKG2D-binding domains enhance human NK cell lysis of MM.1R myeloma cells.
**FIG. 21** are line graphs showing binding of F4-TriNKET, duobody-TriNKET, and BCMA monoclonal antibody to MM.1R myeloma cells.
**FIG. 22** are FACS shows that incubation with F4-TriNKET increases surface BCMA expression over time.
**FIG. 23** are line graphs showing that F4-TriNKET stabilizes surface BCMA.
**FIG. 24** are bar graphs showing that BCMA-targeting F4-TriNKET with A49 binder mediates more potent killing of KMS 12-PE myeloma cells at different concentrations over 30 hours than the Duobody-TriNKET
**FIG. 25** are bar graphs showing that BCMA-targeting F4-TriNKET with A49 binder mediates more potent killing of MM.1S myeloma cells at different concentrations over 30 hours than the Duobody-TriNKET.

### DETAILED DESCRIPTION

The invention provides an improvement on a single-chain variable fragment (scFv) that is linked to an antibody constant domain via a hinge sequence. The hinge sequence provides flexibility of the scFv binding to an antigen. This invention also provides multi-specific binding proteins that includes one or more of the scFv, wherein the multi-specific binding proteins bind the NKG2D receptor and CD16 receptor on natural killer cells, and a tumor-associated antigen. This invention also provides multi-specific binding proteins that contain two tumor-associated antigen binding sites binding to the same tumor-associated antigen, and bind the NKG2D receptor and CD16 receptor on natural killer cells. Pharmaceutical compositions comprising such multi-specific binding proteins, and therapeutic methods using such multi-specific binding proteins and pharmaceutical compositions, for purposes such as treating cancer are also provided. Various aspects of the invention are set forth below in sections; however, aspects of the invention described in one particular section are not to be limited to any particular section.

To facilitate an understanding of the present invention, a number of terms and phrases are defined below.

The terms "a" and "an" as used herein mean "one or more" and include the plural unless the context is inappropriate.

As used herein, the terms "subject" and "patient" refer to an organism to be treated by the methods and compositions described herein. Such organisms preferably include, but are not limited to, mammals (*e.g*., murines, simians, equines, bovines, porcines, canines, felines, and the like), and more preferably include humans.

As used herein, the term "antigen-binding site" refers to the part of the immunoglobulin molecule that participates in antigen binding. In human antibodies, the antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". Thus the term "FR" refers to amino acid sequences which are naturally found between and adjacent to hypervariable regions in immunoglobulins. In a human antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." In certain animals, such as camels and cartilaginous fish, the antigen-binding site is formed by a single antibody chain providing a "single domain antibody." Antigen-binding sites can exist in an intact antibody, in an antigen-binding fragment of an antibody that retains the antigen-binding surface, or in a recombinant polypeptide such as an scFv, using a peptide linker to connect the heavy chain variable domain to the light chain variable domain in a single polypeptide.

As used herein, the terms "antigen-binding T cell receptor fragment" and "antigen-binding TCR fragment" are used interchangeably and refer to a portion of a T cell receptor (TCR) that binds a cognate antigen. In human αβ TCRs, an antigen-binding TCR fragment comprises an alpha chain variable domain (Vα) and a beta chain variable domain (Vβ), each comprising three complementarity-determining regions (CDRs). The hypervariable loops named CDR3α and CDR3β occupy a central position for binding an antigen peptide; the germline-encoded CDR1α, CDR2α, CDR1β, and CDR2β loops make most contact with an MHC that presents the antigen peptide. In human γδ TCRs, an antigen-binding TCR fragment comprises a gamma chain variable domain (Vγ) and a delta chain variable domain (Vδ), each comprising three CDRs. Human γδ TCRs recognize antigens (*e.g.,* peptide or lipid) presented by MHC or MHC-related molecules (*e.g.,* CD1, endothelial protein C receptor (EPCR), or MHC class I polypeptide-related sequence A (MICA)). It is understood that other proteins, such as F1-ATPase, may also present antigens to γδ TCRs. Antigen-binding TCR fragments can exist in an intact TCR, in an angineered TCR having TCR chains linked by a disulfide bond, in an antigen-binding fragment of an intact or engineered TCR that retains the antigen-binding surface, or in a recombinant polypeptide having variable domains of a TCR (*e.g.,* Vα and Vβ) connected by a peptide linker in a single polypeptide. Non-limiting examples of an antigen-binding TCR fragment include a TCR fragment comprising the variable domains (*e.g.,* Vα and Vβ) and constant domains (*e.g.,* Cα and Cβ) but lacking the connecting regions, transmembrane regions, and cytoplasmic regions of the TCR, referred to herein as an "extracellular TCR fragment," and variable regions of a TCR (*e.g.,* Vα and Vβ) connected by a peptide linker, referred to herein as a "single-chain TCR (scTCR) fragment."

As used herein, the term "effective amount" refers to the amount of a compound *(e.g.,* a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g.,* lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (*e*.*g*., such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

Throughout the description, where compositions are described as having, including, or comprising specific components, or where processes and methods are described as having, including, or comprising specific steps, it is contemplated that, additionally, there are compositions of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### I. PROTEINS

The current invention provides an improvement on a single-chain variable fragment (scFv) that is linked to an antibody constant domain via a hinge sequence. In some embodiments, the hinge comprises amino acids Ala-Ser. In some other embodiments, the hinge comprises amino acids Ala-Ser and Thr-Lys-Gly. The scFv may include a heavy chain variable domain and a light chain variable domain. In some embodiments, the scFv binds NKG2D or a tumor-associated antigen. The hinge sequence provides flexibility of the scFv binding to the target antigen.

In some embodiments of the scFv, the heavy chain variable domain forms a disulfide bridge with the light chain variable domain to enhance stability of the scFv. For example, a disulfide bridge can be formed between the C44 residue of the heavy chain variable domain and the C100 residue of the light chain variable domain. In some embodiments, the heavy chain variable domain is linked to the light chain variable domain via a flexible linker. Any suitable linker can be used, for example, the (G₄S)₄ linker. In some embodiments of the scFv, the heavy chain variable domain is positioned at the N-terminus of the light chain variable domain. In some embodiments of the scFv, the heavy chain variable domain is positioned at the C terminus of the light chain variable domain.

In some embodiments, the antibody constant domain linked to the scFv can derive from a constant region of an antibody of any species that binds to CD16. In some embodiments, the amino acid sequence of the constant region is at least 90% identical to a human antibody constant region, such as an human IgG1 constant region, an IgG2 constant region, IgG3 constant region, or IgG4 constant region. In some other embodiments, the amino acid sequence of the constant region is at least 90% identical to an antibody constant region from another mammal, such as rabbit, dog, cat, mouse, or horse. In some embodiments, the antibody constant region includes a hinge, a CH2 domain, a CH3 domain and optionally a CH1 domain. In some embodiments, the antibody constant region that includes a hinge, a CH2 domain, a CH3 domain and optionally a CH1 domain is derived from a human IgG1 antibody. In some embodiments, the antibody constant region includes an amino acid sequence at least 90% identical to amino acids 234-332 of a human IgG1 antibody.

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (see, Sondermann et al., Nature, 406 (6793):267-273). Based on the known domains, mutations can be selected to enhance or reduce the binding affinity to CD16, such as by using phage-displayed libraries or yeast surface-displayed cDNA libraries, or can be designed based on the known three-dimensional structure of the interaction.

In some embodiments, the antibody constant domain comprises a CH2 domain and a CH3 domain of an IgG antibody, for example, a human IgG1 antibody. In some embodiments, mutations are introduced in the antibody constant domain to enable heterodimerization with another antibody constant domain. For example, if the antibody constant domain is derived from the constant domain of a human IgG1, the antibody constant domain can comprise an amino acid sequence at least 90% identical to amino acids 234-332 of a human IgG1 antibody, and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439. In some embodiments, the antibody constant domain can comprise an amino acid sequence at least 90% identical to amino acids 234-332 of a human IgG1 antibody, and differs by one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E.

Listed below are examples of the scFv linked to an antibody constant region that also includes mutations that enable heterodimerization of two polypeptide chains. The scFv containing a heavy chain variable domain (V_{H}) and a light chain variable domain (V_{L}) from Trastuzumab is used an example. Each sequence represents V_{L}-(G₄S)₄-V_{H}-hinge (AS)-Fc containing heterodimerization mutations (underlined). V_{L} and V_{H} contain 44V_{H} -100V_{L} S-S bridge (underlined), and can be from any tumor targeting or NKG2D binding antibody. The Ala-Ser (AS, underlined) is included at the elbow hinge region sequence to balance between flexibility and optimal geometry. In certain embodiments, an additional sequence Thr-Lys-Gly can be added to the AS sequence at the hinge. (G₄S)₄ linker is underlined in the sequences listed in the paragraph below.

Trastuzumab -scFv-Fc A1 and Trastuzumab -scFv-Fc B1 can preferentially pair and form a heterodimer. Trastuzumab -scFv-Fc A2 and Trastuzumab -scFv-Fc B2 can preferentially pair and form a heterodimer.
Trastuzumab -scFv-Fc A1 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGCGTKVEIK
GGGGSGGGGSGGGGSGGGGS
EVQLVESGGGLVQPGGSLRLSCAASGFNIKDTYIHWVRQAPGKCLEWVARIYPTNG
YTRYADSVKGRFTISADTSKNTAYLQMNSLRAEDTAVYYCSRWGGDGFYAMDYW
GQGTLVTVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPCRDELTKNQVSLWCLVKGFYPSDIAVEWESNGQPENN
YKTTPPVLDSDGSFFLYSKLTVDKSRWQQGNVFSCSVMHEALHNHYTQKSLSLSPG
(SEQ ID NO:303)
Trastuzumab -scFv-Fc B1 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGCGTKVEIK
GGGGSGGGGSGGGGSGGGGS
EVQLVESGGGLVQPGGSLRLSCAASGFNIKDTYIHWVRQAPGKCLEWVARIYPTNG
YTRYADSVKGRFTISADTSKNTAYLQMNSLRAEDTAVYYCSRWGGDGFYAMDYW
GQGTLVTVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVCTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPG
(SEQ ID NO:304)
Trastuzumab -scFv-Fc A2 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGCGTKVEIK
GGGGSGGGGSGGGGSGGGGS
EVQLVESGGGLVQPGGSLRLSCAASGFNIKDTYIHWVRQAPGKCLEWVARIYPTNG
YTRYADSVKGRFTISADTSKNTAYLQMNSLRAEDTAVYYCSRWGGDGFYAMDYW
GQGTLVTVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVCTLPPSRDELTENQVSLTCLVKGFYPSDIAVEWESNGQPENN
YKTTPPVLDSDGSFFLYSWLTVDKSRWQQGNVFSCSVMHEALHNHYTQKSLSLSPG
(SEQ ID NO:305)
Trastuzumab -scFv-Fc B2

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGCGTKVEIK
GGGGSGGGGSGGGGSGGGGS
EVQLVESGGGLVQPGGSLRLSCAASGFNIKDTYIHWVRQAPGKCLEWVARIYPTNG
YTRYADSVKGRFTISADTSKNTAYLQMNSLRAEDTAVYYCSRWGGDGFYAMDYW
GQGTLVTVSS
AS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPRVYTLPPCRDELTKNQVSLTCLVKGFYPSDIAVEWESNGQPENN
YKTTPPVLVSDGSFTLYSKLTVDKSRWQQGNVFSCSVMHEALHNHYTQKSLSLSPG
(SEQ ID NO:306)

In another aspect, the current invention provides a protein that contains the scFv linked to an antibody constant region described above. In some embodiments, the protein includes a first antigen-binding site, which comprises the scFv linked to an antibody constant domain; a second antigen-binding site which may take the Fab or the scFv format; and a second antibody constant domain linked to the second antigen-binding site. In some embodiments, the protein is multi-specific, wherein the first antigen binding site binds NKG2D, the second antigen binding sites, in the form of an Fab, bind a tumor-associated antigen, and the antibody constant regions bind CD16 (referred herein as the F3-TriNKET, as shown in FIG. 1B). In some other embodiments, the protein is multi-specific, wherein the first antigen binding site binds a tumor-associated antigen, the second antigen binding site, in the form of an Fab, binds NKG2D, and the antibody constant regions bind CD16 (referred herein as the F3'-TriNKET, as shown in FIG. 1A). The antibody constant region linked to the scFv heterodimerizes with the antibody constant region of the second antigen-binding site of the proteins described herein. The multi-specific binding proteins including the scFv described herein can take various formats as shown in FIG. 1A-1C.

The multi-specific binding proteins can bind to the NKG2D receptor-expressing cells, which can include but are not limited to NK cells, γδ T cells and CD8⁺ αβ T cells. Upon NKG2D binding, the multi-specific binding proteins may block natural ligands, such as ULBP6 and MICA, from binding to NKG2D and activating NKG2D receptors.

The multi-specific binding proteins binds to cells expressing CD16, an Fc receptor on the surface of leukocytes including natural killer cells, macrophages, neutrophils, eosinophils, mast cells, and follicular dendritic cells.

Upon binding to the NKG2D receptor and CD16 receptor on natural killer cells, and a tumor-associated antigen on cancer cells, the multi-specific binding proteins proteins can engage more than one kind of NK-activating receptor, and may block the binding of natural ligands to NKG2D. In certain embodiments, the proteins can agonize NK cells in humans. In some embodiments, the proteins can agonize NK cells in humans and in other species such as rodents and cynomolgus monkeys.

In certain embodiments of the present invention, a multi-specific binding protein comprises a first antigen-binding site that binds a tumor-associated antigen; a second antigen-binding site that binds the same tumor-associated antigen as the first antigen-binding site; a third antigen-binding site that binds NKG2D; and an antibody constant region or a portion thereof sufficient to bind CD16, or a fourth antigen-binding site that binds CD16. Any one of the antigen binding sites can either take the form of Fab or scFv. Exemplary formats are illustrated in FIG. 2B-2C. Both V_{H}-V_{L} and the V_{L}-V_{H} orientations of the scFv are embodiments of the present disclosure.

In certain embodiments, the first antigen-binding site or the second antigen-binding site that bind to the same tumor-associated antigen is an scFv, and the third antigen-binding site that binds NKG2D is an scFv. In certain embodiments, the first antigen-binding site and the second antigen-binding site that bind to the same tumor-associated antigen are each an scFv, and the third antigen-binding site that binds NKG2D is an scFv. In certain embodiments, the first antigen-binding site or the second antigen-binding site that bind to the same tumor-associated antigen is an Fab, and the third antigen-binding site that binds NKG2D is an scFv. In certain embodiments, the first antigen-binding site and the second antigen-binding site that bind to the same tumor-associated antigen are each an Fab, and the third antigen-binding site that binds NKG2D is an scFv. In certain embodiments, the first antigen-binding site and the second antigen-binding site of an F4-TriNKET of the present disclosure have identical amino acid sequences.

In other embodiments, a multi-specific binding protein disclosed herein comprises a first antigen-binding site that binds a tumor-associated antigen; a second antigen-binding site that binds a different antigen; a third antigen-binding site that binds NKG2D; and an antibody constant region or a portion thereof sufficient to bind CD16, or a fourth antigen-binding site that binds CD16. Any one of the antigen binding sites can either take the form of Fab or scFv (in either the VH-VL or the VL-VH orientation). In certain embodiments, the first antigen-binding site or the second antigen-binding site that bind to two different antigens is an scFv, and the third antigen-binding site that binds NKG2D is an scFv. In certain embodiments, the first antigen-binding site and the second antigen-binding site that bind to two different antigens are each an scFv, and the third antigen-binding site that binds NKG2D is an scFv. In certain embodiments, the first antigen-binding site or the second antigen-binding site that bind to two different antigens is an Fab, and the third antigen-binding site that binds NKG2D is an scFv. In certain embodiments, the first antigen-binding site and the second antigen-binding site that bind to two different antigens are each an Fab, and the third antigen-binding site that binds NKG2D is an scFv.

In certain embodiments, the multi-specific binding protein (referred herein as the F4-TriNKETs) provided here provides bivalent engagement of tumor-associated antigen, thereby stabilizing and maintaining the tumor-associated antigen on cancer cell surface, and enhance cytotoxicity towards the cancer cells by NK cells. In some embodiments, bivalent engagement of tumor-associated antigens by the multi-specific binding proteins confer higher avidity of the multi-specific binding proteins to the cancer cells, thereby facilitating stronger cytotoxic response from the NK cells towards the cancer cells, especially towards the cancer cells expressing a low level of tumor-associated antigen.

The current invention also provides a multi-specific binding protein comprising (a) an antigen-binding site that binds NKG2D; (b) an antigen-binding TCR fragment; and (c) an antibody constant region or a portion thereof sufficient to bind CD16, or an additional antigen-binding site that binds CD16. In certain embodiments, the antigen-binding TCR fragment binds a peptide from a tumor-associated antigen (TAA) presented by an MHC, *e.g.,* a peptide from a human tumor-associated antigen presented by a human leukocyte antigen (HLA). Element (b) can exist in various formats (*e*.*g*., soluble formats), such as an extracellular TCR fragment or an scTCR fragment. Elements (a) and (c) can exist in various formats and/or comprise various mutations disclosed above. For example, in certain embodiments, element (c) is an antibody constant region or a portion thereof sufficient to bind CD16, the antibody constant region or portion thereof comprising (i) a first antibody constant domain linked to the antigen-bindind site that binds NKG2D, and (ii) a second antibody constant domain linked to the antigen-binding TCR fragment, wherein the first and second antibody constant domains can heterodimerize.

In certain embodiments, the antigen-binding site is an Fab fragment, and the antigen-binding TCR fragment is an scTCR fragment. Given that its NKG2D-binding portion is an Fab and its TAA-binding portion comprises variable domains linked by a peptide linker in a single chain (similar to the multi-specific binding protein comprising a first antigen-binding site, which comprises an scFv linked to an antibody constant domain; a second antigen-binding site, which takes the form of Fab; and a second antibody constant domain linked to the second antigen-binding site, wherein the first antigen binding site binds a tumor-associated antigen, the second antigen binding site binds NKG2D, and the antibody constant regions bind CD16), the format of this multi-specific binding protein is also referred to herein as F3'-TriNKET, as illustrated in FIG. 1A.

In certain embodiments, the antigen-binding site is an scFv, and the antigen-binding TCR fragment is an extracellular TCR fragment. Given that its NKG2D-binding portion is an scFv and its TAA-binding portion comprises variable domains and constant domains (similar to the multi-specific binding protein comprising a first antigen-binding site, which comprises an scFv linked to an antibody constant domain; a second antigen-binding site, which takes the form of Fab; and a second antibody constant domain linked to the second antigen-binding site, wherein the first antigen binding site binds NKG2D, the second antigen binding sites bind a tumor-associated antigen, and the antibody constant regions bind CD16), the format of this multi-specific binding protein is also referred to herein as F3-TriNKET, as illustrated in FIG. 1B.

In certain embodiments, the antigen-binding site is an scFv, and the antigen-binding TCR fragment is an scTCR fragment. Such format is illustrated in FIG. 1C. In certain embodiments, the antigen-binding site is a Fab, and the antigen-binding TCR fragment is an extracellular TCR fragment. Such format is illustrated in FIG. 2A.

In certain embodiments, the multi-specific binding protein comprises a first antigen-binding TCR fragment that binds an antigen *(e.g.,* a TAA peptide presented by an MHC); a second antigen-binding TCR fragment that binds the same antigen as the first antigen-binding TCR fragment; an antigen-binding site that binds NKG2D; and an antibody constant region or a portion thereof sufficient to bind CD16, or a fourth antigen-binding site that binds CD16. Alternatively, it is contemplated that the first antigen-binding TCR fragment and the second antigen-binding TCR fragment can bind two different TAA peptides presented by the same or different MHCs. Any one of the antigen binding sites can take the form of either Fab or scFv. The first and second antigen-binding TCR fragments can take the form of either extracellular TCR fragment or scTCR fragment. Exemplary formats, which provide bivalent engagement of an antigen (*e.g.,* a TAA peptide presented by an MHC), are referred to herein as F4-TriNKETs and illustrated in FIG. 2B-2C.

In certain embodiments, the first antigen-binding TCR fragment and the second antigen-binding TCR fragment of an F4-TriNKET of the present disclosure bind to the same TAA peptide presented by the same MHC. In certain embodiments, the first antigen-binding TCR fragment or the second antigen-binding TCR fragment is an scFv. In certain embodiments, the first antigen-binding TCR fragment and the second antigen-binding TCR fragment are each an scFv. In certain embodiments, the first antigen-binding TCR fragment or the second antigen-binding TCR fragment is an Fab. In certain embodiments, the first antigen-binding TCR fragment and the second antigen-binding TCR fragment are each an Fab.

The F4-TriNKETs with antigen-binding TCR fragments provided here provides bivalent engagement of antigens (*e.g*., TAA peptides presented by MHCs), thereby stabilizing and maintaining the TAA peptide on cancer cell surface, and enhance cytotoxicity towards the cancer cells by NK cells. In some embodiments, bivalent engagement of TAA peptides by the multi-specific binding proteins confer higher avidity of the multi-specific binding proteins to the cancer cells, thereby facilitating stronger cytotoxic response from the NK cells towards the cancer cells, especially towards the cancer cells presenting a low level of the TAA peptide.

It is understood that where the protein of the present invention comprises an scFv, the V_{H} can be positioned either to the C-terminus or to the N-terminus of V_{L}. Similarly, where the protein of the present invention comprises an scTCR fragment, the Vα can be positioned either to the C-terminus or to the N-terminus of Vβ.

Exemplary sequences of an NKG2D binding site and a tumor-associated antigen binding site or antigen-binding TCR fragments, which can incorporated into the F3/F3' and F4-TriNKETs are listed herein.

### NKG2D-binding site

Table 1 lists peptide sequences of heavy chain variable domains and light chain variable domains that, in combination, can bind to NKG2D. Unless indicated otherwise, the CDR sequences provided in Table 1 are determined under Kabat. In some embodiments, the heavy chain variable domain and the light chain variable domain are arranged in Fab format. In some embodiments, the heavy chain variable domain and the light chain variable domain are fused together to from an scFv.

The NKG2D binding domains can vary in their binding affinity to NKG2D, nevertheless, they all activate human NKG2D and NK cells.

| Table 1 | | |
|---|---|---|
| Clones | Heavy chain variable region amino acid sequence | Light chain variable region amino acid sequence |
| ADI-27705 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:1) CDR1: GSFSGYYWS (non-Kabat) (SEQ ID NO:3) or GYYWS (SEQ ID NO:307) CDR2: EIDHSGSTNYNPSLKS (SEQ ID NO:4) CDR3: ARARGPWSFDP (non-Kabat) (SEQ ID NO:5) or ARGPWSFDP (SEQ ID NO:308) | DIQMTQSPSTLSASVGDRVTIT CRASQSISSWLAWYQQKPGKA PKLLIYKASSLESGVPSRFSGSG SGTEFTLTISSLQPDDFATYYC QQYNSYPITFGGGTKVEIK (SEQ ID NO:2) |
| ADI-27724 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:6) | EIVLTQSPGTLSLSPGERATLSC RASQSVSSSYLAWYQQKPGQA PRLLIYGASSRATGIPDRFSGSG SGTDFTLTISRLEPEDFAVYYC QQYGSSPITFGGGTKVEIK (SEQ ID NO:7) |
| ADI-27740 (A40) | QVQLQQWGAGLLKPSETLSLTCAV YGGSFSGYYWSWIRQPPGKGLEWIG EIDHSGSTNYNPSLKSRVTISVDTSK | DIQMTQSPSTLSASVGDRVTIT CRASQSIGSWLAWYQQKPGK APKLLIYKASSLESGVPSRFSGS |
| | NQFSLKLSSVTAADTAVYYCARAR GPWSFDPWGQGTLVTVSS (SEQ ID NO:8) | GSGTEFTLTISSLQPDDFATYY CQQYHSFYTFGGGTKVEIK (SEQ ID NO:9) |
| ADI-27741 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:10) | DIQMTQSPSTLSASVGDRVTIT CRASQSIGSWLAWYQQKPGK APKLLIYKASSLESGVPSRFSGS GSGTEFTLTISSLQPDDFATYY CQQSNSYYTFGGGTKVEIK (SEQ ID NO:11) |
| ADI-27743 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:12) | DIQMTQSPSTLSASVGDRVTIT CRASQSISSWLAWYQQKPGKA PKLLIYKASSLESGVPSRFSGSG SGTEFTLTISSLQPDDFATYYC QQYNSYPTFGGGTKVEIK (SEQ ID NO:13) |
| ADI-28153 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWGFDPWGQGTLVTVSS (SEQ ID NO:14) | ELQMTQSPSSLSASVGDRVTIT CRTSQSISSYLNWYQQKPGQPP KLLIYWASTRESGVPDRFSGSG SGTDFTLTISSLQPEDSATYYC QQSYDIPYTFGQGTKLEIK (SEQ ID NO:15) |
| ADI-28226 (C26) | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:16) | DIQMTQSPSTLSASVGDRVTIT CRASQSISSWLAWYQQKPGKA PKLLIYKASSLESGVPSRFSGSG SGTEFTLTISSLQPDDFATYYC QQYGSFPITFGGGTKVEIK (SEQ ID NO:17) |
| ADI-28154 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:18) | DIQMTQSPSTLSASVGDRVTIT CRASQSISSWLAWYQQKPGKA PKLLIYKASSLESGVPSRFSGSG SGTDFTLTISSLQPDDFATYYC QQSKEVPWTFGQGTKVEIK (SEQ ID NO:19) |
| ADI-29399 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:20) | DIQMTQSPSTLSASVGDRVTIT CRASQSISSWLAWYQQKPGKA PKLLIYKASSLESGVPSRFSGSG SGTEFTLTISSLQPDDFATYYC QQYNSFPTFGGGTKVEIK (SEQ ID NO:21) |
| ADI-29401 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:22) | DIQMTQSPSTLSASVGDRVTIT CRASQSIGSWLAWYQQKPGK APKLLIYKASSLESGVPSRFSGS GSGTEFTLTISSLQPDDFATYY CQQYDIYPTFGGGTKVEIK (SEQ ID NO:23) |
| ADI-29403 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:24) | DIQMTQSPSTLSASVGDRVTIT CRASQSISSWLAWYQQKPGKA PKLLIYKASSLESGVPSRFSGSG SGTEFTLTISSLQPDDFATYYC QQYDSYPTFGGGTKVEIK (SEQ ID NO:25) |
| ADI-29405 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:26) | DIQMTQSPSTLSASVGDRVTIT CRASQSISSWLAWYQQKPGKA PKLLIYKASSLESGVPSRFSGSG SGTEFTLTISSLQPDDFATYYC QQYGSFPTFGGGTKVEIK (SEQ ID NO:27) |
| ADI-29407 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:28) | DIQMTQSPSTLSASVGDRVTIT CRASQSISSWLAWYQQKPGKA PKLLIYKASSLESGVPSRFSGSG SGTEFTLTISSLQPDDFATYYC QQYQSFPTFGGGTKVEIK (SEQ ID NO:29) |
| ADI-29419 | QVQLQQWGAGLLKPSETLSLTCAV YGGSFSGYYWSWIRQPPGKGLEWIG EIDHSGSTNYNPSLKSRVTISVDTSK | DIQMTQSPSTLSASVGDRVTIT CRASQSISSWLAWYQQKPGKA PKLLIYKASSLESGVPSRFSGSG |
| | NQFSLKLSSVTAADTAVYYCARAR GPWSFDPWGQGTLVTVSS (SEQ ID NO:30) | SGTEFTLTISSLQPDDFATYYC QQYSSFSTFGGGTKVEIK (SEQ ID NO:31) |
| ADI-29421 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:32) | DIQMTQSPSTLSASVGDRVTIT CRASQSISSWLAWYQQKPGKA PKLLIYKASSLESGVPSRFSGSG SGTEFTLTISSLQPDDFATYYC QQYESYSTFGGGTKVEIK (SEQ ID NO:33) |
| ADI-29424 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:34) | DIQMTQSPSTLSASVGDRVTIT CRASQSISSWLAWYQQKPGKA PKLLIYKASSLESGVPSRFSGSG SGTEFTLTISSLQPDDFATYYC QQYDSFITFGGGTKVEIK (SEQ ID NO:35) |
| ADI-29425 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:36) | DIQMTQSPSTLSASVGDRVTIT CRASQSISSWLAWYQQKPGKA PKLLIYKASSLESGVPSRFSGSG SGTEFTLTISSLQPDDFATYYC QQYQSYPTFGGGTKVEIK (SEQ ID NO:37) |
| ADI-29426 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:38) | DIQMTQSPSTLSASVGDRVTIT CRASQSIGSWLAWYQQKPGK APKLLIYKASSLESGVPSRFSGS GSGTEFTLTISSLQPDDFATYY CQQYHSFPTFGGGTKVEIK (SEQ ID NO:39) |
| ADI-29429 | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:40) | DIQMTQSPSTLSASVGDRVTIT CRASQSIGSWLAWYQQKPGK APKLLIYKASSLESGVPSRFSGS GSGTEFTLTISSLQPDDFATYY CQQYELYSYTFGGGTKVEIK (SEQ ID NO:41) |
| ADI-29447 (F47) | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:42) | DIQMTQSPSTLSASVGDRVTIT CRASQSISSWLAWYQQKPGKA PKLLIYKASSLESGVPSRFSGSG SGTEFTLTISSLQPDDFATYYC QQYDTFITFGGGTKVEIK (SEQ ID NO:43) |
| ADI-27727 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCARG DSSIRHAYYYYGMDVWGQGTTVTV SS (SEQ ID NO:44) | DIVMTQSPDSLAVSLGERATIN CKSSQSVLYSSNNKNYLAWYQ QKPGQPPKLLIYWASTRESGVP DRFSGSGSGTDFTLTISSLQAE DVAVYYCQQYYSTPITFGGGT KVEIK (SEQ ID NO:48) |
| | CDR1: GTFSSYAIS (non-Kabat) (SEQ ID NO:45) or SYAIS (SEQ ID NO:309) | CDR1: KSSQSVLYSSNNKNYLA (SEQ ID NO:49) |
| | CDR2: GIIPIFGTANYAQKFQG (SEQ ID NO:46) | |
| | | CDR2: WASTRES (SEQ ID NO:50) |
| | CDR3: ARGDSSIRHAYYYYGMDV (non-Kabat) (SEQ ID NO:47) or GDSSIRHAYYYYGMDV (SEQ ID NO:310) | |
| | | CDR3: QQYYSTPIT (SEQ ID NO:51) |
| ADI-29443 (F43) | QLQLQESGPGLVKPSETLSLTCTVSG GSISSSSYYWGWIRQPPGKGLEWIGS IYYSGSTYYNPSLKSRVTISVDTSKN QFSLKLSSVTAADTAVYYCARGSDR FHPYFDYWGQGTLVTVSS (SEQ ID NO:52) | EIVLTQSPATLSLSPGERATLSC RASQSVSRYLAWYQQKPGQA PRLLIYDASNRATGIPARFSGS GSGTDFTLTISSLEPEDFAVYY CQQFDTWPPTFGGGTKVEIK (SEQ ID NO:56) |
| | CDR1: GSISSSSYYWG (non-Kabat) (SEQ ID NO:53) or SSSYYWG (SEQ ID NO:311) | CDR1: RASQSVSRYLA (SEQ ID NO:57) |
| | | CDR2: DASNRAT (SEQ ID NO:58) |
| | CDR2: SIYYSGSTYYNPSLKS (SEQ ID NO:54) | |
| | | CDR3: QQFDTWPPT (SEQ ID NO:59) |
| | CDR3: ARGSDRFHPYFDY (non-Kabat) (SEQ ID NO:55) or GSDRFHPYFDY (SEQ ID NO:312) | |
| ADI-29404 (F04) | QVQLQQWGAGLLKPSETLSLTCAV YGGSFSGYYWSWIRQPPGKGLEWIG EIDHSGSTNYNPSLKSRVTISVDTSK NQFSLKLSSVTAADTAVYYCARAR GPWSFDPWGQGTLVTVSS (SEQ ID NO:60) | DIQMTQSPSTLSASVGDRVTIT CRASQSISSWLAWYQQKPGKA PKLLIYKASSLESGVPSRFSGSG SGTEFTLTISSLQPDDFATYYCE QYDSYPTFGGGTKVEIK (SEQ ID NO:61) |
| ADI-28200 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCARR GRKASGSFYYYYGMDVWGQGTTV TVSS (SEQ ID NO:62) |  (SEQ ID NO:66) |
| | CDR1: GTFSSYAIS (non-Kabat) (SEQ ID NO:63) | CDR1: ESSQSLLNSGNQKNYLT (SEQ ID NO:67) |
| | CDR2: GIIPIFGTANYAQKFQG (SEQ ID NO:64) | CDR2: WASTRES (SEQ ID NO:68) |
| | CDR3: ARRGRKASGSFYYYYGMDV (non-Kabat) (SEQ ID NO:65) | CDR3: QNDYSYPYT (SEQ ID NO:69) |
| ADI-29379 (E79) | QVQLVQSGAEVKKPGASVKVSCKA SGYTFTSYYMHWVRQAPGQGLEW MGIINPSGGSTSYAQKFQGRVTMTR DTSTSTVYMELSSLRSEDTAVYYCA RGAPNYGDTTHDYYYMDVWGKGT TVTVSS (SEQ ID NO:70) | EIVMTQSPATLSVSPGERATLS CRASQSVSSNLAWYQQKPGQ APRLLIYGASTRATGIPARFSGS GSGTEFTLTISSLQSEDFAVYY CQQYDDWPFTFGGGTKVEIK (SEQ ID NO:74) |
| | | CDR1: RASQSVSSNLA (SEQ ID NO:75) |
| | CDR1: YTFTSYYMH (non-Kabat) (SEQ ID NO:71) or SYYMH (SEQ ID NO:313) | |
| | | CDR2: GASTRAT (SEQ ID NO:76) |
| | CDR2: IINPSGGSTSYAQKFQG (SEQ ID NO:72) CDR3: ARGAPNYGDTTHDYYYMDV (non-Kabat) (SEQ ID NO:73) or GAPNYGDTTHDYYYMDV (SEQ ID NO:314) | CDR3: QQYDDWPFT (SEQ ID NO:77) |
| ADI-29463 (F63) | QVQLVQSGAEVKKPGASVKVSCKA SGYTFTGYYMHWVRQAPGQGLEW MGWINPNSGGTNYAQKFQGRVTMT RDTSISTAYMELSRLRSDDTAVYYC ARDTGEYYDTDDHGMDVWGQGTT VTVSS (SEQ ID NO:78) | EIVLTQSPGTLSLSPGERATLSC RASQSVSSNLAWYQQKPGQAP RLLIYGASTRATGIPARFSGSGS GTEFTLTISSLQSEDFAVYYCQ QDDYWPPTFGGGTKVEIK (SEQ ID NO:82) |
| | | CDR1: RASQSVSSNLA (SEQ ID NO:83) |
| | CDR1: YTFTGYYMH (non-Kabat) (SEQ ID NO:79) or GYYMH (SEQ ID NO:315) | |
| | | CDR2: GASTRAT (SEQ ID NO:84) |
| | CDR2: WINPNSGGTNYAQKFQG (SEQ ID NO:80) | CDR3: QQDDYWPPT (SEQ ID NO:85) |
| | CDR3: ARDTGEYYDTDDHGMDV (non-Kabat) (SEQ ID NO:81) or DTGEYYDTDDHGMDV (SEQ ID NO:316) | |
| ADI-27744 (A44) | EVQLLESGGGLVQPGGSLRLSCAAS GFTFSSYAMSWVRQAPGKGLEWVS AISGSGGSTYYADSVKGRFTISRDNS KNTLYLQMNSLRAEDTAVYYCAKD GGYYDSGAGDYWGQGTLVTVSS (SEQ ID NO:86) | DIQMTQSPSSVSASVGDRVTIT CRASQGIDSWLAWYQQKPGK APKLLIYAASSLQSGVPSRFSG SGSGTDFTLTISSLQPEDFATYY CQQGVSYPRTFGGGTKVEIK (SEQ ID NO:90) |
| | CDR1: FTFSSYAMS (non-Kabat) (SEQ ID NO:87) or SYAMS (SEQ ID NO:317) CDR2: AISGSGGSTYYADSVKG (SEQ ID NO:88) | CDR1: RASQGIDSWLA (SEQ ID NO:91) |
| | | CDR2: AASSLQS (SEQ ID NO:92) |
| | CDR3: AKDGGYYDSGAGDY (non-Kabat) (SEQ ID NO:89) or DGGYYDSGAGDY (SEQ ID NO:318) | CDR3: QQGVSYPRT (SEQ ID NO:93) |
| ADI-27749 (A49) | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PMGAAAGWFDPWGQGTLVTVSS (SEQ ID NO:94) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN (SEQ ID NO:319) | CDR1: RASQGISSWLA (SEQ ID NO:99) |
| | CDR2: SISSSSSYIYYADSVKG (SEQ ID NO:96) | CDR2: AASSLQS (SEQ ID NO: 100) |
| | CDR3: ARGAPMGAAAGWFDP (non-Kabat) (SEQ ID NO:97) or GAPMGAAAGWFDP (SEQ ID NO:320) | CDR3: QQGVSFPRT (SEQ ID NO:101) |
| ADI-29378 (E78) | QVQLVQSGAEVKKPGASVKVSCKA SGYTFTSYYMHWVRQAPGQGLEW MGIINPSGGSTSYAQKFQGRVTMTR DTSTSTVYMELSSLRSEDTAVYYCA REGAGFAYGMDYYYMDVWGKGTT VTVSS (SEQ ID NO:102) | EIVLTQSPATLSLSPGERATLSC RASQSVSSYLAWYQQKPGQAP RLLIYDASNRATGIPARFSGSG SGTDFTLTISSLEPEDFAVYYC QQSDNWPFTFGGGTKVEIK (SEQ ID NO:106) |
| | | CDR1 (SEQ ID NO:107) - RASQSVSSYLA |
| | CDR1: YTFTSYYMH (non-Kabat) (SEQ ID NO:103) or SYYMH (SEQ ID NO:313) | |
| | | CDR2 (SEQ ID NO:108) - DASNRAT |
| | CDR2: IINPSGGSTSYAQKFQG (SEQ ID NO:104) | CDR3 (SEQ ID NO:109) - QQSDNWPFT |
| | CDR3: AREGAGFAYGMDYYYMDV (non-Kabat) (SEQ ID NO:105) or EGAGFAYGMDYYYMDV (SEQ ID NO:321) | |
| A49MI | EVQLVESGGGLVKPGGSLRLSCAAS GFTFSSYSMNWVRQAPGKGLEWVS SISSSSSYIYYADSVKGRFTISRDNAK | DIQMTQSPSSVSASVGDRVTIT CRASQGISSWLAWYQQKPGK APKLLIYAASSLQSGVPSRFSG |
| | NSLYLQMNSLRAEDTAVYYCARGA PIGAAAGWFDPWGQGTLVTVSS (SEQ ID NO:322) | SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN | CDR1: RASQGISSWLA (SEQ ID NO:99) |
| | (SEQ ID NO:319) | CDR2: AASSLQS (SEQ ID NO: 100) |
| | CDR2: SISSSSSYIYYADSVKG (SEQ ID NO:96) | CDR3: QQGVSFPRT (SEQ ID NO:101) |
| | CDR3: ARGAPIGAAAGWFDP (non-Kabat) (SEQ ID NO:323) or GAPIGAAAGWFDP (SEQ ID NO:324) | |
| A49MQ | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PQGAAAGWFDPWGQGTLVTVSS (SEQ ID NO:325) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN (SEQ ID NO:319) | CDR1: RASQGISSWLA (SEQ ID NO:99) |
| | | CDR2: AASSLQS (SEQ ID NO: 100) |
| | CDR2: SISSSSSYIYYADSVKG (SEQ ID NO:96) | CDR3: QQGVSFPRT (SEQ ID NO:101) |
| | CDR3: ARGAPQGAAAGWFDP (non-Kabat) (SEQ ID NO:326) or GAPQGAAAGWFDP (SEQ ID NO:327) | |
| A49ML | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PLGAAAGWFDPWGQGTLVTVSS (SEQ ID NO:328) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN (SEQ ID NO:319) | CDR1: RASQGISSWLA (SEQ ID NO:99) |
| | | CDR2: AASSLQS (SEQ ID NO: 100) |
| | CDR2: SISSSSSYIYYADSVKG (SEQ ID NO:96) | |
| | | CDR3: QQGVSFPRT (SEQ ID NO:101) |
| | CDR3: ARGAPLGAAAGWFDP (non-Kabat) (SEQ ID NO:329) or GAPLGAAAGWFDP (SEQ ID NO:330) | |
| A49MF | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PFGAAAGWFDPWGQGTLVTVSS (SEQ ID NO:331) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN (SEQ ID NO:319) | CDR1: RASQGISSWLA (SEQ ID NO:99) |
| | | CDR2: AASSLQS (SEQ ID NO: 100) |
| | CDR2: SISSSSSYIYYADSVKG (SEQ ID NO:96) | |
| | | CDR3: QQGVSFPRT (SEQ ID NO:101) |
| | CDR3: ARGAPFGAAAGWFDP (non-Kabat) (SEQ ID NO:332) or GAPFGAAAGWFDP (SEQ ID NO:333) | |
| A49MV | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PVGAAAGWFDPWGQGTLVTVSS (SEQ ID NO:334) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | CDR1: FTFSSYSMN (non-Kabat) (SEQ ID NO:95) or SYSMN (SEQ ID NO:319) CDR2: SISSSSSYIYYADSVKG (SEQ ID NO:96) | CDR1: RASQGISSWLA (SEQ ID NO:99) |
| | CDR3: ARGAPVGAAAGWFDP (non-Kabat) (SEQ ID NO:335) or GAPVGAAAGWFDP (SEQ ID NO:336) | CDR2: AASSLQS (SEQ ID NO: 100) |
| | | CDR3: QQGVSFPRT (SEQ ID NO:101) |
| A49-consensus | EVQLVESGGGLVKPGGSLRLSCAAS GFTFSSYSMNWVRQAPGKGLEWVS SISSSSSYIYYADSVKGRFTISRDNAK NSLYLQMNSLRAEDTAVYYCARGA PXGAAAGWFDPWGQGTLVTVSS, wherein X is M, L, I, V, Q, or F (SEQ ID NO:337) | DIQMTQSPSSVSASVGDRVTIT CRASQGISSWLAWYQQKPGK APKLLIYAASSLQSGVPSRFSG SGSGTDFTLTISSLQPEDFATYY CQQGVSFPRTFGGGTKVEIK (SEQ ID NO:98) |
| | | CDR1: RASQGISSWLA (SEQ ID NO:99) |
| | CDR1: FTFSSYSMN (non-Kabat) (SEQ ID NO:95) or SYSMN (SEQ ID NO:319) | |
| | | CDR2: AASSLQS (SEQ ID NO: 100) |
| | CDR2: SISSSSSYIYYADSVKG (SEQ ID NO:96) | CDR3: QQGVSFPRT (SEQ ID NO:101) |
| | CDR3: ARGAPXGAAAGWFDP, wherein X is M, L, I, V, Q, or F (non-Kabat) (SEQ ID NO:338) or GAPXGAAAGWFDP, wherein X is M, L, I, V, Q, or F (SEQ ID NO:339) | |

Alternatively, a heavy chain variable domain represented by SEQ ID NO:110 can be paired with a light chain variable domain represented by SEQ ID NO:111 to form an antigen-binding site that can bind to NKG2D, as illustrated in US 9,273,136.
SEQ ID NO:110 
QVQLVESGGGLVKPGGSLRLSCAASGFTFSSYGMHWVRQAPGKGLEWVAFIRYDGS
NKYYADSVKGRFTISRDNSKNTLYLQMNSLRAEDTAVYYCAKDRGLGDGTYFDYW
GQGTTVTVSS
SEQ ID NO:111

QSALTQPASVSGSPGQSITISCSGSSSNIGNNAVNWYQQLPGKAPKLLIYYDDLLPSG
VSDRFSGSKSGTSAFLAISGLQSEDEADYYCAAWDDSLNGPVFGGGTKLTVL

Alternatively, a heavy chain variable domain represented by SEQ ID NO:112 can be paired with a light chain variable domain represented by SEQ ID NO:113 to form an antigen-binding site that can bind to NKG2D, as illustrated in US 7,879,985.
SEQ ID NO:112 
QVHLQESGPGLVKPSETLSLTCTVSDDSISSYYWSWIRQPPGKGLEWIGHISYSGSAN
YNPSLKSRVTISVDTSKNQFSLKLSSVTAADTAVYYCANWDDAFNIWGQGTMVTVS
S
SEQ ID NO:113

EIVLTQSPGTLSLSPGERATLSCRASQSVSSSYLAWYQQKPGQAPRLLIYGASSRATGI
PDRFSGSGSGTDFTLTISRLEPEDFAVYYCQQYGSSPWTFGQGTKVEIK

### Tumor-associated antigen binding site

Tumor-associated antigen used herein means any antigen including but not limited to a protein, glycoprotein, ganglioside, carbohydrate, lipid that is associated with cancer. Such antigen can be expressed on malignant cells or in the tumor microenvironment such as on tumor-associated blood vessels, extracellular matrix, mesenchymal stroma, or immune infiltrates. For example, the tumor-associated antigen can include ANO1, BCMA, EpCAM, CAIX, CEA, CCR4, CD2, CD123, CD133, CD19, CD20, CD22, CD25, CD30, CD33, CD37, CD38, CD40, CD52, CD70, CLAUDIN-18.2, DLL3, EGFR/ERBB1, GD2, IGF1R, HER2, HER3/ERBB3, HER4/ERBB4, MUC1, cMET, SLAMF7, PSMA, mesothelin, MICA, MICB, TRAILR1, TRAILR2, TROP2, MAGE-A3, B7.1, B7.2, CTLA4, PD1, 5T4, GPNMB, FR-alpha, PAPP-A, FLT3, GPC3, CXCR4, ROR1, ROR2, HLA-E, PD-L1, VLA4, CD44, CD13, CD15, CD47, CLL1, CD81, CD23, CD79a, CD79b, CD80, CRLF2, SLAMF7, CD138, CA125, NaPi2b, Nectin4, ADAM8, ADAM9, SLC44A4, CA19-9, LILRB1, LILRB2, LILRB3, LILRB4, LILRB5, ULRA 1, LILRA2, LILRA3, ULRA4, LILRA5, and ULRA6, CCR8, CD7, CTLA4, CX3CR1, ENTPD1, HAVCR2, IL-1R2, PDCD1LG2, TIGIT, TNFRSF4, TNFRSF8, TNFRSF9, GEM, NT5E, TNFRSF18, MUC1, P-cadherin, Plexin-A1, TNFRSF10B, STEAP1, CDCP1, PTK7, Axl, erbB-3, EDNRB, Tyrp1, CD14, CD163, CSF3R, Siglec-9, ITGAM, VISTA, B7-H4 (VTCN1), CCR1, LRRC25, PTAFR, SIRPB1, TLR2, TLR4, CD300LB, ATP1A3, CCR5, MUC1 (or MUC1-C), Plexin-A1, TNFRSF10B, STEAP1, CDCP1, PTK7, AXL, EDNRB, OLR1, and TYRP1 expressed on cancer cells.

A tumor-associated antigen binding site can be developed to bind to any tumor-associated antigen. In some embodiments, the tumor-associated antigen binding site includes a heavy chain variable domain and a light chain variable domain, which can pair to bind to a tumor-associated antigen. In some embodiments, the heavy chain variable domain and the light chain variable domain are arranged in the Fab format. In some embodiments, the heavy chain variable domain and the light chain variable domain are fused together to from an scFv. Exemplary tumor-associated antigen binding sites are listed below.

**Table 2 lists peptide sequences of heavy chain variable domains and light chain variable domains that, in combination, can bind to BCMA.**

| Table 2 | | |
|---|---|---|
| Clones | Heavy chain variable domain peptide sequence | Light chain variable domain peptide sequence |
| 1 (US14/776, 649) | QVQLVQSGAEVKKPGASVKVSC KASGYSFPDYYINWVRQAPGQGL EWMGWIYFASGNSEYNQKFTGR VTMTRDTSSSTAYMELSSLRSEDT AVYFCASLYDYDWYFDVWGQGT MVTVSS (SEQ ID NO:114) | DIVMTQTPLSLSVTPGEPASISCKS SQSLVHSNGNTYLHWYLQKPGQ SPQLLIYKVSNRFSGVPDRFSGSG SGADFTLKISRVEAEDVGVYYCA ETSHVPWTFGQGTKLEIK (SEQ ID NO:118) |
| | | or |
| | CDR1(SEQ ID NO:115) - DYYIN | DIVMTQTPLSLSVTPGQPASISCK SSQSLVHSNGNTYLHWYLQKPG QSPQLLIYKVSNRFSGVPDRFSGS GSGTDFTLKISRVEAEDVGIYYCS QSSIYPWTFGQGTKLEIK (SEQ ID NO:119) |
| | CDR2 (SEQ ID NO:116) - WIYFASGNSEYNQKFTG | |
| | CDR3 (SEQ ID NO:117) - LYDYDWYFDV | |
| | | CDR1(SEQ ID NO:120) - KSSQSLVHSNGNTYLH |
| | | CDR2 (SEQ ID NO:121) - KVSNRFS |
| | | CDR3 - AETSHVPWT (SEQ ID NO:122) or SQSSIYPWT (SEQ ID NO:123) |
| 2 (PCT/US15 /64269) | QIQLVQSGPELKKPGETVKISCKA SGYTFTDYSINWVKRAPGKGLK WMGWINTETREPAYAYDFRGRF AFSLETSASTAYLQINNLKYEDTA TYFCALDYSYAMDYWGQGTSVT VSS (SEQ ID NO:124) | DIVLTQSPPSLAMSLGKRATISCR ASESVTILGSHLIHWYQQKPGQPP TLLIQLASNVQTGVPARFSGSGSR TDFTLTIDPVEEDDVAVYYCLQS RTIPRTFGGGTKLEIK (SEQ ID NO:128) |
| | | CDR1 (SEQ ID NO:129) - RASESVTILGSHLIH |
| | CDR1 (SEQ ID NO:125) - DYSIN | |
| | CDR2 (SEQ ID NO:126) - WINTETREPAYAYDFR | CDR2 (SEQ ID NO:130) - LASNVQT |
| | CDR3 (SEQ ID NO:127) - DYSYAMDY | CDR3 (SEQ ID NO:131) - LQSRTIPRT |
| 3 (US14/122, 391) | QVQLVQSGAEVKKPGSSVKVSCK ASGGTFSNYWMHWVRQAPGQGL EWMGATYRGHSDTYYNQKFKGR VTITADKSTSTAYMELSSLRSEDT AVYYCARGAIYNGYDVLDNWGQ GTLVTVSS (SEQ ID NO:132) | DIQMTQSPSSLSASVGDRVTITCS ASQDISNYLNWYQQKPGKAPKL LIYYTSNLHSGVPSRFSGSGSGTD FTLTISSLQPEDFATYYCQQYRKL PWTFGQGTKLEIKR (SEQ ID NO:136) |
| | | CDR1 (SEQ ID NO:137) - SASQDISNYLN |
| | CDR1 (SEQ ID NO:133) - NYWMH | |
| | CDR2 (SEQ ID NO:134) - ATYRGHSDTYYNQKFKG | CDR2 (SEQ ID NO:138) - YTSNLHS |
| | CDR3 (SEQ ID NO:135) - GAIYNGYDVLDN | CDR3 (SEQ ID NO:139) - QQYRKLPWT |
| 4 (US201700 51068) | QLQLQESGPGLVKPSETLSLTCTV SGGSISSSSYFWGWIRQPPGKGLE WIGSIYYSGITYYNPSLKSRVTISV DTSKNQFSLKLSSVTAADTAVYY CARHDGATAGLFDYWGQGTLVT VSS (SEQ ID NO:140) | SYVLTQPPSVSVAPGQTARITCGG NNIGSKSVHWYQQPPGQAPVVV VYDDSDRPSGIPER FSGSNSGNTA TLTISRVEAGDEAVYYCQVWDSS SDHVVFGGGTKLTVL (SEQ ID |
| | | NO:144) |
| | CDR1: SSSYFWG (SEQ ID NO:141) | |
| | CDR2: SIYYSGITYYNPSLKS (SEQ ID NO:142) | CDR1: GGNNIGSKSVH (SEQ ID NO:145) |
| | CDR3: HDGATAGLFDY (SEQ ID NO:143) | CDR2: DDSDRPS (SEQ ID NO:146) |
| | | CDR3: QVWDSSSDHVV (SEQ ID NO:147) |
| 5 (WO20170 21450) | EVQLLESGGGLVQPGGSLRLSCA ASGFTFSDNAMGWVRQAPGKGL EWVSAISGPGSSTYYADSVKGRF TISRDNSKNTLYLQMNSLRAEDT AVYYCAKVLGWFDYWGQGTLV TVSS (SEQ ID NO:148) | EIVLTQSPGTLSLSPGERATLSCR ASQSVSDEYLSWYQQKPGQAPR LLIHSASTRATGIPDRFSGSGSGT DFTLAISRLEPEDFAVYYCQQYG YPPDFTFGQGTKVEIK (SEQ ID NO:152) |
| | CDR1: RASQSVSDEYLS (SEQ ID NO:149) | CDR1: RASQSVSDEYLS (SEQ ID NO:153) |
| | CDR2: SASTRAT (SEQ ID NO:150) | CDR2: SASTRAT (SEQ ID NO:154) |
| | CDR3: QQYGYPPDFT (SEQ ID NO:151) | CDR3: QQYGYPPDFT (SEQ ID NO:155) |

Alternatively, a BCMA-binding domain can include a heavy chain variable domain and light chain variable domain as listed below in EM-801 and EM-901.
EM-801 heavy chain variable domain (SEQ ID NO:157): 
EVQLLESGGGLVQPGGSLRLSCAASGFTFSSYAMSWVRQAPGKGLEWVSAISGSGG
CDR1 CDR2
STYYADSVKGRFTISRDNSKNTLYLQMNSLRAEDTAVYYCAKVLGWFDYWGQGTL
CDR3
VTVSS
EM-801 light chain variable domain (SEQ ID NO:158): 
EIVLTQSPGTLSLSPGERATLSCRASQSVSSSYLAWYQQKPGQAPRLLIYGASSRATGI
CDR1 CDR2
 
PDRFSGSGSGTDFTLTISRLEPEDFAVYYCQQYGYPPDFTFGQGTKVEIK
CDR3
EM-901 heavy chain variable domain (SEQ ID NO:159) 
EVQLLESGGGLVQPGGSLRLSCAASGFTFSDNAMGWVRQAPGKGLEWVSAISGPGS
CDR1 CDR2
STYYADSVKGRFTISRDNSKNTLYLQMNSLRAEDTAVYYCAKVLGWFDYWGQGTL
CDR3
VTVSS
EM-901 light chain variable domain (SEQ ID NO:160)

EIVLTQSPGTLSLSPGERATLSCRASQSVSDEYLSWYQQKPGQAPRLLIHSASTRATGI
CDR1
CDR2
PDRFSGSGSGTDFTLAISRLEPEDFAVYYCQQYGYPPDFTFGQGTKVEIK
CDR3

Alternatively, novel antigen-binding sites that can bind to BCMA can be identified by screening for binding to the amino acid sequence defined by SEQ ID NO:156.

SEQ ID NO:156
MLQMAGQCSQNEYFDSLLHACIPCQLRCSSNTPPLTCQRYCNASVTNSVKGTNAIL
WTCLGLSLIISLAVFVLMFLLRKINSEPLKDEFKNTGSGLLGMANIDLEKSRTGDEIILP
RGLEYTVEECTCEDCIKSKPKVDSDHCFPLPAMEEGATILVTTKTNDYCKSLPAALSA
TEIEKSISAR

Table 3 lists peptide sequences of heavy chain variable domains and light chain variable domains that, in combination, can bind to CD33. The CD33-binding domains can vary in their binding affinity to CD33.

| Table 3 | | |
|---|---|---|
| | Heavy chain variable domain peptide sequence | Light chain variable domain peptide sequence |
| ADI-10159 [Ab1] (G59) | EVQLVESGGGLVQPGGSLRLSC AASGFTFSSYGMSWVRQAPGK GLEWVANIKQDGSEKYYVDSV | DIQMTQSPSTLSASVGDRVTITCR ASQSISSWLAWYQQKPGKAPKL LIYDASSLESGVPSRFSGSGSGTE |
| | KGRFTISRDNAKNSLYLQMNSL RAEDTAVYYCAREGGPYYDSS GYFVYYGMDVWGQGTTVTVSS [SEQ ID NO:161] | FTLTISSLQPDDFATYYCQQYESF PTFGGGTKVEIK [SEQ ID NO165] |
| | | CDR1: RASQSISSWLA [SEQ ID NO:166] |
| | CDR1: FTFSSYGMS [SEQ ID NO:162] | CDR2: DASSLES [SEQ ID NO:167] |
| | CDR2: NIKQDGSEKYYVDSVKG [SEQ ID NO:163] | CDR3: QQYESFPT [SEQ ID NO:168] |
| | CDR3: AREGGPYYDSSGYFVYYGMDV [SEQ ID NO:164] | |
| ADI-10177 [Ab2] | EVQLVESGGGLVQPGGSLRLSC AASGFTFSSYWMSWVRQAPGK GLEWVANIKQDGSEKYYVDSV KGRFTISRDNAKNSLYLQMNSL RAEDTAVYYCARPLNAGELDV WGQGTMVTVSS [SEQ ID NO:169] | DIQMTQSPSTLSASVGDRVTITCR ASQSISSWLAWYQQKPGKAPKL LIYEASSLESGVPSRFSGSGSGTE FTLTISSLQPDDFATYYCQQLESY PLTFGGGTKVEIK [SEQ ID NO:173] |
| | | CDR1: RASQSISSWLA [SEQ ID NO:174] |
| | CDR1: FTFSSYWMS [SEQ ID NO:170] | |
| | | CDR2: EASSLES [SEQ ID NO:175] |
| | CDR2: NIKQDGSEKYYVDSVKG [SEQ ID NO:171] | CDR3: QQLESYPLT [SEQ ID NO:176] |
| | CDR3: ARPLNAGELDV [SEQ ID NO: 172] | |
| ADI-11776 [Ab3] (H76) | EVQLLESGGGLVQPGGSLRLSC AASGFTFSKYTMSWVRQAPGK GLEWVSAIVGSGESTYFADSVK GRFTISRDNSKNTLYLQMNSLR AEDTAVYYCAREGGPYYDSSG YFVYYGMDVWGQGTTVTVSS [SEQ ID NO:177] | DIQMTQSPSTLSASVGDRVTITCR ASQSISSWLAWYQQKPGKAPKL LIYKASSLESGVPSRFSGSGSGTE FTLTISSLQPDDFATYYCQQYDD LPTFGGGTKVEIK [SEQ ID NO:181] |
| | | CDR1: RASQSISSWLA [SEQ ID NO:182] |
| | CDR1: FTFSKYTMS [SEQ ID NO: 178] | |
| | | CDR2: KASSLES [SEQ ID NO:183] |
| | CDR2: AIVGSGESTYFADSVKG [SEQ ID NO:179] | CDR3: QQYDDLPT [SEQ ID NO:184] |
| | CDR3: AREGGPYYDSSGYFVYYGMDV [SEQ ID NO:180] | |
| ADI-11801 [Ab4] | QVQLVQSGAEVKKPGASVKVS CKASGYTFSDYYMHWVRQAPG QGLEWMGMINPSWGSTSYAQK FQGRVTMTRDTSTSTVYMELSS LRSEDTAVYYCAREAADGFVGE RYFDLWGRGTLVTVSS [SEQ ID NO:185] | DIVMTQSPLSLPVTPGEPASISCR SSQSLLYSNGYNYLDWYLQKPG QSPQLLIYLGSNRASGVPDRFSGS GSGTDFTLKISRVEAEDVGVYYC MQDVALPITFGGGTKVEIK [SEQ ID NO:189] |
| | | CDR1: RSSQSLLYSNGYNYLD [SEQ ID NO:190] |
| | CDR1: YTFSDYYMH [SEQ ID NO:186] | |
| | | CDR2: LGSNRAS [SEQ ID NO:191] |
| | CDR2: MINPSWGSTSYAQKFQG [SEQ ID NO:187] | CDR3: MQDVALPIT [SEQ ID NO:192] |
| | CDR3: AREAADGFVGERYFDL [SEQ ID NO:188] | |
| ADI-11807 [Ab5] (I07) | EVQLVESGGGLVQPGGSLRLSC AASGFTFGSYWMSWVRQAPGK GLEWVATIKQDGSEKSYVDSVK GRFTISRDNAKNSLYLQMNSLR AEDTAVYYCARPLNAGELDVW GQGTMVTVSS [SEQ ID NO:193] | DIQMTQSPSTLSASVGDRVTITCR ASQSISSWLAWYQQKPGKAPKL LIYEASSLESGVPSRFSGSGSGTE FTLTISSLQPDDFATYYCQQSQSY PPITFGGGTKVEIK [SEQ ID NO:197] |
| | CDR1: FTFGSYWMS [SEQ ID NO:194] | CDR1: RASQSISSWLA [SEQ ID NO:198] |
| | CDR2: TIKQDGSEKSYVDSVKG [SEQ ID NO:195] | CDR2: EASSLES [SEQ ID NO:199] |
| | CDR3: ARPLNAGELDV [SEQ ID NO:196] | CDR3: QQSQSYPPIT [SEQ ID NO:200] |
| ADI-11809 [Ab6] | EVQLVESGGGLVQPGGSLRLSC AASGFTFPSYWMSWVRQAPGK GLEWVATIKRDGSEKGYVDSV KGRFTISRDNAKNSLYLQMNSL RAEDTAVYYCARPLNAGELDV WGQGTMVTVSS [SEQ ID NO:201] | DIQMTQSPSTLSASVGDRVTITCR ASQSISSWLAWYQQKPGKAPKL LIYEASSLESGVPSRFSGSGSGTE FTLTISSLQPDDFATYYCQQSQSY PPITFGGGTKVEIK [SEQ ID NO:205] |
| | | CDR1: RASQSISSWLA [SEQ ID NO:206] |
| | CDR1: FTFPSYWMS [SEQ ID NO:202] | |
| | | CDR2: EASSLES [SEQ ID NO:207] |
| | CDR2: TIKRDGSEKGYVDSVKG [SEQ ID NO:203] | CDR3: QQSQSYPPIT [SEQ ID NO:208] |
| | CDR3: ARPLNAGELDV [SEQ ID NO:204] | |
| ADI-11815 [Ab7] | QVQLVQSGAEVKKPGASVKVS CKASGYTFGTYYMHWVRQAPG QGLEWMGIINPSRGSTVYAQKF QGRVTMTRDTSTSTVYMELSSL RSEDTAVYYCARGAGYDDEDM DVWGKGTTVTVSS [SEQ ID NO:209] | DIQMTQSPSSVSASVGDRVTITC RASQGIDSWLAWYQQKPGKAPK LLIYAASSLQSGVPSRFSGSGSGT DFTLTISSLQPEDFATYYCQQAHS YPLTFGGGTKVEIK [SEQ ID NO:213] |
| | | CDR1: RASQGIDSWLA [SEQ ID NO:214] |
| | CDR1: YTFGTYYMH [SEQ ID NO:210] | |
| | | CDR2: AASSLQS [SEQ ID NO:215] |
| | CDR2: IINPSRGSTVYAQKFQG [SEQ ID NO:211] | CDR3: QQAHSYPLT [SEQ ID NO:216] |
| | CDR3: ARGAGYDDEDMDV [SEQ ID NO:212] | |
| ADI-11819 [Ab8] | EVQLVESGGGLVKPGGSLRLSC AASGFTFSSYAMSWVRQAPGK | DIQMTQSPSTLSASVGDRVTITCR ASNSISSWLAWYQQKPGKAPKL |
| | GLEWVSSISSSSEGIYYADSVKG RFTISRDNAKNSLYLQMNSLRA EDTAVYYCAREGGPYYDSSGYF VYYGMDVWGQGTTVTVSS [SEQ ID NO:217] | LIYEASSTKSGVPSRFSGSGSGTE FTLTISSLQPDDFATYYCQQYDD LPTFGGGTKVEIK [SEQ ID NO:221] |
| | | CDR1: RASNSISSWLA [SEQ ID NO:222] |
| | CDR1: FTFSSYAMS [SEQ ID NO:218] | |
| | | CDR2: EASSTKS [SEQ ID NO:223] |
| | CDR2: SISSSSEGIYYADSVKG [SEQ ID NO:219] | CDR3: QQYDDLPT [SEQ ID NO:224] |
| | CDR3: AREGGPYYDSSGYFVYYGMDV [SEQ ID NO:220] | |
| ADI-11830 [Ab9] | EVQLVESGGGLVQPGGSLRLSC AASGFTFSSYWMSWVRQAPGK GLEWVANINTDGSEVYYVDSV KGRFTISRDNAKNSLYLQMNSL RAEDTAVYYCARDVGPGIAYQ GHFDYWGQGTLVTVSS [SEQ ID NO:225] | DIQMTQSPSSLSASVGDRVTITCR ASQVIYSYLNWYQQKPGKAPKL LIYAASSLKSGVPSRFSGSGSGTD FTLTISSLQPEDFATYYCQQVYD TPLTFGGGTKVEIK [SEQ ID NO:229] |
| | | CDR1: RASQVIYSYLN [SEQ ID NO:230] |
| | CDR1: FTFSSYWMS [SEQ ID NO:226] | |
| | | CDR2: AASSLKS [SEQ ID NO:231] |
| | CDR2: NINTDGSEVYYVDSVKG [SEQ ID NO:227] | CDR3: QQVYDTPLT [SEQ ID NO:232] |
| | CDR3: ARDVGPGIAYQGHFDY [SEQ ID NO:228] | |
| ADI-11835 [Ab10] (I35) | QLQLQESGPGLVKPSETLSLTCT VSGGSISSTDYYWGWIRQPPGK GLEWIGSIGYSGTYYNPSLKSRV TISVDTSKNQFSLKLSSVTAADT AVYYCARETAHDVHGMDVWG QGTTVTVSS [SEQ ID NO:233] | EIVLTQSPATLSLSPGERATLSCR ASHSVYSYLAWYQQKPGQAPRL LIYDASNRATGIPARFSGSGSGTD FTLTISSLEPEDFAVYYCQQYDN LPTFGGGTKVEIK [SEQ ID NO:237] |
| | CDR1: GSISSTDYYWG [SEQ ID NO:234] | CDR1: RASHSVYSYLA [SEQ ID NO:238] |
| | CDR2: SIGYSGTYYNPSLKS [SEQ ID NO:235] | CDR2: DASNRAT [SEQ ID NO:239] |
| | CDR3: ARETAHDVHGMDV [SEQ ID NO:236] | CDR3: QQYDNLPT [SEQ ID NO:240] |
| Lintuzumab | QVQLVQSGAEVKKPGSSVKVSC KASGYTFTDYNMHWVRQAPGQ GLEWIGYIYPYNGGTGYNQKFK SKATITADESTNTAYMELSSLRS EDTAVYYCARGRPAMDYWGQ GTLVTVSS (SEQ ID NO:241) | DIQMTQSPSSLSASVGDRVTITCR ASESVDNYGISFMNWFQQKPGK APKLLIYAASNQGSGVPSRFSGS GSGTDFTLTISSLQPDDFATYYCQ QSKEVPWTFGQGTKVEIK (SEQ ID NO:245) |
| | | CDR1(SEQ ID NO:246) - ESVDNYGISFMN |
| | CDR1 (SEQ ID NO:242) - GYTFTDY | |
| | | CDR2 (SEQ ID NO:247) - AASNQGS |
| | CDR2 (SEQ ID NO:243) - YIYPYNGGTG | |
| | | CDR3 (SEQ ID NO:248) - QQSKEVPWT |
| | CDR3 (SEQ ID NO:244) - GRPAMDY | |
| Gemtuzumab |  (SEQ ID NO:249) |  (SEQ ID NO:253) |
| | CDR1 (SEQ ID NO:250) - GYTITDS | CDR1 (SEQ ID NO:254) - ESLDNYGIRFLT |
| | CDR2 (SEQ ID NO:251) - YIYPYNGGTD | CDR2 (SEQ ID NO:255) - AASNQGS |
| | CDR3 (SEQ ID NO:252) - GNPWLAY | CDR3 (SEQ ID NO:256) - QQTKEVPWS |
| anti-CD33 (US 7,557,189) | QVQLQQPGAEVVKPGASVKMS CKASGYTFTSYYIHWIKQTPGQ GLEWVGVIYPGNDDISYNQKFQ GKATLTADKSSTTAYMQLSSLT SEDSAVYYCAREVRLRYFDVW GQGTTVTVSS (SEQ ID NO:257) | EIVLTQSPGSLAVSPGERVTMSC KSSQSVFFSSSQKNYLAWYQQIP GQSPRLLIYWASTRESGVPDRFT GSGSGTDFTLTISSVQPEDLAIYY CHQYLSSRTFGQGTKLEIKR (SEQ ID NO:261) |
| | | CDR1 (SEQ ID NO:262) - QSVFFSSSQKNYLA |
| | CDR1 (SEQ ID NO:258) - GYTFTSY | |
| | | CDR2 (SEQ ID NO:263) - WASTRES |
| | CDR2 (SEQ ID NO:259) - YPGNDD | |
| | | CDR3 (SEQ ID NO:264) - HQYLSSRT |
| | CDR3 (SEQ ID NO:260) - EVRLRYFDV | |
| vadastuximab (US 13/804,227) | QVQLVQSGAEVKKPGASVKVS CKASGYTFTNYDINWVRQAPG QGLEWIGWIYPGDGSTKYNEKF KAKATLTADTSTSTAYMELRSL RSDDTAVYYCASGYEDAMDY WGQGTTVTVSSA (SEQ ID NO:265) | DIQMTQSPSSLSASVGDRVTINC KASQDINSYLSWFQQKPGKAPKT LIYRANRLVDGVPSRFSGSGSGQ DYTLTISSLQPEDFATYYCLQYD EFPLTFGGGTKVEIKR (SEQ ID NO:269) |
| | | CDR1 (SEQ ID NO:270): QDINSYLS |
| | CDR1 (SEQ ID NO:266): GYTFTNY | |
| | | CDR2 (SEQ ID NO:271): RANRLVD |
| | CDR2 (SEQ ID NO:267): YPGDGS | |
| | | CDR3 (SEQ ID NO:272): LQYDEFPLT |
| | CDR3 (SEQ ID NO:268): GYEDAMDY | |

Alternatively, novel antigen-binding sites that can bind to CD33 can be identified by screening for binding to the amino acid sequence defined by SEQ ID NO:273.

SEQ ID NO:273
MPLLLLLPLLWAGALAMDPNFWLQVQESVTVQEGLCVLVPCTFFHPIPYYDKNSPV
HGYWFREGAIISRDSPVATNKLDQEVQEETQGRFRLLGDPSRNNCSLSIVDARRRDN
GSYFFRMERGSTKYSYKSPQLSVHVTDLTHRPKILIPGTLEPGHSKNLTCSVSWACEQ
GTPPIFSWLSAAPTSLGPRTTHSSVLIITPRPQDHGTNLTCQVKFAGAGVTTERTIQLN
VTYVPQNPTTGIFPGDGSGKQETRAGVVHGAIGGAGVTALLALCLCLIFFIVKTHRRK
AARTAVGRNDTHPTTGSASPKHQKKSKLHGPTETSSCSGAAPTVEMDEELHYASLN
FHGMNPSKDTSTEYSEVRTQ

**Table 4 lists peptide sequences of heavy chain variable domains and light chain variable domains that, in combination, can bind to HER2.**

| Table 4 | | |
|---|---|---|
| Clones | Heavy chain variable domain amino acid sequence | Light chain variable domain amino acid sequence |
| Trastuzumab |  (SEQ ID NO:274) |  (SEQ ID NO:278) |
| | | CDR1(SEQ ID NO:279) - QDVNTAVA |
| | CDR1(SEQ ID NO:275) - GFNIKDT | |
| | CDR2 (SEQ ID NO:276) - YPTNGY | CDR2 (SEQ ID NO:280) - SASFLYS |
| | CDR3 (SEQ ID NO:277) - WGGDGFYAMDY | |
| | | CDR3 (SEQ ID NO:281) - QQHYTTPPT |
| Pertuzumab | EVQLVESGGGLVQPGGSLRLSCA ASGFTFTDYTMDWVRQAPGKGLE WVADVNPNSGGSIYNQRFKGRFT LSVDRSKNTLYLQMNSLRAEDTA VYYCARNLGPSFYFDYWGQGTLV TVSSA | DIQMTQSPSSLSASVGDRVTITC KASQDVSIGVAWYQQKPGKAP KLLIYSASYRYTGVPSRFSGSGS GTDFTLTISSLQPEDFATYYCQQ YYIYPYTFGQGTKVEIKR (SEQ ID NO:286) |
| | (SEQ ID NO:282) | |
| | CDR1 (SEQ ID NO:283) - GFTFTDY | CDR1 (SEQ ID NO:287) - QDVSIGVA |
| | CDR2 (SEQ ID NO:284) - NPNSGG | CDR2 (SEQ ID NO:288) - SASYRYT |
| | CDR3 (SEQ ID NO:285) - NLGPSFYFDY | |
| | | CDR3 (SEQ ID NO:289) - QQYYIYPYT |
| **MGAH22** (US 8,802,093) | QVQLQQSGPELVKPGASLKLSCTA SGFNIKDTYIHWVKQRPEQGLEWI GRIYPTNGYTRYDPKFQDKATITA DTSSNTAYLQVSRLTSEDTAVYYC SRWGGDGFYAMDYWGQGASVTV SSA (SEQ ID NO:290) | DIVMTQSHKFMSTSVGDRVSIT CKASQDVNTAVAWYQQKPGH SPKLLIYSASFRYTGVPDRFTGS RSGTDFTFTISSVQAEDLAVYY CQQHYTTPPTFGGGTKVEIKR (SEQ ID NO:294) |
| | | CDR1 (SEQ ID NO:295) - QDVNTAVA |
| | CDR1 (SEQ ID NO:291) - GFNIKDT | |
| | CDR2 (SEQ ID NO:292) - YPTNGY | CDR2 (SEQ ID NO:296) - SASFRYT |
| | CDR3 (SEQ ID NO:293) - WGGDGFYAMDY | |
| | | CDR3 (SEQ ID NO:297) - QQHYTTPPT |

Alternatively, novel antigen-binding sites that can bind to HER2 can be identified by screening for binding to the amino acid sequence defined by SEQ ID NO:298.
























 (SEQ ID NO:298).

An antigen-binding TCR fragment can be developed to bind a tumor-associated antigen peptide presented by an MHC. In some embodiments, the antigen-binding TCR fragment includes an alpha chain variable domain and a beta chain variable domain, which can pair to bind to a TAA peptide presented by an MHC. In some embodiments, the alpha chain variable domain and the beta chain variable domain are arranged in the extracellular TCR fragment format. In some embodiments, the alpha chain variable domain and the beta chain variable domain are fused together to from an scTCR fragment.

Non-limiting examples of proteins that can be processed into TCR-targetable TAA peptides include tissue differentiation antigens (e.g., MART-1, gp100, CEA, CD19, and tyrosinase), tumor germline antigens (e.g., NY-ESO-1, MAGE-A1, MAGE-A3, MAGE-A4, MAGE-A12, MAGE-C2, BAGE1, GAGE1, CTAG1, CTAG2, XAGE-1B, and SSX2), normal proteins overexpressed by cancer cells *(e.g.,* hTERT, EGFR, ERBB2, WT1, MUC1, and mesothelin), viral proteins (e.g., HPV, EBV, and MCC), and tumor-specific mutated antigens. Exemplary tumor-associated antigen binding sites are listed below.

Table 5 lists peptide sequences of TCR targets and corresponding TCR alpha chain and beta chain sequences.

| Table 5 | | |
|---|---|---|
| Target protein and MHC-presented peptide | Extracellular TCRα fragment | Extracellular TCR-beta fragment |
| | α chain variable domain (Vα) | β chain variable domain (Vβ) |
| | α chain CDR3 (CDR3α) | β chain CDR (CDR3β) |
| ELAVL4 (UniProt ID P26378) | KEVEQNSGPLSVPEGAIASL NCTYSDRGSQSFFWYRQYS GKSPELIMSIYSNGDKEDGRF TAQLNKASQYVSLLIRDSQP SDSATYLCAVTTDSWGKLQ FGAGTQVVVTPDIQNPDPAV YQLRDSKSSDKSVCLFTDFD SQTNVSQSKDSDVYITDKTV LDMRSMDFKSNSAVAWSNK SDFACANAFNNSIIPEDTFFPS (SEQ ID NO:349) | NAGVTQTPKFQVLKTGQSMTLQ CAQDMNHEYMSWYRQDPGMG LRLIHYSVGAGITDQGEVPNGYN VSRSTTEDFPLRLLSAAPSQTSVY FCASRPGLAGGRPEQYFGPGTRL TVTEDLKNVFPPEVAVFEPSEAEI SHTQKATLVCLATGFYPDHVELS WWVNGKEVHSGVSTDPQPLKE QPALNDSRYALSSRLRVSATFW QDPRNHFRCQVQFYGLSENDEW TQDRAKPVTQIVSAEAWGRAD (SEQ ID NO:350) |
| LGYGFVNYI (SEQ ID NO:425) presented by HLA-A*02:01:48 | | |
| | Vα: | Vβ: |
| | KEVEQNSGPLSVPEGAIASL NCTYSDRGSQSFFWYRQYS GKSPELIMSIYSNGDKEDGRF TAQLNKASQYVSLLIRDSQP SDSATYLCAVTTDSWGKLQ FGAGTQVVVTPDIQNP (SEQ ID NO:351) | NAGVTQTPKFQVLKTGQSMTLQ CAQDMNHEYMSWYRQDPGMG LRLIHYSVGAGITDQGEVPNGYN VSRSTTEDFPLRLLSAAPSQTSVY FCASRPGLAGGRPEQYFGPGTRL TVTEDLKNVF (SEQ ID NO:352) |
| | CDR3α: CAVTTDSWGKLQF (SEQ ID NO:353) | CDR3β: CASRPGLAGGRPEQYF (SEQ ID NO:354) |
| Insulin (UniProt ID P01308) | EVEQDPGPLSVPEGAIVSLNC TYSNSAFQYFMWYRQYSRK GPELLMYTYSSGNKEDGRFT AQVDKSSKYISLFIRDSQPSD SATYLCAMRGDSSYKLIFGS GTRLLVRPDIQNPDPAVYQL RDSKSSDKSVCLFTDFDSQT NVSQSKDSDVYITDKCVLD MRSMDFKSNSAVAWSNKSD FACANAFNNSIIPEDTFFPS (SEQ ID NO:355) | AGVIQSPRHEVTEMGQQVTLRC KPISGHDYLFWYRQTMMRGLEL LIYFNNNVPIDDSGMPEDRFSAK MPNASFSTLKIQPSEPRDSAVYF CASSLWEKLAKNIQYFGAGTRLS VLEDLKNVFPPEVAVFEPSEAEIS HTQKATLVCLATGFYPDHVELS WWVNGKEVHSGVCTDPQPLKE QPALNDSRYALSSRLRVSATFW QDPRNHFRCQVQFYGLSENDEW TQDRAKPVTQIVSAEAWGRAD (SEQ ID NO:356) |
| ALWGPDPAAA (SEQ ID NO:426) presented by HLA-A*02:01:48 | | |
| | Vα: | Vβ: |
| | EVEQDPGPLSVPEGAIVSLNC TYSNSAFQYFMWYRQYSRK GPELLMYTYSSGNKEDGRFT AQVDKSSKYISLFIRDSQPSD | AGVIQSPRHEVTEMGQQVTLRC KPISGHDYLFWYRQTMMRGLEL LIYFNNNVPIDDSGMPEDRFSAK |
| | SATYLCAMRGDSSYKLIFGS GTRLLVRPDIQNP (SEQ ID NO:357) | MPNASFSTLKIQPSEPRDSAVYF CASSLWEKLAKNIQYFGAGTRLS VLEDLKNVF (SEQ ID NO:358) |
| | CDR3α: CAMRGDSSYKLIF (SEQ ID NO:359) | CDR3β: CASSLWEKLAKNIQYF (SEQ ID NO:360) |
| TERT (UniProt ID O14746) | IQVEQSPPDLILQEGANSTLR CNFSDSVNNLWWFHQNPW GQLINLFYIPSGTKQNGRLSA TTVATERYSLLYISSSQTTDS GVYFCAVDSATALPYGYIFG TGTRLKVLANIQNPDPAVYQ LRDSKSSDKSVCLFTDFDSQ TNVSQSKDSDVYITDKCVLD MRSMDFKSNSAVAWSNKSD FACANAFNNSIIPEDTFFPS (SEQ ID NO:361) | AGVTQTPKFQVLKTGQSMTLQC AQDMNHEYMSWYRQDPGMGL RLIHYSIHPEYTDQGEVPNGYNV SRSTTEDFPLRLLSAAPSQTSVYF CASSYQGTEAFFGQGTRLTVVE DLNKVFPPEVAVFEPSEAEISHTQ KATLVCLATGFYPDHVELSWWV NGKEVHSGVCTDPQPLKEQPAL NDSRYALSSRLRVSATFWQDPR NHFRCQVQFYGLSENDEWTQDR AKPVTQIVSAEAWGRAD (SEQ ID NO:362) |
| ILAKFLHWL (SEQ ID NO:340) presented by HLA-A*02:01:48 | | |
| | Vα: | Vβ: |
| | IQVEQSPPDLILQEGANSTLR CNFSDSVNNLWWFHQNPW GQLINLFYIPSGTKQNGRLSA TTVATERYSLLYISSSQTTDS GVYFCAVDSATALPYGYIFG TGTRLKVLANIQNP (SEQ ID NO:363) | AGVTQTPKFQVLKTGQSMTLQC AQDMNHEYMSWYRQDPGMGL RLIHYSIHPEYTDQGEVPNGYNV SRSTTEDFPLRLLSAAPSQTSVYF CASSYQGTEAFFGQGTRLTVVE DLNKVF (SEQ ID NO:364) |
| | CDR3α: CAVDSATALPYGYIF (SEQ ID NO:365) | CDR3β: CASSYQGTEAFF (SEQ ID NO:366) |
| ERBB2 (UniProt ID P04626) | EVEQDPGPLSVPEGAIVSLNC TYSNSAFQYFMWYRQYSRK GPELLMYTYSSGNKEDGRFT AQVDKSSKYISLFIRDSQPSD SATYLCAMSLYYGGSQGNLI FGKGTKLSVKPDPAVYQLR DSKSSDKSVCLFTDFDSQTN VSQSKDSDVYITDKCVLDM RSMDFKSNSAVAWSNKSDF ACANAFNNSIIPEDTFFPS (SEQ ID NO:428) | AGVAQSPRYKIIEKRQSVAFWCN PISGHATLYWYQQILGQGPKLLI QFQNNGVVDDSQLPKDRFSAER LKGVDSTLKIQPAKLEDSAVYLC ASSLEIFGGIADTDTQYFGPGTRL TVLEDLKNVFPPEVAVFEPSEAEI SHTQKATLVCLATGFYPDHVELS WWVNGKEVHSGVCTDPQPLKE QPALNDSRYALSSRLRVSATFW QDPRNHFRCQVQFYGLSENDEW TQDRAKPVTQIVSAEAWGRAD (SEQ ID NO:429) |
| KIFGSLAFL (SEQ ID NO:341) presented by HLA-A*02 | | |
| | Vα: | Vβ: |
| | EVEQDPGPLSVPEGAIVSLNC TYSNSAFQYFMWYRQYSRK GPELLMYTYSSGNKEDGRFT | AGVAQSPRYKIIEKRQSVAFWCN PISGHATLYWYQQILGQGPKLLI |
| | AQVDKSSKYISLFIRDSQPSD SATYLCAMSLYYGGSQGNLI FGKGTKLSVKP (SEQ ID NO:430) | QFQNNGVVDDSQLPKDRFSAER LKGVDSTLKIQPAKLEDSAVYLC ASSLEIFGGIADTDTQYF GPGTRLTVLEDLKNVF (SEQ ID NO:431) |
| | CDR3α: CAMSLYYGGSQGNLIF (SEQ ID NO:367) | CDR3β: CASSLEIFGGIADTDTQYF (SEQ ID NO:368) |
| WT1 (UniProt ID P19544) | EVEQNSGPLSVPEGAIASLNC TYSDRGSQSFFWYRQYSGKS PELIMFIYSNGDKEDGRFTA QLNKASQYVSLLIRDSQPSD SATYLCAVNDQGGGADGLT FGKGTHLIIQPDPAVYQLRDS KSSDKSVCLFTDFDSQTNVS QSKDSDVYITDKCVLDMRS MDFKSNSAVAWSNKSDFAC ANAFNNSIIPEDTFFPS (SEQ ID NO:432) | NAGVTQTPKFRVLKTGQSMTLL CAQDMNHEYMYWYRQDPGMG LRLIHYSVGEGTTAKGEVPDGY NVSRLKKQNFLLGLESAAPSQTS VYFCASSWWDTGELFFGEGSRL TVLEDLKNVFPPEVAVFEPSEAEI SHTQKATLVCLATGFYPDHVELS WWVNGKEVHSGVCTDPQPLKE QPALNDSRYALSSRLRVSATFW QDPRNHFRCQVQFYGLSENDEW TQDRAKPVTQIVSAEAWGRAD (SEQ ID NO:433) |
| RMFPNAPYL (SEQ ID NO:342) presented by HLA-A*02 | | |
| | Vα: | Vβ: |
| | EVEQNSGPLSVPEGAIASLNC TYSDRGSQSFFWYRQYSGKS PELIMFIYSNGDKEDGRFTA QLNKASQYVSLLIRDSQPSD SATYLCAVNDQGGGADGLT FGKGTHLIIQP (SEQ ID NO:434) | NAGVTQTPKFRVLKTGQSMTLL CAQDMNHEYMYWYRQDPGMG LRLIHYSVGEGTTAKGEVPDGY NVSRLKKQNFLLGLESAAPSQTS VYFCASSWWDTGELFFGEGSRL TVLEDLKNVF (SEQ ID NO:435) |
| | CDR3α: CAVNDQGGGADGLTF (SEQ ID NO:369) | CDR3β: CASSWWDTGELFF (SEQ ID NO:370) |
| WT1 (UniProt ID P19544) | AQSVTQLGSHVSVSEGALVL LRCNYSSSVPPYLFWYVQYP QGLQLLLKYTSAATLVKGIN GFEAEFKKSETSFHLTKPSAH MSDAAEYFCAVSEGGDYKL SFGAGTTVTVRADPAVYQL RDSKSSDKSVCLFTDFDSQT NVSQSKDSDVYITDKCVLD MRSMDFKSNSAVAWSNKSD FACANAFNNSIIPEDTFFPS (SEQ ID NO:436) | DVKVTQSSRYLVKRTGEKVFLE CVQDMDHENMFWYRQDPGLGL RLIYFSYDVKMKEKGDIPEGYSV SREKKERFSLILESASTNQTSMYL CAWGTLATEQYFGPGTRLTVTE DLKNVFPPEVAVFEPSEAEISHTQ KATLVCLATGFYPDHVELSWWV NGKEVHSGVCTDPQPLKEQPAL NDSRYALSSRLRVSATFWQDPR NHFRCQVQFYGLSENDEWTQDR AKPVTQIVSAEAWGRAD |
| RMFPNAPYL (SEQ ID NO:342) presented by HLA-A*02 | | |
| | Vα: | (SEQ ID NO:437) |
| | AQSVTQLGSHVSVSEGALVL LRCNYSSSVPPYLFWYVQYP QGLQLLLKYTSAATLVKGIN GFEAEFKKSETSFHLTKPSAH MSDAAEYFCAVSEGGDYKL SFGAGTTVTVRA (SEQ ID NO:438) | Vβ: |
| | | DVKVTQSSRYLVKRTGEKVFLE CVQDMDHENMFWYRQDPGLGL RLIYFSYDVKMKEKGDIPEGYSV SREKKERFSLILESASTNQTSMYL CAWGTLATEQYFGPGTRLTVTE DLKNVF (SEQ ID NO:439) |
| | CDR3α: CAVSEGGDYKLSF (SEQ ID NO:371) | CDR3β: CAWGTLATEQYF (SEQ ID NO:372) |
| MAGE-A3 (UniProt ID P43357) | AQEVTQIPAALSVPEGENLV LNCSFTDSAIYNLQWFRQDP GKGLTSLLYVRPYQREQTSG RLNASLDKSSGRSTLYIAAS QPGDSATYLCAVRPGGAGPF FVVFGKGTKLSVIPNIQNPDP AVYQLRDSKSSDKSVCLFTD FDSQTNVSQSKDSDVYITDK CVLDMRSMDFKSNSAVAWS NKSDFACANAFNNSIIP (SEQ ID NO:373) | AGVTQTPRYLIKTRGQQVTLSCS PISGHRSVSWYQQTPGQGLQFLF EYFSETQRNKGNFPGRFSGRQFS NSRSEMNVSTLELGDSALYLCAS SFNMATGQYFGPGTRLTVTEDL KNVFPPEVAVFEPSEAEISHTQK ATLVCLATGFYPDHVELSWWVN GKEVHSGVCTDPQPLKEQPALN DSRYALSSRLRVSATFWQDPRN HFRCQVQFYGLSENDEWTQDRA KPVTQIVSAEAWGRAD (SEQ ID NO:374) |
| CASSFNMATG QYF (SEQ ID NO:343) presented by HLA-A1 | | |
| | Vα: | Vβ: |
| | AQEVTQIPAALSVPEGENLV LNCSFTDSAIYNLQWFRQDP GKGLTSLLYVRPYQREQTSG RLNASLDKSSGRSTLYIAAS QPGDSATYLCAVRPGGAGPF FVVFGKGTKLSVIPNIQNP (SEQ ID NO:375) | AGVTQTPRYLIKTRGQQVTLSCS PISGHRSVSWYQQTPGQGLQFLF EYFSETQRNKGNFPGRFSGRQFS NSRSEMNVSTLELGDSALYLCAS SFNMATGQYFGPGTRLTVTEDL KNVF (SEQ ID NO:376) |
| | CDR3α: CAVRPGGAGPFF (SEQ ID NO:377) | CDR3β: CASSFNMATGQYF (SEQ ID NO:378) |
| MART1 (UniProt ID Q16655) | EVEQDPGPLSVPEGAIVSLNC TYSNSAFQYFMWYRQYSRK GPELLMYTYSSGNKEDGRFT AQVDKSSKYISLFIRDSQPSD SATYLCAMSETGGFKTIFGA GTRLFVKANIQNPDPAVYQL RDSKSSDKSVCLFTDFDSQT NVSQSKDSDVYITDKTVLD MRSMDFKSNSAVAWSNKSD FACANAFNNSIIPEDTFFPS | EAQVTQNPRYLITVTGKKLTVTC SQNMNHEYMSWYRQDPGLGLR QIYYSMNVEVTDKGDVPEGYKV SRKEKRNFPLILESPSPNQTSLYF CASSLVGTAGSPLHFGNGTRLTV TEDLNKVFPPEVAVFEPSEAEISH TQKATLVCLATGFFPDHVELSW WVNGKEVHSGVSTDPQPLKEQP ALNDSRYCLSSRLRVSATFWQN PRNHFRCQVQFYGLSENDEWTQ |
| EAAGIGILTV (SEQ ID NO:344) presented by HLA-A2 | | |
| | (SEQ ID NO:379) | DRAKPVTQIVSAEAWGRAD (SEQ ID NO:380) |
| | Vα: | Vβ: |
| | EVEQDPGPLSVPEGAIVSLNC TYSNSAFQYFMWYRQYSRK GPELLMYTYSSGNKEDGRFT AQVDKSSKYISLFIRDSQPSD SATYLCAMSETGGFKTIFGA GTRLFVKANIQNP (SEQ ID NO:381) | EAQVTQNPRYLITVTGKKLTVTC SQNMNHEYMSWYRQDPGLGLR QIYYSMNVEVTDKGDVPEGYKV SRKEKRNFPLILESPSPNQTSLYF CASSLVGTAGSPLHFGNGTRLTV TEDLNKVF (SEQ ID NO:382) |
| | CDR3α: CAMSETGGFKTIF (SEQ ID NO:383) | CDR3β: CASSLVGTAGSPLHF (SEQ ID NO:384) |
| SSX2 (UniProt ID Q16385) | CDR3α: CAMTSGFGNEKLTF (SEQ ID NO:387) | CDR3β: CATSRGQGGQPQHF (SEQ ID NO:388) |
| KASEKIFYV (SEQ ID NO:345) presented by HLA-A2 | | |
| BIRC5, also called survivin (UniProt ID O15392) | GESVGLHLPTLSVQEGDNSII NCAYSNSASDYFIWYKQESG KGPQFIIDIRSNMDKRQGQR VTVLLNKTVKHLSLQIAATQ PGDSAVYFCAENCAETVTDS WGKLQFGAGTQVVVTPDPA VYQLRDSKSSDKSVCLFTDF DSQTNVSQSKDSDVYITDKT VLDMRSMDFKSNSAVAWSN KSDFACANAFNNSIIPEDTFF PS (SEQ ID NO:440) | DAMVIQNPRYQVTQFGKPVTLS CSQTLNHNVMYWYQQKSSQAP KLLFHYYDKDFNNEADTPDNFQ SRRPNTSFCFLDIRSPGLGDAAM YLCATSRGDSTAEPQHFGDGTRL SILEDLNKVFPPEVAVFEPSEAEI SHTQKATLVCLATGFYPDHVELS WWVNGKEVHSGVCTDPQPLKE QPALNDSRYALSSRLRVSATFW QDPRNHFRCQVQFYGLSENDEW TQDRAKPVTQIVSAEAWGRAD (SEQ ID NO:441) |
| ELTLGEFLKL (SEQ ID NO:346) presented by HLA-A2 | | |
| | Vα: | Vβ: |
| | GESVGLHLPTLSVQEGDNSII NCAYSNSASDYFIWYKQESG KGPQFIIDIRSNMDKRQGQR VTVLLNKTVKHLSLQIAATQ PGDSAVYFCAENCAETVTDS WGKLQFGAGTQVVVTP (SEQ ID NO:442) | DAMVIQNPRYQVTQFGKPVTLS CSQTLNHNVMYWYQQKSSQAP KLLFHYYDKDFNNEADTPDNFQ SRRPNTSFCFLDIRSPGLGDAAM YLCATSRGDSTAEPQHFGDGTRL SILEDLNKVF (SEQ ID NO:443) |
| | CDR3α: CAETVTDSWGKLQF (SEQ ID NO:389) | CDR3β: CATSRGDSTAEPQHF (SEQ ID NO:390) |
| BIRC5, also called survivin (UniProt ID O15392) | AQKITQTQPGMFVQEKEAVT LDCTYDTSDQSYGLFWYKQ PSSGEMIFLIYQGSYDEQNAT EGRYSLNFQKARKSANLVIS ASQLGDSAMYFCAMREGGG YNKLIFGAGTRLAVHPDPAV YQLRDSKSSDKSVCLFTDFD SQTNVSQSKDSDVYITDKTV LDMRSMDFKSNSAVAWSNK SDFACANAFNNSIIPEDTFFPS (SEQ ID NO:385) | SQTIHQWPATLVQPVGSPLSLEC TVEGTSNPNLYWYRQAAGRGLQ LLFYSVGIGQISSEVPQNLSASRP QDRQFILSSKKLLLSDSGFYLCA GQDLNTEAFFGQGTRLTVVEDL NKVFPPEVAVFEPSEAEISHTQK ATLVCLATGFYPDHVELSWWVN GKEVHSGVCTDPQPLKEQPALN DSRYALSSRLRVSATFWQDPRN HFRCQVQFYGLSENDEWTQDRA KPVTQIVSAEAWGRAD (SEQ ID NO:386) |
| ELTLGEFLKL (SEQ ID NO:346) presented by HLA-A2 | | |
| | Vα: | Vβ: |
| | AQKITQTQPGMFVQEKEAVT LDCTYDTSDQSYGLFWYKQ PSSGEMIFLIYQGSYDEQNAT EGRYSLNFQKARKSANLVIS ASQLGDSAMYFCAMREGGG YNKLIFGAGTRLAVHP (SEQ ID NO:444) | SQTIHQWPATLVQPVGSPLSLEC TVEGTSNPNLYWYRQAAGRGLQ LLFYSVGIGQISSEVPQNLSASRP QDRQFILSSKKLLLSDSGFYLCA GQDLNTEAFFGQGTRLTVVEDL NKVF (SEQ ID NO:445) |
| | CDR3α: CAMREGGGYNKLIF (SEQ ID NO:391) | CDR3β: CAGQDLNTEAFF (SEQ ID NO:392) |
| PRAME (UniProt ID P78395) | AQSVTQLGSHVSVSERALVL LRCNYSSSVPPYLFWYVQYP NQGLQLLLKYTSAATLVKGI NGFEAEFKKSETSFHLTKPSA HMSDAAEYFCAVSGQTGAN NLFFGTGTRLTVIPYIQNPDP AVYQLRDSKSSDKSVCLFTD FDSQTNVSQSKDSDVYITDK TVLDMRSMDFKSNSAVAWS NKSDFACANAFNNSIIPEDTF FPS (SEQ ID NO:393) | DSGVTQTPKHLITATGQRVTLRC SPRSGDLSVYWYQQSLDQGLQF LIQYYNGEERAKGNILERFSAQQ FPDLHSELNLSSLELGDSALYFC ASARWDRGGEQYFGPGTRLTVT EDLKNVFPPEVAVFEPSEAEISHT QKATLVCLATGFYPDHVELSWW VNGKEVHSGVSTDPQPLKEQPA LNDSRYCLSSRLRVSATFWQNPR NHFRCQVQFYGLSENDEWTQDR AKPVTQIVSAEAWGRAD (SEQ ID NO:394) |
| QLLALLPSL (SEQ ID NO:347) presented by HLA-A2 | | |
| | Vα: | Vβ: |
| | AQSVTQLGSHVSVSERALVL LRCNYSSSVPPYLFWYVQYP NQGLQLLLKYTSAATLVKGI NGFEAEFKKSETSFHLTKPSA HMSDAAEYFCAVSGQTGAN NLFFGTGTRLTVIPYIQNP (SEQ ID NO:395) | DSGVTQTPKHLITATGQRVTLRC SPRSGDLSVYWYQQSLDQGLQF LIQYYNGEERAKGNILERFSAQQ FPDLHSELNLSSLELGDSALYFC ASARWDRGGEQYFGPGTRLTVT EDLKNVF (SEQ ID NO:396) |
| | CDR3α: CAVSGQTGANNLF (SEQ ID NO:397) | CDR3β: CASARWDRGGEQYF (SEQ ID NO:398) |
| PRAME (UniProt ID P78395) | GQQLNQSPQSMFIQEGEDVS MNCTSSSIFNTWLWYKQDP GEGPVLLIALYKAGELTSNG RLTAQFGITRKDSFLNISASIP SDVGIYFCAGIPRDNYGQNF VFGPGTRLSVLPYIQNPDPA VYQLRDSKSSDKSVCLFTDF DSQTNVSQSKDSDVYITDKT VLDMRSMDFKSNSAVAWSN KSDFACANAFNNSIIPEDTFF PS (SEQ ID NO:399) | DVKVTQSSRYLVKRTGEKVFLE CVQDMDHENMFWYRQDPGLGL RLIYFSYDVKMKEKGDIPEGYSV SREKKERFSLILESASTNQTSMYL CASTPWLAGGNEQFFGPGTRLT VLEDLKNVFPPEVAVFEPSEAEIS HTQKATLVCLATGFYPDHVELS WWVNGKEVHSGVSTDPQPLKE QPALNDSRYCLSSRLRVSATFW QNPRNHFRCQVQFYGLSENDEW TQDRAKPVTQIVSAEAWGRAD (SEQ ID NO:400) |
| QLLALLPSL (SEQ ID NO:347) presented by HLA-A2 | | |
| | Vα: | Vβ: |
| | GQQLNQSPQSMFIQEGEDVS MNCTSSSIFNTWLWYKQDP GEGPVLLIALYKAGELTSNG RLTAQFGITRKDSFLNISASIP SDVGIYFCAGIPRDNYGQNF VFGPGTRLSVLPYIQNP (SEQ ID NO:401) | DVKVTQSSRYLVKRTGEKVFLE CVQDMDHENMFWYRQDPGLGL RLIYFSYDVKMKEKGDIPEGYSV SREKKERFSLILESASTNQTSMYL CASTPWLAGGNEQFFGPGTRLT VLEDLKNVF (SEQ ID NO:402) |
| | CDR3α: CAGIPRDNYGQNFVF (SEQ ID NO:403) | CDR3β: CASTPWLAGGNEQFF (SEQ ID NO:404) |
| PRAME (UniProt ID P78395) | QKEVEQNSGPLSVPEGAIAS LNCTYSDRGSQSFFWYRQYS GKSPELIMFIYSNGDKEDGRF TAQLNKASQYVSLLIRDSQP SDSATYLCAVKDNAGNMLT FGGGTRLMVKPHIQNPDPAV YQLRDSKSSDKSVCLFTDFD SQTNVSQSKDSDVYITDKTV LDMRSMDFKSNSAVAWSNK SDFACANAFNNSIIPEDTFFPS (SEQ ID NO:405) | DSGVTQTPKHLITATGQRVTLRC SPRSGDLSVYWYQQSLDQGLQF LIQYYNGEERAKGNILERFSAQQ FPDLHSELNLSSLELGDSALYFC ASSDGGGVYEQYFGPGTRLTVT EDLKNVFPPEVAVFEPSEAEISHT QKATLVCLATGFYPDHVELSWW VNGKEVHSGVSTDPQPLKEQPA LNDSRYCLSSRLRVSATFWQNPR NHFRCQVQFYGLSENDEWTQDR AKPVTQIVSAEAWGRAD (SEQ ID NO:406) |
| QLLALLPSL (SEQ ID NO:347) presented by HLA-A2 | | |
| | Vα: | Vβ: |
| | QKEVEQNSGPLSVPEGAIAS LNCTYSDRGSQSFFWYRQYS GKSPELIMFIYSNGDKEDGRF TAQLNKASQYVSLLIRDSQP SDSATYLCAVKDNAGNMLT FGGGTRLMVKPHIQNP | DSGVTQTPKHLITATGQRVTLRC SPRSGDLSVYWYQQSLDQGLQF LIQYYNGEERAKGNILERFSAQQ FPDLHSELNLSSLELGDSALYFC ASSDGGGVYEQYFGPGTRLTVT |
| | (SEQ ID NO:407) | EDLKNVF (SEQ ID NO:408) |
| | CDR3α: CAVKDNAGNMLTF (SEQ ID NO:409) | CDR3β: CASSDGGGVYEQYF (SEQ ID NO:410) |
| NY-ESO-1 (UniProt ID P78358) |  (SEQ ID NO:411) |  (SEQ ID NO:412) |
| SLLMWITQC (SEQ ID NO:348) presented by HLA-A*02:01:48 | | |
| | Vα: | Vβ: |
| | QEVTQIPAALSVPEGENLVL NCSFTDSAIYNLQWFRQDPG KGLTSLLLIQSSQREQTSGRL NASLDKSSGRSTLYIAASQP GDSATYLCAVRPTSGGSYIP TFGRGTSLIVHPYIQNP (SEQ ID NO:413) | GVTQTPKFQVLKTGQSMTLQCA QDMNHEYMSWYRQDPGMGLRL IHYSVGAGITDQGEVPNGYNVSR STTEDFPLRLLSAAPSQTSVYFCA SSYVGNTGELFFGEGSRLTVLED LKNVF (SEQ ID NO:414) |
| | CDR3α: CAVRPTSGGSYIPTF (SEQ ID NO:415) | CDR3β: CASSYVGNTGELFF (SEQ ID NO:416) |
| NY-ESO-1 (UniProt ID P78358) | KQQVTQIPAALSVPEGENLV LNCSFTDSAIYNLQWFRQDP GGKLTSLLLIQSSQREQTSGR LNASLDKSAGSSTLYIAASQP GDSATYLCAVRPTSGGSYIP TFGRGTSLIVHPYIQNPDPAV YQLRDSKSSDKSVCLFTDFD SQTNVSQSKDSDVYITDKCV LDMRSMDFKSNSAVAWSNK SDFACANAFNNSIIPEDTFFPS PE (SEQ ID NO:417) |  (SEQ ID NO:412) |
| SLLMWITQC (SEQ ID NO:348) presented by HLA-A*02:01:48 | | |
| | Vα: | Vβ: |
| | KQQVTQIPAALSVPEGENLV LNCSFTDSAIYNLQWFRQDP GGKLTSLLLIQSSQREQTSGR LNASLDKSAGSSTLYIAASQP GDSATYLCAVRPTSGGSYIP | GVTQTPKFQVLKTGQSMTLQCA QDMNHEYMSWYRQDPGMGLRL IHYSVGAGITDQGEVPNGYNVSR STTEDFPLRLLSAAPSQTSVYFCA |
| | TFGRGTSLIVHPYIQNP (SEQ ID NO:418) | SSYVGNTGELFFGEGSRLTVLED LKNVF (SEQ ID NO:414) |
| | CDR3α: CAVRPTSGGSYIPTF (SEQ ID NO:415) | CDR3β: CASSYVGNTGELFF (SEQ ID NO:416) |
| NY-ESO-1 (UniProt ID P78358) | KQEVTQIPAALSVPEGENLV LNCSFTDSAIYNLQWFRQDP GKGLTSLLLIQSSQREQTSGR LNASLDKSSGSSTLYIAASQP GDSATYLCAVRPLLDGTYIP TFGRGTSLIVHPYIQNPDPAV YQLRDSKSSDKSVCLFTDFD SQTNVSQSKDSDVYITDKCV LDMRSMDFKSNSAVAWSNK SDFACANAFNNS (SEQ ID NO:419) | GVTQTPKFQVLKTGQSMTLQCA QDMNHEYMSWYRQDPGMGLRL IHYSVGAGTTDQGEVPNGYNVS RSTIEDFPLRLLSAAPSQTSVYFC ASSYLGNTGELFFGEGSRLTVLE DLKNVFPPEVAVFEPSEAEISHTQ KATLVCLATGFYPDHVELSWWV NGKEVHSGVCTDPQPLKEQPAL NDSRYALSSRLRVSATFWQDPR NHFRCQVQFYGLSENDEWTQDR AKPVTQIVSAEAWGRAD (SEQ ID NO:420) |
| SLLMWITQC (SEQ ID NO:348) presented by HLA-A*02:01:48 | | |
| | Vα: | Vβ: |
| | KQEVTQIPAALSVPEGENLV LNCSFTDSAIYNLQWFRQDP GKGLTSLLLIQSSQREQTSGR LNASLDKSSGSSTLYIAASQP GDSATYLCAVRPLLDGTYIP TFGRGTSLIVHPYIQNP (SEQ ID NO:421) | GVTQTPKFQVLKTGQSMTLQCA QDMNHEYMSWYRQDPGMGLRL IHYSVGAGTTDQGEVPNGYNVS RSTIEDFPLRLLSAAPSQTSVYFC ASSYLGNTGELFFGEGSRLTVLE DLKNVF (SEQ ID NO:422) |
| | CDR3α: CAVRPLLDGTYIPTF (SEQ ID NO:423) | CDR3β: CASSYLGNTGELFF (SEQ ID NO:424) |

### Antibody constant region and heterodimerization

The antibody constant region (Fc domain) described in the current invention can derive from a constant region of an antibody of any species that binds to CD16. In some embodiments, the amino acid sequence of the constant region is at least 90% identical to a human antibody constant region, such as an human IgG1 constant region, an IgG2 constant region, IgG3 constant region, or IgG4 constant region. In some other embodiments, the amino acid sequence of the constant region is at least 90% identical to an antibody constant region from another mammal, such as rabbit, dog, cat, mouse, or horse. In some embodiments, the antibody constant region includes a hinge, a CH2 domain, a CH3 domain and optionally a CH1 domain. In some embodiments, the antibody constant region that includes a hinge, a CH2 domain, a CH3 domain and optionally a CH1 domain is derived from a human IgG1 antibody. In some embodiments, the antibody constant region includes an amino acid sequence at least 90% identical to amino acids 234-332 of a human IgG1 antibody.

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (see, Sondermann et al., Nature, 406 (6793):267-273). Based on the known domains, mutations can be selected to enhance or reduce the binding affinity to CD16, such as by using phage-displayed libraries or yeast surface-displayed cDNA libraries, or can be designed based on the known three-dimensional structure of the interaction.

In certain embodiments, mutations that can be incorporated into the CH1 of a human IgG1 constant region may be at amino acid V125, F126, P127, T135, T139, A140, F170, P171, and/or V173. In certain embodiments, mutations that can be incorporated into the Cκ of a human IgG1 constant region may be at amino acid E123, F116, S176, V163, S174, and/or T164.

The assembly of heterodimeric antibody heavy chains can be accomplished by expressing two different antibody heavy chain sequences in the same cell, which may lead to the assembly of homodimers of each antibody heavy chain as well as assembly of heterodimers. Promoting the preferential assembly of heterodimers can be accomplished by incorporating different mutations in the CH3 domain of each antibody constant region as shown in US13/494870, US16/028850, US11/533709, US12/875015, US13/289934, US14/773418, US12/811207, US13/866756, US14/647480, and US14/830336. For example, mutations can be made in the CH3 domain based on human IgG1 and incorporating distinct pairs of amino acid substitutions within a first polypeptide and a second polypeptide that allow these two chains to selectively heterodimerize with each other. The positions of amino acid substitutions illustrated below are all numbered according to the EU index as in Kabat.

In one scenario, an amino acid substitution in the first polypeptide replaces the original amino acid with a larger amino acid, selected from arginine (R), phenylalanine (F), tyrosine (Y) or tryptophan (W), and at least one amino acid substitution in the second polypeptide replaces the original amino acid(s) with a smaller amino acid(s), chosen from alanine (A), serine (S), threonine (T), or valine (V), such that the larger amino acid substitution (a protuberance) fits into the surface of the smaller amino acid substitutions (a cavity). For example, one polypeptide can incorporate a T366W substitution, and the other can incorporate three substitutions including T366S, L368A, and Y407V.

One or more mutations can be incorporated into the constant region as compared to human IgG1 constant region, for example at Q347, Y349, L351, S354, E356, E357, K360, Q362, S364, T366, L368, K370, N390, K392, T394, D399, S400, D401, F405, Y407, K409, T411 and/or K439. Exemplary substitutions include, for example, Q347E, Q347R, Y349S, Y349K, Y349T, Y349D, Y349E, Y349C, T350V, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, T394W, D399R, D399K, D399V, S400K, S400R, D401K, F405A, F405T, Y407A, Y407I , Y407V, K409F, K409W, K409D, T411D, T411E, K439D, and K439E.

Alternatively, amino acid substitutions could be selected from the following sets of substitutions shown in Table 6.

| Table 6 | | |
|---|---|---|
| | First Polypeptide | Second Polypeptide |
| Set 1 | S364E/F405A | Y349K/T394F |
| Set 2 | S364H/D401K | Y349T/T411E |
| Set 3 | S364H/T394F | Y349T/F405A |
| Set 4 | S364E/T394F | Y349K/F405A |
| Set 5 | S364E/T411E | Y349K/D401K |
| Set 6 | S364D/T394F | Y349K/F405A |
| Set 7 | S364H/F405A | Y349T/T394F |
| Set 8 | S364K/E357Q | L368D/K370S |
| Set 9 | L368D/K370S | S364K |
| Set 10 | L368E/K370S | S364K |
| Set 11 | K360E/Q362E | D401K |
| Set 12 | L368D/K370S | S364K/E357L |
| Set 13 | K370S | S364K/E357Q |
| Set 14 | F405L | K409R |
| Set 15 | K409R | F405L |

Alternatively, amino acid substitutions could be selected from the following sets of substitutions shown in Table 7.

| Table 7 | | |
|---|---|---|
| | First Polypeptide | Second Polypeptide |
| Set 1 | K409W | D399V/F405T |
| Set 2 | Y349S | E357W |
| Set 3 | K360E | Q347R |
| Set 4 | K360E/K409W | Q347R/D399V/F405T |
| Set 5 | Q347E/K360E/K409W | Q347R/D399V/F405T |
| Set 6 | Y349S/K409W | E357W/D399V/F405T |

Alternatively, amino acid substitutions could be selected from the following set of substitutions shown in Table 8.

| Table 8 | | |
|---|---|---|
| | First Polypeptide | Second Polypeptide |
| Set 1 | T366K/L351K | L351D/L368E |
| Set 2 | T366K/L351K | L351D/Y349E |
| Set 3 | T366K/L351K | L351D/Y349D |
| Set 4 | T366K/L351K | L351D/Y349E/L368E |
| Set 5 | T366K/L351K | L351D/Y349D/L368E |
| Set 6 | E356K/D399K | K392D/K409D |

Alternatively, at least one amino acid substitution in each polypeptide chain could be selected from Table 9.

| Table 9 | |
|---|---|
| First Polypeptide | Second Polypeptide |
| L351Y, D399R, D399K, S400K, | T366V, T366I, T366L, T366M, |
| S400R, Y407A, Y407I, Y407V | N390D, N390E, K392L, K392M, K392V, K392F K392D, K392E, K409F, K409W, T411D and T411E |

Alternatively, at least one amino acid substitutions could be selected from the following set of substitutions in Table 10, where the position(s) indicated in the First Polypeptide column is replaced by any known negatively-charged amino acid, and the position(s) indicated in the Second Polypeptide Column is replaced by any known positively-charged amino acid.

| Table 10 | |
|---|---|
| First Polypeptide | Second Polypeptide |
| K392, K370, K409, or K439 | D399, E356, or E357 |

Alternatively, at least one amino acid substitutions could be selected from the following set of in Table 11, where the position(s) indicated in the First Polypeptide column is replaced by any known positively-charged amino acid, and the position(s) indicated in the Second Polypeptide Column is replaced by any known negatively-charged amino acid.

| Table 11 | |
|---|---|
| First Polypeptide | Second Polypeptide |
| D399, E356, or E357 | K409, K439, K370, or K392 |

Alternatively, amino acid substitutions could be selected from the following set in Table 12.

| Table 12 | |
|---|---|
| First Polypeptide | Second Polypeptide |
| T350V, L351Y, F405A, and Y407V | T350V, T366L, K392L, and T394W |

Alternatively, or in addition, the structural stability of a hetero-multimeric protein may be increased by introducing S354C on either of the first or second polypeptide chain, and Y349C on the opposing polypeptide chain, which forms an artificial disulfide bridge within the interface of the two polypeptides.

In some embodiments, the amino acid sequence of one polypeptide chain of the antibody constant region differs from the amino acid sequence of an IgG1 constant region at position T366, and wherein the amino acid sequence of the other polypeptide chain of the antibody constant region differs from the amino acid sequence of an IgG1 constant region at one or more positions selected from the group consisting of T366, L368 and Y407.

In some embodiments, the amino acid sequence of one polypeptide chain of the antibody constant region differs from the amino acid sequence of an IgG1 constant region at one or more positions selected from the group consisting of T366, L368 and Y407, and wherein the amino acid sequence of the other polypeptide chain of the antibody constant region differs from the amino acid sequence of an IgG1 constant region at position T366.

In some embodiments, the amino acid sequence of one polypeptide chain of the antibody constant region differs from the amino acid sequence of an IgG1 constant region at one or more positions selected from the group consisting of E357, K360, Q362, S364, L368, K370, T394, D401, F405, and T411 and wherein the amino acid sequence of the other polypeptide chain of the antibody constant region differs from the amino acid sequence of an IgG1 constant region at one or more positions selected from the group consisting of Y349, E357, S364, L368, K370, T394, D401, F405 and T411.

In some embodiments, the amino acid sequence of one polypeptide chain of the antibody constant region differs from the amino acid sequence of an IgG1 constant region at one or more positions selected from the group consisting of Y349, E357, S364, L368, K370, T394, D401, F405 and T411 and wherein the amino acid sequence of the other polypeptide chain of the antibody constant region differs from the amino acid sequence of an IgG1 constant region at one or more positions selected from the group consisting of E357, K360, Q362, S364, L368, K370, T394, D401, F405, and T411.

In some embodiments, the amino acid sequence of one polypeptide chain of the antibody constant region differs from the amino acid sequence of an IgG1 constant region at one or more positions selected from the group consisting of L351, D399, S400 and Y407 and wherein the amino acid sequence of the other polypeptide chain of the antibody constant region differs from the amino acid sequence of an IgG1 constant region at one or more positions selected from the group consisting of T366, N390, K392, K409 and T411.

In some embodiments, the amino acid sequence of one polypeptide chain of the antibody constant region differs from the amino acid sequence of an IgG1 constant region at one or more positions selected from the group consisting of T366, N390, K392, K409 and T411 and wherein the amino acid sequence of the other polypeptide chain of the antibody constant region differs from the amino acid sequence of an IgG1 constant region at one or more positions selected from the group consisting of L351, D399, S400 and Y407.

In some embodiments, the amino acid sequence of one polypeptide chain of the antibody constant region differs from the amino acid sequence of an IgG1 constant region at one or more positions selected from the group consisting of Q347, Y349, K360, and K409, and wherein the amino acid sequence of the other polypeptide chain of the antibody constant region differs from the amino acid sequence of an IgG1 constant region at one or more positions selected from the group consisting of Q347, E357, D399 and F405.

In some embodiments, the amino acid sequence of one polypeptide chain of the antibody constant region differs from the amino acid sequence of an IgG1 constant region at one or more positions selected from the group consisting of Q347, E357, D399 and F405, and wherein the amino acid sequence of the other polypeptide chain of the antibody constant region differs from the amino acid sequence of an IgG1 constant region at one or more positions selected from the group consisting of Y349, K360, Q347 and K409.

In some embodiments, the amino acid sequence of one polypeptide chain of the antibody constant region differs from the amino acid sequence of an IgG1 constant region at one or more positions selected from the group consisting of K370, K392, K409 and K439, and wherein the amino acid sequence of the other polypeptide chain of the antibody constant region differs from the amino acid sequence of an IgG1 constant region at one or more positions selected from the group consisting of D356, E357 and D399.

In some embodiments, the amino acid sequence of one polypeptide chain of the antibody constant region differs from the amino acid sequence of an IgG1 constant region at one or more positions selected from the group consisting of D356, E357 and D399, and wherein the amino acid sequence of the other polypeptide chain of the antibody constant region differs from the amino acid sequence of an IgG1 constant region at one or more positions selected from the group consisting of K370, K392, K409 and K439.

In some embodiments, the amino acid sequence of one polypeptide chain of the antibody constant region differs from the amino acid sequence of an IgG1 constant region at one or more positions selected from the group consisting of L351, E356, T366 and D399, and wherein the amino acid sequence of the other polypeptide chain of the antibody constant region differs from the amino acid sequence of an IgG1 constant region at one or more positions selected from the group consisting of Y349, L351, L368, K392 and K409.

In some embodiments, the amino acid sequence of one polypeptide chain of the antibody constant region differs from the amino acid sequence of an IgG1 constant region at one or more positions selected from the group consisting of Y349, L351, L368, K392 and K409, and wherein the amino acid sequence of the other polypeptide chain of the antibody constant region differs from the amino acid sequence of an IgG1 constant region at one or more positions selected from the group consisting of L351, E356, T366 and D399.

In some embodiments, the amino acid sequence of one polypeptide chain of the antibody constant region differs from the amino acid sequence of an IgG1 constant region by an S354C substitution and wherein the amino acid sequence of the other polypeptide chain of the antibody constant region differs from the amino acid sequence of an IgG1 constant region by a Y349C substitution.

In some embodiments, the amino acid sequence of one polypeptide chain of the antibody constant region differs from the amino acid sequence of an IgG1 constant region by a Y349C substitution and wherein the amino acid sequence of the other polypeptide chain of the antibody constant region differs from the amino acid sequence of an IgG1 constant region by an S354C substitution.

In some embodiments, the amino acid sequence of one polypeptide chain of the antibody constant region differs from the amino acid sequence of an IgG1 constant region by K360E and K409W substitutions and wherein the amino acid sequence of the other polypeptide chain of the antibody constant region differs from the amino acid sequence of an IgG1 constant region by O347R, D399V and F405T substitutions.

In some embodiments, the amino acid sequence of one polypeptide chain of the antibody constant region differs from the amino acid sequence of an IgG1 constant region by O347R, D399V and F405T substitutions and wherein the amino acid sequence of the other polypeptide chain of the antibody constant region differs from the amino acid sequence of an IgG1 constant region by K360E and K409W substitutions.

In some embodiments, the amino acid sequence of one polypeptide chain of the antibody constant region differs from the amino acid sequence of an IgG1 constant region by a T366W substitutions and wherein the amino acid sequence of the other polypeptide chain of the antibody constant region differs from the amino acid sequence of an IgG1 constant region by T366S, T368A, and Y407V substitutions.

In some embodiments, the amino acid sequence of one polypeptide chain of the antibody constant region differs from the amino acid sequence of an IgG1 constant region by T366S, T368A, and Y407V substitutions and wherein the amino acid sequence of the other polypeptide chain of the antibody constant region differs from the amino acid sequence of an IgG1 constant region by a T366W substitution.

In some embodiments, the amino acid sequence of one polypeptide chain of the antibody constant region differs from the amino acid sequence of an IgG1 constant region by T350V, L351Y, F405A, and Y407V substitutions and wherein the amino acid sequence of the other polypeptide chain of the antibody constant region differs from the amino acid sequence of an IgG1 constant region by T350V, T366L, K392L, and T394W substitutions.

In some embodiments, the amino acid sequence of one polypeptide chain of the antibody constant region differs from the amino acid sequence of an IgG1 constant region by T350V, T366L, K392L, and T394W substitutions and wherein the amino acid sequence of the other polypeptide chain of the antibody constant region differs from the amino acid sequence of an IgG1 constant region by T350V, L351Y, F405A, and Y407V substitutions.

Listed below are examples of the mutations in the constant regions that enable heterodimerization of two polypeptide chains. Trastuzumab heavy chain A1, A2, B1, and B2 each includes a heavy chain variable domain of trastuzumab (bold sequence) and a constant region derived from human IgG1 with mutations in underlined amino acids. Trastuzumab heavy chain A1 and Trastuzumab heavy chain B1 can preferentially pair and form a heterodimer. Trastuzumab heavy chain A2 and Trastuzumab heavy chain B2 can preferentially pair and form a heterodimer.
Trastuzumab Heavy chain A 
EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVCTLPPSRDELTENQVSLTCLVKGFYPSDIAVEWESNGQPENN
YKTTPPVLDSDGSFFLYSWLTVDKSRWQQGNVFSCSVMHEALHNHYTQKSLSLSPG
(SEQ ID NO:299).
Trastuzumab Heavy chain B 
EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPRVYTLPPCRDELTKNQVSLTCLVKGFYPSDIAVEWESNGQPEN
NYKTTPPVLVSDGSFTLYSKLTVDKSRWQQGNVFSCSVMHEALHNHYTQKSLSLSPG
(SEQ ID NO:300).
Trastuzumab Heavy chain A1 
EVQLVESGGGLVQPGGSLRLSCAASGFNIKDTYIHWVRQAPGKGLEWVARIYP
TNGYTRYADSVKGRFTISADTSKNTAYLQMNSLRAEDTAVYYCSRWGGDGFYA
MDYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALP
APIEKTISKAKGQPREPQVYTLPPCRDELTKNQVSLWCLVKGFYPSDIAVEWESNGQ
 
PENNYKTTPPVLDSDGSFFLYSKLTVDKSRWQQGNVFSCSVMHEALHNHYTQKSLS
LSPG* (SEQ ID NO:301).
Trastuzumab Heavy chain B1

EVQLVESGGGLVQPGGSLRLSCAASGFNIKDTYIHWVRQAPGKGLEWVARIYP
TNGYTRYADSVKGRFTISADTSKNTAYLQMNSLRAEDTAVYYCSRWGGDGFYA
MDYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALP
APIEKTISKAKGQPREPQVCTLPPSRDELTKNQVSLSCAVKGFYPSDIAVEWESNGQP
ENNYKTTPPVLDSDGSFFLVSKLTVDKSRWQQGNVFSCSVMHEALHNHYTQKSLSL
SPG* (SEQ ID NO:302).

The proteins described above can be made using recombinant DNA technology well known to a skilled person in the art. For example, a first nucleic acid sequence encoding the first immunoglobulin chain comprising an scFv, hinge, and an antibody constant region can be cloned into a first expression vector; a second nucleic acid sequence encoding the second immunoglobulin chain, for example, comprising another scFv, hinge, and an antibody constant region or comprising an antibody heavy chain can be cloned into a second expression vector; and a third nucleic acid sequence encoding an antibody light chain can be cloned into a third expression vector. The first, second, and optionally the third expression vectors can be stably transfected together into host cells to produce the multimeric proteins described herein.

To achieve the highest yield of the multi-specific binding protein, different ratios of the first, second, and third expression vector can be explored to determine the optimal ratio for transfection into the host cells. After transfection, single clones can be isolated for cell bank generation using methods known in the art, such as limited dilution, ELISA, FACS, microscopy, or Clonepix.

Clones can be cultured under conditions suitable for bio-reactor scale-up and maintained expression of the multi-specific binding protein. The multi-specific binding proteins can be isolated and purified using methods known in the art including centrifugation, depth filtration, cell lysis, homogenization, freeze-thawing, affinity purification, gel filtration, ion exchange chromatography, hydrophobic interaction exchange chromatography, and mixed-mode chromatography.

### II. THERAPEUTIC APPLICATIONS

The invention provides methods for treating cancer using a multi-specific binding protein described herein and/or a pharmaceutical composition described herein. The methods may be used to treat a variety of cancers, including a solid tumor, a lymphoma, and a leukemia. The type of cancer to be treated is desirably matched with the type of cancer cell to which the protein binds. For example, treatment of a cancer expressing HER2, such as a breast cancer expressing HER2, is desirably treated using a protein described herein that binds to protein. In certain embodiments, the invention provides methods for treating a variety of cancers which express BCMA by administering to a patient in need thereof a therapeutically effective amount of a multi-specific binding protein described herein. In certain embodiments, the invention provides methods for treating a variety of cancers which express CD33 by administering to a patient in need thereof a therapeutically effective amount of a multi-specific binding protein described herein.

Accordingly, one aspect of the invention provides a method of treating cancer in a patient, wherein the method comprises administering to a patient in need thereof a therapeutically effective amount of a protein described herein to treat the cancer. Additional aspects and embodiments of the therapeutic methods are described below.

The therapeutic method can be characterized according to the cancer to be treated. For example, in certain embodiments, the cancer is a solid tumor. In certain other embodiments, the cancer is brain cancer, bladder cancer, breast cancer, cervical cancer, colon cancer, colorectal cancer, endometrial cancer, esophageal cancer, leukemia, lung cancer, liver cancer, melanoma, ovarian cancer, pancreatic cancer, prostate cancer, rectal cancer, renal cancer, stomach cancer, testicular cancer, or uterine cancer. In yet other embodiments, the cancer is a vascularized tumor, squamous cell carcinoma, adenocarcinoma, small cell carcinoma, melanoma, neuroblastoma, sarcoma *(e.g.,* an angiosarcoma or chondrosarcoma), larynx cancer, parotid cancer, biliary tract cancer, thyroid cancer, acral lentiginous melanoma, actinic keratoses, acute lymphocytic leukemia, acute myeloid leukemia, adenoid cystic carcinoma, adenomas, adenosarcoma, adenosquamous carcinoma, anal canal cancer, anal cancer, anorectum cancer, astrocytic tumor, bartholin gland carcinoma, basal cell carcinoma, biliary cancer, bone cancer, bone marrow cancer, bronchial cancer, bronchial gland carcinoma, carcinoid, cholangiocarcinoma, chondosarcoma, choroid plexus papilloma/carcinoma, chronic lymphocytic leukemia, chronic myeloid leukemia, clear cell carcinoma, connective tissue cancer, cystadenoma, digestive system cancer, duodenum cancer, endocrine system cancer, endodermal sinus tumor, endometrial hyperplasia, endometrial stromal sarcoma, endometrioid adenocarcinoma, endothelial cell cancer, ependymal cancer, epithelial cell cancer, Ewing's sarcoma, eye and orbit cancer, female genital cancer, focal nodular hyperplasia, gallbladder cancer, gastric antrum cancer, gastric fundus cancer, gastrinoma, glioblastoma, glucagonoma, heart cancer, hemangiblastomas, hemangioendothelioma, hemangiomas, hepatic adenoma, hepatic adenomatosis, hepatobiliary cancer, hepatocellular carcinoma, Hodgkin's disease, ileum cancer, insulinoma, intraepithelial neoplasia, interepithelial squamous cell neoplasia, intrahepatic bile duct cancer, invasive squamous cell carcinoma, jejunum cancer, joint cancer, Kaposi's sarcoma, pelvic cancer, large cell carcinoma, large intestine cancer, leiomyosarcoma, lentigo maligna melanomas, lymphoma, male genital cancer, malignant melanoma, malignant mesothelial tumors, medulloblastoma, medulloepithelioma, meningeal cancer, mesothelial cancer, metastatic carcinoma, mouth cancer, mucoepidermoid carcinoma, multiple myeloma, muscle cancer, nasal tract cancer, nervous system cancer, neuroepithelial adenocarcinoma nodular melanoma, non-epithelial skin cancer, oat cell carcinoma, oligodendroglial cancer, oral cavity cancer, osteosarcoma, papillary serous adenocarcinoma, penile cancer, pharynx cancer, pituitary tumors, plasmacytoma, pseudosarcoma, pulmonary blastoma, rectal cancer, renal cell carcinoma, respiratory system cancer, retinoblastoma, rhabdomyosarcoma, sarcoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striated muscle cancer, submesothelial cancer, superficial spreading melanoma, T cell leukemia, tongue cancer, undifferentiated carcinoma, ureter cancer, urethra cancer, urinary bladder cancer, urinary system cancer, uterine cervix cancer, uterine corpus cancer, uveal melanoma, vaginal cancer, verrucous carcinoma, VIPoma, vulva cancer, well-differentiated carcinoma, or Wilms tumor.

In certain other embodiments, the cancer to be treated is non-Hodgkin's lymphoma, such as a B-cell lymphoma or a T-cell lymphoma. In certain embodiments, the non-Hodgkin's lymphoma is a B-cell lymphoma, such as a diffuse large B-cell lymphoma, primary mediastinal B-cell lymphoma, follicular lymphoma, small lymphocytic lymphoma, mantle cell lymphoma, marginal zone B-cell lymphoma, extranodal marginal zone B-cell lymphoma, nodal marginal zone B-cell lymphoma, splenic marginal zone B-cell lymphoma, Burkitt lymphoma, lymphoplasmacytic lymphoma, hairy cell leukemia, or primary central nervous system (CNS) lymphoma. In certain other embodiments, the non-Hodgkin's lymphoma is a T-cell lymphoma, such as a precursor T-lymphoblastic lymphoma, peripheral T-cell lymphoma, cutaneous T-cell lymphoma, angioimmunoblastic T-cell lymphoma, extranodal natural killer/T-cell lymphoma, enteropathy type T-cell lymphoma, subcutaneous panniculitis-like T-cell lymphoma, anaplastic large cell lymphoma, or peripheral T-cell lymphoma.

In certain embodiments, the cancers are AML, myelodysplastic syndromes, chronic myelomonocytic leukemia, myeloid blast crisis of chronic myeloid leukemia, and ALLs.

In certain embodiments, the cancer to be treated can be characterized according to the presence of a particular antigen expressed on the surface of the cancer cell. In certain embodiments, the cancer cell can expresses one or more of the following in addition to BCMA: CD2, CD19, CD20, CD30, CD38, CD40, CD52, CD70, EGFR/ERBB1, IGF1R, HER3/ERBB3, HER4/ERBB4, MUC1, cMET, SLAMF7, PSCA, MICA, MICB, TRAILR1, TRAILR2, MAGE-A3, B7.1, B7.2, CTLA4, and PD1.

In certain embodiments, the cancer cell can express one or more of the following in addition to CD33: CD2, CD19, CD20, CD30, CD38, CD40, CD52, CD70, EGFR/ERBB1, IGF1R, HER3/ERBB3, HER4/ERBB4, MUC1, TROP2, cMET, SLAMF7, PSCA, MICA, MICB, TRAILR1, TRAILR2, MAGE-A3, B7.1, B7.2, CTLA4, and PD1.

In certain embodiments, the cancer cell can express one or more of the following in addition to HER2: CD2, CD19, CD20, CD30, CD38, CD40, CD52, CD70, EGFR/ERBB1, IGF1R, HER3/ERBB3, HER4/ERBB4, MUC1, cMET, SLAMF7, PSCA, MICA, MICB, TRAILR1, TRAILR2, MAGE-A3, B7.1, B7.2, CTLA4, and PD1.

In certain embodiments, the cancer to be treated can be characterized according to the presentation of a particular TAA peptide by an MHC on the surface of the cancer cell. Non-limiting examples of proteins that can be processed into TCR-targetable TAA peptides include tissue differentiation antigens (*e.g.,* MART-1, gp100, CEA, CD19, and tyrosinase), tumor germline antigens (*e.g.,* NY-ESO-1, MAGE-A1, MAGE-A3, MAGE-A4, MAGE-A12, MAGE-C2, BAGE1, GAGE1, CTAG1, CTAG2, XAGE-1B, and SSX2), normal proteins overexpressed by cancer cells *(e.g.,* hTERT, EGFR, ERBB2, WT1, MUC1, and mesothelin), viral proteins (*e.g.,* HPV, EBV, and MCC), and tumor-specific mutated antigens. In certain embodiments, the cancer cell contains an ELAVL4 peptide having the amino acid sequence of SEQ ID NO:425 presented by HLA-A*02:01:48. In certain embodiments, the cancer cell contains an Insulin peptide having the amino acid sequence of SEQ ID NO:426 presented by HLA-A*02:01:48. In certain embodiments, the cancer cell contains an hTERT peptide having the amino acid sequence of SEQ ID NO:340 presented by HLA-A*02:01:48. In certain embodiments, the cancer cell contains an ERBB2 peptide having the amino acid sequence of SEQ ID NO:341 presented by HLA-A*02. In certain embodiments, the cancer cell contains a WT1 peptide having the amino acid sequence of SEQ ID NO:342 presented by HLA-A*02. In certain embodiments, the cancer cell contains a MAGE-A3 peptide having the amino acid sequence of SEQ ID NO:343 presented by HLA-A1. In certain embodiments, the cancer cell contains a MART1 peptide having the amino acid sequence of SEQ ID NO:344 presented by HLA-A2. In certain embodiments, the cancer cell contains a BIRC5 peptide having the amino acid sequence of SEQ ID NO:346 presented by HLA-A2. In certain embodiments, the cancer cell contains a PRAME peptide having the amino acid sequence of SEQ ID NO:347 presented by HLA-A2. In certain embodiments, the cancer cell contains an NY-ESO-1 peptide having the amino acid sequence of SEQ ID NO:348 presented by HLA-A2. In certain embodiments, the cancer cell contains an SSX2 peptide having the amino acid sequence of SEQ ID NO:345 presented by HLA-A2.

### III. COMBINATION THERAPY

Another aspect of the invention provides for combination therapy. A multi-specific binding protein described herein can be used in combination with additional therapeutic agents to treat the cancer.

Exemplary therapeutic agents that may be used as part of a combination therapy in treating cancer, include, for example, radiation, mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, luteinizing hormone releasing factor and variations of the aforementioned agents that may exhibit differential binding to its cognate receptor, and increased or decreased serum half-life.

An additional class of agents that may be used as part of a combination therapy in treating cancer is immune checkpoint inhibitors. Exemplary immune checkpoint inhibitors include agents that inhibit one or more of (i) cytotoxic T-lymphocyte-associated antigen 4 (CTLA4), (ii) programmed cell death protein 1 (PD1), (iii) PDL1, (iv) LAG3, (v) B7-H3, (vi) B7-H4, and (vii) TIM3. The CTLA4 inhibitor ipilimumab has been approved by the United States Food and Drug Administration for treating melanoma.

Yet other agents that may be used as part of a combination therapy in treating cancer are monoclonal antibody agents that target non-checkpoint targets (e.g., herceptin) and non-cytotoxic agents (e.g., tyrosine-kinase inhibitors).

Yet other categories of anti-cancer agents include, for example: (i) an inhibitor selected from an ALK Inhibitor, an ATR Inhibitor, an A2A Antagonist, a Base Excision Repair Inhibitor, a Bcr-Abl Tyrosine Kinase Inhibitor, a Bruton's Tyrosine Kinase Inhibitor, a CDC7 Inhibitor, a CHK1 Inhibitor, a Cyclin-Dependent Kinase Inhibitor, a DNA-PK Inhibitor, an Inhibitor of both DNA-PK and mTOR, a DNMT1 Inhibitor, a DNMT1 Inhibitor plus 2-chloro-deoxyadenosine, an HDAC Inhibitor, a Hedgehog Signaling Pathway Inhibitor, an IDO Inhibitor, a JAK Inhibitor, a mTOR Inhibitor, a MEK Inhibitor, a MELK Inhibitor, a MTH1 Inhibitor, a PARP Inhibitor, a Phosphoinositide 3-Kinase Inhibitor, an Inhibitor of both PARP1 and DHODH, a Proteasome Inhibitor, a Topoisomerase-II Inhibitor, a Tyrosine Kinase Inhibitor, a VEGFR Inhibitor, and a WEE1 Inhibitor; (ii) an agonist of OX40, CD137, CD40, GITR, CD27, HVEM, TNFRSF25, or ICOS; and (iii) a cytokine selected from IL-12, IL-15, GM-CSF, and G-CSF.

Proteins of the invention can also be used as an adjunct to surgical removal of the primary lesion.

The amount of multi-specific binding protein and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a multi-specific binding protein may be administered during a time when the additional therapeutic agent(s) exerts its prophylactic or therapeutic effect, or *vice versa.*

### IV. PHARMACEUTICAL COMPOSITIONS

The present disclosure also features pharmaceutical compositions/formulations that contain a therapeutically effective amount of a multi-specific binding protein described herein.

The composition can be formulated for use in a variety of drug delivery systems. One or more physiologically acceptable excipients or carriers can also be included in the composition for proper formulation. Suitable formulations for use in the present disclosure are found in Remington's Pharmaceutical Sciences, Mack Publishing Company, Philadelphia, Pa., 17th ed., 1985. For a brief review of methods for drug delivery, *see, e.g.,* Langer (Science 249:1527-1533, 1990).

The intravenous drug delivery formulation of the present disclosure may be contained in a bag, a pen, or a syringe. In certain embodiments, the bag may be connected to a channel comprising a tube and/or a needle. In certain embodiments, the formulation may be a lyophilized formulation or a liquid formulation. In certain embodiments, the formulation may freeze-dried (lyophilized) and contained in about 12-60 vials. In certain embodiments, the formulation may be freeze-dried and 45 mg of the freeze-dried formulation may be contained in one vial. In certain embodiments, the about 40 mg - about 100 mg of freeze-dried formulation may be contained in one vial. In certain embodiments, freeze dried formulation from 12, 27, or 45 vials are combined to obtained a therapeutic dose of the protein in the intravenous drug formulation. In certain embodiments, the formulation may be a liquid formulation and stored as about 250 mg/vial to about 1000 mg/vial. In certain embodiments, the formulation may be a liquid formulation and stored as about 600 mg/vial. In certain embodiments, the formulation may be a liquid formulation and stored as about 250 mg/vial.

The proteins of the present disclosure could exist in a liquid aqueous pharmaceutical formulation including a therapeutically effective amount of the protein in a buffered solution forming a formulation.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents. The composition in solid form can also be packaged in a container for a flexible quantity.

In certain embodiments, the present disclosure provides a formulation with an extended shelf life including the protein of the present disclosure, in combination with mannitol, citric acid monohydrate, sodium citrate, disodium phosphate dihydrate, sodium dihydrogen phosphate dihydrate, sodium chloride, polysorbate 80, water, and sodium hydroxide.

In certain embodiments, an aqueous formulation is prepared including the protein of the present disclosure in a pH-buffered solution. The buffer of this invention may have a pH ranging from about 4 to about 8, *e.g.,* from about 4.5 to about 6.0, or from about 4.8 to about 5.5, or may have a pH of about 5.0 to about 5.2. Ranges intermediate to the above recited pH's are also intended to be part of this disclosure. For example, ranges of values using a combination of any of the above recited values as upper and/or lower limits are intended to be included. Examples of buffers that will control the pH within this range include acetate (e.g., sodium acetate), succinate (such as sodium succinate), gluconate, histidine, citrate and other organic acid buffers.

In certain embodiments, the formulation includes a buffer system which contains citrate and phosphate to maintain the pH in a range of about 4 to about 8. In certain embodiments the pH range may be from about 4.5 to about 6.0, or from about pH 4.8 to about 5.5, or in a pH range of about 5.0 to about 5.2. In certain embodiments, the buffer system includes citric acid monohydrate, sodium citrate, disodium phosphate dihydrate, and/or sodium dihydrogen phosphate dihydrate. In certain embodiments, the buffer system includes about 1.3 mg/ml of citric acid *(e.g.,* 1.305 mg/ml), about 0.3 mg/ml of sodium citrate *(e.g.,* 0.305 mg/ml), about 1.5 mg/ml of disodium phosphate dihydrate *(e.g.,* 1.53 mg/ml), about 0.9 mg/ml of sodium dihydrogen phosphate dihydrate *(e.g.,* 0.86), and about 6.2 mg/ml of sodium chloride *(e.g.,* 6.165 mg/ml). In certain embodiments, the buffer system includes 1-1.5 mg/ml of citric acid, 0.25 to 0.5 mg/ml of sodium citrate, 1.25 to 1.75 mg/ml of disodium phosphate dihydrate, 0.7 to 1.1 mg/ml of sodium dihydrogen phosphate dihydrate, and 6.0 to 6.4 mg/ml of sodium chloride. In certain embodiments, the pH of the formulation is adjusted with sodium hydroxide.

A polyol, which acts as a tonicifier and may stabilize the antibody, may also be included in the formulation. The polyol is added to the formulation in an amount which may vary with respect to the desired isotonicity of the formulation. In certain embodiments, the aqueous formulation may be isotonic. The amount of polyol added may also be altered with respect to the molecular weight of the polyol. For example, a lower amount of a monosaccharide (e.g., mannitol) may be added, compared to a disaccharide (such as trehalose). In certain embodiments, the polyol which may be used in the formulation as a tonicity agent is mannitol. In certain embodiments, the mannitol concentration may be about 5 to about 20 mg/ml. In certain embodiments, the concentration of mannitol may be about 7.5 to 15 mg/ml. In certain embodiments, the concentration of mannitol may be about 10-14 mg/ml. In certain embodiments, the concentration of mannitol may be about 12 mg/ml. In certain embodiments, the polyol sorbitol may be included in the formulation.

A detergent or surfactant may also be added to the formulation. Exemplary detergents include nonionic detergents such as polysorbates (e.g., polysorbates 20, 80 etc.) or poloxamers (e.g., poloxamer 188). The amount of detergent added is such that it reduces aggregation of the formulated antibody and/or minimizes the formation of particulates in the formulation and/or reduces adsorption. In certain embodiments, the formulation may include a surfactant which is a polysorbate. In certain embodiments, the formulation may contain the detergent polysorbate 80 or Tween 80. Tween 80 is a term used to describe polyoxyethylene (20) sorbitanmonooleate *(see* Fiedler, Lexikon der Hifsstoffe, Editio Cantor Verlag Aulendorf, 4th edi., 1996). In certain embodiments, the formulation may contain between about 0.1 mg/mL and about 10 mg/mL of polysorbate 80, or between about 0.5 mg/mL and about 5 mg/mL. In certain embodiments, about 0.1% polysorbate 80 may be added in the formulation.

In embodiments, the protein product of the present disclosure is formulated as a liquid formulation. The liquid formulation may be presented at a 10 mg/mL concentration in either a USP / Ph Eur type I 50R vial closed with a rubber stopper and sealed with an aluminum crimp seal closure. The stopper may be made of elastomer complying with USP and Ph Eur. In certain embodiments vials may be filled with 61.2 mL of the protein product solution in order to allow an extractable volume of 60 mL. In certain embodiments, the liquid formulation may be diluted with 0.9% saline solution.

In certain embodiments, the liquid formulation of the proteins from the disclosure may be prepared as a 10 mg/mL concentration solution in combination with a sugar at stabilizing levels. In certain embodiments the liquid formulation may be prepared in an aqueous carrier. In certain embodiments, a stabilizer may be added in an amount no greater than that which may result in a viscosity undesirable or unsuitable for intravenous administration. In certain embodiments, the sugar may be a disaccharide, e.g., sucrose. In certain embodiments, the liquid formulation may also include one or more of a buffering agent, a surfactant, and a preservative.

In certain embodiments, the pH of the liquid formulation may be set by addition of a pharmaceutically acceptable acid and/or base. In certain embodiments, the pharmaceutically acceptable acid may be hydrochloric acid. In certain embodiments, the base may be sodium hydroxide.

In addition to aggregation, deamidation is a common product variant of peptides and proteins that may occur during fermentation, harvest/cell clarification, purification, drug substance/drug product storage and during sample analysis. Deamidation is the loss of NH₃ from a protein forming a succinimide intermediate that can undergo hydrolysis. The succinimide intermediate results in a 17 dalton mass decrease of the parent peptide. The subsequent hydrolysis results in an 18 dalton mass increase. Isolation of the succinimide intermediate is difficult due to instability under aqueous conditions. As such, deamidation is typically detectable as 1 dalton mass increase. Deamidation of an asparagine results in either aspartic or isoaspartic acid. The parameters affecting the rate of deamidation include pH, temperature, solvent dielectric constant, ionic strength, primary sequence, local polypeptide conformation and tertiary structure. The amino acid residues adjacent to Asn in the peptide chain affect deamidation rates. Gly and Ser following an Asn in protein sequences results in a higher susceptibility to deamidation.

In certain embodiments, the liquid formulation of the proteins from the present disclosure may be preserved under conditions of pH and humidity to prevent deamination of the protein product.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (e.g., phosphate-buffered saline), sterile saline solution, Ringer's solution or dextrose solution.

A preservative may be optionally added to the formulations herein to reduce bacterial action. The addition of a preservative may, for example, facilitate the production of a multi-use (multiple-dose) formulation.

Intravenous (IV) formulations may be the preferred administration route in particular instances, such as when a patient is in the hospital after transplantation receiving all drugs via the IV route. In certain embodiments, the liquid formulation is diluted with 0.9% Sodium Chloride solution before administration. In certain embodiments, the diluted drug product for injection is isotonic and suitable for administration by intravenous infusion.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

A preservative may be optionally added to the formulations herein to reduce bacterial action. The addition of a preservative may, for example, facilitate the production of a multi-use (multiple-dose) formulation.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (e.g., phosphate-buffered saline), sterile saline solution, Ringer's solution or dextrose solution.

The proteins of the present disclosure could exist in a lyophilized formulation including the proteins and a lyoprotectant. The lyoprotectant may be sugar, *e.g.,* disaccharides. In certain embodiments, the lyoprotectant may be sucrose or maltose. The lyophilized formulation may also include one or more of a buffering agent, a surfactant, a bulking agent, and/or a preservative.

The amount of sucrose or maltose useful for stabilization of the lyophilized drug product may be in a weight ratio of at least 1:2 protein to sucrose or maltose. In certain embodiments, the protein to sucrose or maltose weight ratio may be of from 1:2 to 1:5.

In certain embodiments, the pH of the formulation, prior to lyophilization, may be set by addition of a pharmaceutically acceptable acid and/or base. In certain embodiments the pharmaceutically acceptable acid may be hydrochloric acid. In certain embodiments, the pharmaceutically acceptable base may be sodium hydroxide.

Before lyophilization, the pH of the solution containing the protein of the present disclosure may be adjusted between 6 to 8. In certain embodiments, the pH range for the lyophilized drug product may be from 7 to 8.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

In certain embodiments, a "bulking agent" may be added. A "bulking agent" is a compound which adds mass to a lyophilized mixture and contributes to the physical structure of the lyophilized cake *(e.g.,* facilitates the production of an essentially uniform lyophilized cake which maintains an open pore structure). Illustrative bulking agents include mannitol, glycine, polyethylene glycol and sorbitol. The lyophilized formulations of the present invention may contain such bulking agents.

A preservative may be optionally added to the formulations herein to reduce bacterial action. The addition of a preservative may, for example, facilitate the production of a multi-use (multiple-dose) formulation.

In certain embodiments, the lyophilized drug product may be constituted with an aqueous carrier. The aqueous carrier of interest herein is one which is pharmaceutically acceptable *(e.g.,* safe and non-toxic for administration to a human) and is useful for the preparation of a liquid formulation, after lyophilization. Illustrative diluents include sterile water for injection (SWFI), bacteriostatic water for injection (BWFI), a pH buffered solution (e.g., phosphate-buffered saline), sterile saline solution, Ringer's solution or dextrose solution.

In certain embodiments, the lyophilized drug product of the current disclosure is reconstituted with either Sterile Water for Injection, USP (SWFI) or 0.9% Sodium Chloride Injection, USP. During reconstitution, the lyophilized powder dissolves into a solution.

In certain embodiments, the lyophilized protein product of the instant disclosure is constituted to about 4.5 mL water for injection and diluted with 0.9% saline solution (sodium chloride solution).

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The specific dose can be a uniform dose for each patient, for example, 50-5000 mg of protein. Alternatively, a patient's dose can be tailored to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data. An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

In general, dosages based on body weight are from about 0.01 µg to about 100 mg per kg of body weight, such as about 0.01 µg to about 100 mg/kg of body weight, about 0.01 µg to about 50 mg/kg of body weight, about 0.01 µg to about 10 mg/kg of body weight, about 0.01 µg to about 1 mg/kg of body weight, about 0.01 µg to about 100 µg/kg of body weight, about 0.01 µg to about 50 µg/kg of body weight, about 0.01 µg to about 10 µg/kg of body weight, about 0.01 µg to about 1 µg/kg of body weight, about 0.01 µg to about 0.1 µg/kg of body weight, about 0.1 µg to about 100 mg/kg of body weight, about 0.1 µg to about 50 mg/kg of body weight, about 0.1 µg to about 10 mg/kg of body weight, about 0.1 µg to about 1 mg/kg of body weight, about 0.1 µg to about 100 µg/kg of body weight, about 0.1 µg to about 10 µg/kg of body weight, about 0.1 µg to about 1 µg/kg of body weight, about 1 µg to about 100 mg/kg of body weight, about 1 µg to about 50 mg/kg of body weight, about 1 µg to about 10 mg/kg of body weight, about 1 µg to about 1 mg/kg of body weight, about 1 µg to about 100 µg/kg of body weight, about 1 µg to about 50 µg/kg of body weight, about 1 µg to about 10 µg/kg of body weight, about 10 µg to about 100 mg/kg of body weight, about 10 µg to about 50 mg/kg of body weight, about 10 µg to about 10 mg/kg of body weight, about 10 µg to about 1 mg/kg of body weight, about 10 µg to about 100 µg/kg of body weight, about 10 µg to about 50 µg/kg of body weight, about 50 µg to about 100 mg/kg of body weight, about 50µg to about 50 mg/kg of body weight, about 50 µg to about 10 mg/kg of body weight, about 50 µg to about 1 mg/kg of body weight, about 50 µg to about 100 µg/kg of body weight, about 100 µg to about 100 mg/kg of body weight, about 100 µg to about 50 mg/kg of body weight, about 100 µg to about 10 mg/kg of body weight, about 100 µg to about 1 mg/kg of body weight, about 1 mg to about 100 mg/kg of body weight, about 1 mg to about 50 mg/kg of body weight, about 1 mg to about 10 mg/kg of body weight, about 10 mg to about 100 mg/kg of body weight, about 10 mg to about 50 mg/kg of body weight, about 50 mg to about 100 mg/kg of body weight.

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Administration of the present invention could be intravenous, intraarterial, intraperitoneal, intramuscular, subcutaneous, intrapleural, intrathecal, intracavitary, by perfusion through a catheter or by direct intralesional injection. This may be administered once or more times daily, once or more times weekly, once or more times monthly, and once or more times annually.

The description above describes multiple aspects and embodiments of the invention. The patent application specifically contemplates all combinations and permutations of the aspects and embodiments.

### EXAMPLES

The invention now being generally described, will be more readily understood by reference to the following examples, which are included merely for purposes of illustration of certain aspects and embodiments of the present invention, and is not intended to limit the invention.

### Example 1 - Purification of F3-TriNKET and F3'-TriNKET

Both F3-TriNKET and F3'-TriNKET proteins were purified following the steps that are used to purify therapeutic monoclonal antibodies. Briefly, the purification method included a Protein A purification (achieved about 80-90% monomer) followed by Poros HS CIEX salt step elution (achieved about 97-99% monomer). For clinical manufacturing process, an additional purification step with Q Cepharose or Q Mustang (flow-through mode) (to achieve about 99% monomer) could be added to further reduce whole cell protein (WCP) and CHO DNA.

The purified F3'-TriNKET has high thermal stability (DSC), similar to therapeutic monoclonal antibodies. And the stability of the mutant Fc containing F3' -TrinKETs is close to IgG1 Fc. *See* Table 13 below.

**Table 13**

| **Molecule** | **Tm₁ (°C)** | **Tm₂ (°C)** | **Tm₃ (°C)** |
|---|---|---|---|
| Herceptin | 72.4 | 83.6 | |
| F3'-TriNKET | 73.2 | 79.0 | 86.7 |

### Example 2 - F3'-TriNKET is stable for at least 4 weeks

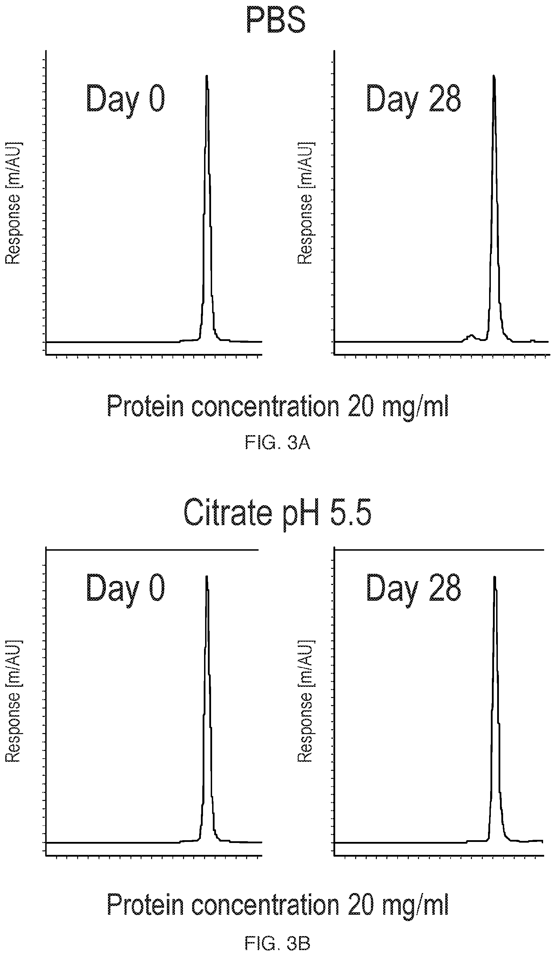

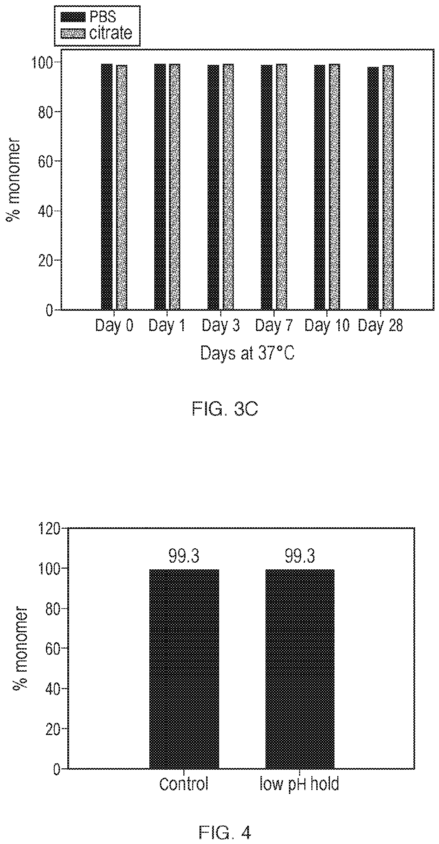

In an accelerated stability study carried out at 37 °C, F3'-TriNKET was found to be table over 4 weeks. *See* FIGs. 3A-3C.

The purified F3'-TriNKET was stable during low pH hold. 20 mg/mL of the purified protein was incubated for 2 hours in glycine at pH 3.0. FIG. 4 shows that the purified F3'-TriNKET was stable during low pH hold.

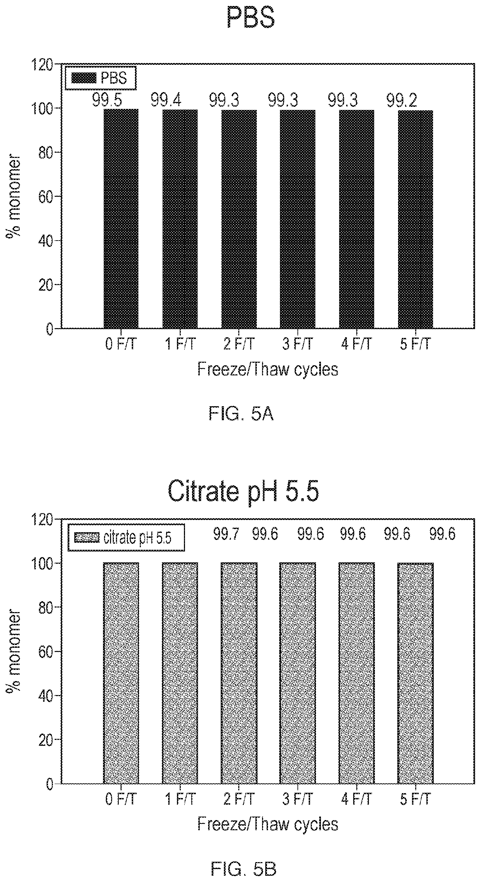

The purified F3'-TriNKET was stable after 5 cycles of freeze-thaw. FIG. 5A and FIG. 5B shows that the purified F3'-TriNKET was stable after 5 cycles of freeze-thaw, irrespective of the pH (FIG. 5A shows freeze-thaw cycles in PBS, and FIG. 5B shows freeze-thaw cycles in citrate at pH 5.5).

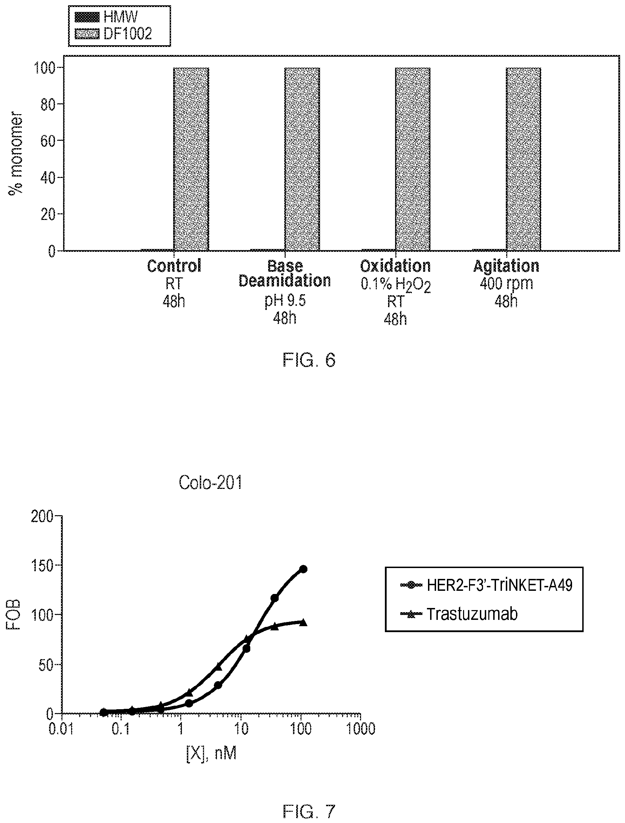

The purified F3'-TriNKET was stable in forced degradation studies. FIG. 6 shows bar graphs of the forced degradation conditions in which the F3'-TriNKET remained stable.

### Example 3 - Assessment of TriNKET binding to cell expressed human cancer antigens

**Human cancer cell lines expressing a cancer antigen of interest were used to assess** tumor antigen binding of TriNKETs. The human AML cell line Molm-13 was used to assess binding of F3'-TriNKET-CD33 to CD33 expressing cells. The HER2+ Colo-201 cell line was used to assess binding of F3'-TriNKET-HER2 to HER2 expressing cells. F3'-TriNKET-HER2 and F3'-TriNKET-CD33 were diluted, and were incubated with the respective cells. Binding of the F3'-TriNKET-HER2 and F3'-TriNKET-CD33 to the HER2-expressing and CD33-expressing cells, respectively, were detected using a fluorophore-conjugated anti-human IgG secondary antibody. Cells were analyzed by flow cytometry, binding MFI to HER2- and CD33-expressing cells, respectively, was normalized to secondary antibody controls to obtain fold over background values.

### Example 4 - Primary human NK cell cytotoxicity assay

Peripheral blood mononuclear cells (PBMCs) were isolated from human peripheral blood buffy coats using density gradient centrifugation. Isolated PBMCs were washed and prepared for NK cell isolation. NK cells were isolated using a negative selection technique with magnetic beads, purity of isolated NK cells was typically >90% CD3-CD56+. Isolated NK cells were rested overnight, and used the following day in cytotoxicity assays.

### DELFIA cytotoxicity assay:

Human cancer cell lines expressing a target of interest were harvested from culture, cells were washed with PBS, and were resuspended in growth media at 10⁶/mL for labeling with BATDA reagent (Perkin Elmer AD0116). Manufacturer instructions were followed for labeling of the target cells. After labeling cells were washed 3x with PBS, and were resuspended at 0.5-1.0x10⁵/mL in culture media. To prepare the background wells an aliquot of the labeled cells was put aside, and the cells were spun out of the media. 100 µl of the media was carefully added to wells in triplicate to avoid disturbing the pelleted cells. 100 µl of BATDA labeled cells were added to each well of the 96-well plate. Wells were saved for spontaneous release from target cells, and wells were prepared for max lysis of target cells by addition of 1% Triton-X. Monoclonal antibodies or TriNKETs against the tumor target of interest were diluted in culture media, 50 µl of diluted mAb or TriNKET was added to each well. Rested and/or activated NK cells were harvested from culture, cells were washed, and were resuspended at 10⁵-2.0x10⁶/mL in culture media depending on the desired E:T ratio. 50 µl of NK cells was added to each well of the plate to make a total of 200 µl culture volume. The plate was incubated at 37 °C with 5%CO₂ for 2-3 hours before developing the assay.

After culturing for 2-3 hours, the plate was removed from the incubator and the cells were pelleted by centrifugation at 200g for 5 minutes. 20 µl of culture supernatant was transferred to a clean microplate provided from the manufacturer and 200 µl of room temperature europium solution was added to each well. The plate was protected from the light and incubated on a plate shaker at 250 rpm for 15 minutes. The plate was read using either Victor 3 or SpectraMax i3X instruments. % Specific lysis was calculated as follows: % Specific lysis = ((Experimental release - Spontaneous release) / (Maximum release - Spontaneous release)) * 100%.

### Flow cytometry cytotoxicity assay

Human cancer cell lines expressing BCMA and transduced to stably express NucLight Green (Essen BioScience 4475) after puromycin selection were harvested from culture, spun down, and resuspended at 10⁵/mL in culture media. 100 µl of target cells was added to each well of a 96-well plate. TriNKETs against BCMA were diluted in culture media and 50 µl of each was added to duplicate wells. Purified human NKs rested overnight were harvested from culture, washed, and resuspended at 4x10⁵/mL in culture media. For a 2:1 E:T ratio, 50 µl of NK cells was added to all wells with the exception of target-only controls, which received 100 µl of culture media. The plate was incubated at 37 °C with 5% CO₂ for 30 hours.

After co-culture, cells were stained, fixed and analyzed by flow cytometry. Remaining target cells were detected with strong shifts in the FITC channel, with dead cells excluded with viability staining. The number of green events was exported and % killing calculated by comparison to target-only control samples. Counting beads were included to ensure recorded volumes were comparable.

### Example 5 - F3' TriNKETs bind to cell expressed tumor antigens

FIG. 7 shows binding of F3'-TriNKET-HER2 or Trastuzumab to HER2+ Colo-201 cells. F3'-TriNKET-HER2 bound Colo-201 cells to a higher maximum fold over background, but had a slightly reduced EC₅₀ binding value.

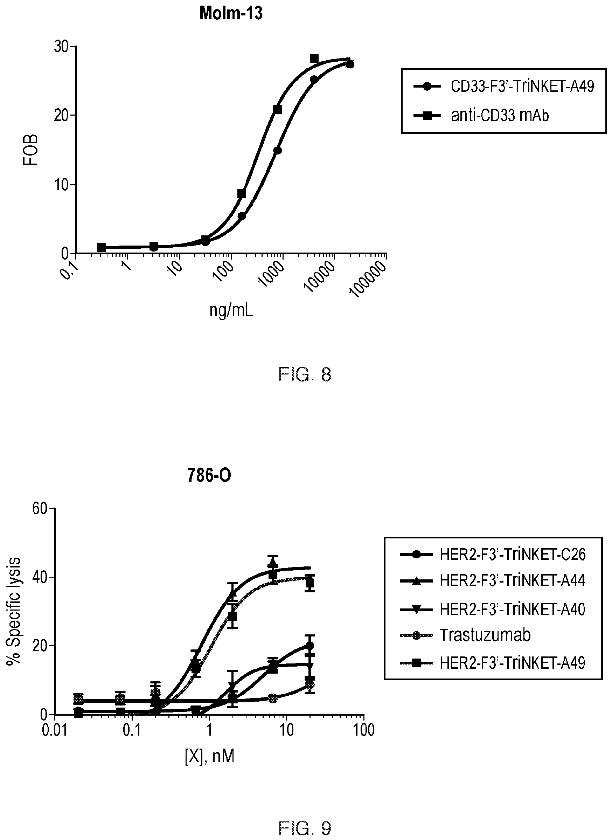

FIG. 8 shows binding of F3'-TriNKET-CD33 or CD33 monoclonal antibody to CD33 expressed on Molm-13 cells. F3'-TriNKET bound Molm-13 cells to a similar maximum fold over background and EC₅₀ binding value compared to the CD33 mAb.

### Example 6 - F3'-TriNKETs mediate NK cytotoxicity against HER2+ and CD33+ target cells:

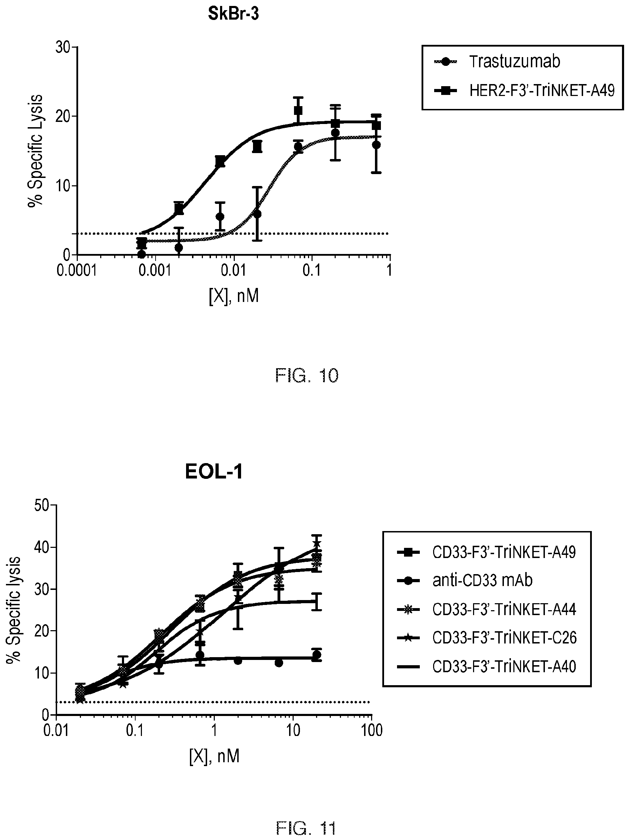

FIG. 9 and FIG. 10 show F3'-TriNKET-HER2 mediated cytotoxicity against the HER2-low cell line 786-O, and HER2-high cell line SkBr-3, respectively. The F3'-TriNKET-HER2 provided potent EC₅₀ values for induction of NK mediated cytotoxicity against both HER2-low and HER2-high cell lines. Compared to trastuzumab, the F3'-TriNKET-HER2 provided a greater maximum specific lysis and more potent EC₅₀ values on both high and low HER2-expressing cells.

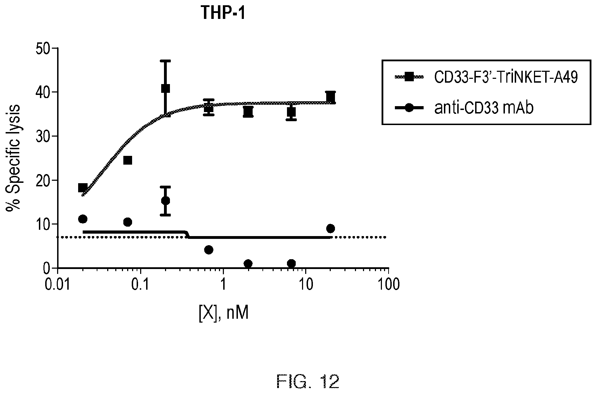

FIG. 11 and FIG. 12 show F3'-TriNKET-CD33 mediated cytotoxicity against two CD33 positive human cell lines, EOL-1 and THP-1, respectively. The CD33 monoclonal antibody was able to increase NK cell lysis of EOL-1 target cells (FIG. 11), but was found to be ineffective against the FcR-high THP-1 cell line (FIG. 12). The F3'-TriNKET-CD33 provided subnanomolar EC₅₀ values for induction of NK mediated cytotoxicity against both THP-1 and EOL-1 target cells.

### Example 7 - F4-TriNKETs mediate NK cytotoxicity

This example describes the potency of the bivalent F4 format TriNKET-mediated enhancement of NK cell killing of target cancer cells. This example describes binding of the bivalent F4 format TriNKET to target cells. This example describes the effects of the bivalent F4 format TriNKET in stabilizing and maintaining high cell surface expression of BCMA on cell surfaces. This example describes that the avidity of a bivalent F4 format TriNKET to its target improves the affinity with which the TriNKET binds to the target antigen, which in effect stabilizes expression and maintenance of high levels of the target antigen on the cell surface.

### BCMA surface stabilization by F4-TriNKETs

BCMA-positive KMS12-PE myeloma cells were incubated with 10 µg/mL F4-TriNKET or monoclonal antibody (EM-901), samples were divided into thirds, for each sample aliquots were placed on ice for 20 minutes, at 37 °C for 2 hours or 37 °C for 24 hours. After the incubation period cells were washed and bound F4-TriNKET was detected using an anti-human IgG secondary antibody. After staining the cells were fixed and stored at 4 °C, all samples were analyzed at the end of the study.

### Assessment of F4-TriNKET binding to cell expressed human cancer antigens

Human cancer cell lines expressing BCMA were used to assess tumor antigen binding of F4-TriNKETs (e.g., A49-F4-TriNKET-BCMA, an NKG2D-binding domain from clone ADI-27749 and a BCMA-binding domain derived from EM-901). The human multiple myeloma cell line MM.1R, which expresses BCMA at a higher level than KMS12-PE myeloma cells, was used to assess binding of TriNKETs to cells that express high levels of BCMA. F4-TriNKETs were diluted, and were incubated with MM.1R cells. Binding of the F4-TriNKET was detected using a fluorophore-conjugated anti-human IgG secondary antibody. Cells were analyzed by flow cytometry, binding MFI to cell expressed BCMA was normalized to secondary antibody controls to obtain fold over background values.

### Primary human NK cell cytotoxicity assay

Peripheral blood mononuclear cells (PBMCs) were isolated from human peripheral blood buffy coats using density gradient centrifugation. Isolated PBMCs were washed and prepared for NK cell isolation. NK cells were isolated using a negative selection technique with magnetic beads, purity of isolated NK cells was typically >90% CD3-CD56+. Isolated NK cells were rested overnight, and used the following day in cytotoxicity assays.

*DELFIA cytotoxicity assay:* Human cancer cell lines expressing a target of interest were harvested from culture, cells were washed with PBS, and were resuspended in growth media at 10⁶/mL for labeling with BATDA reagent (Perkin Elmer AD0116). Manufacturer instructions were followed for labeling of the target cells. After labeling cells were washed 3x with PBS, and were resuspended at 0.5-1.0x10⁵/mL in culture media. To prepare the background wells an aliquot of the labeled cells was put aside, and the cells were spun out of the media. 100 µl of the media was carefully added to wells in triplicate to avoid disturbing the pelleted cells. 100 µl of BATDA labeled cells were added to each well of the 96-well plate. Wells were saved for spontaneous release from target cells, and wells were prepared for max lysis of target cells by addition of 1% Triton-X. Monoclonal antibodies or TriNKETs against the tumor target of interest were diluted in culture media, 50 µl of diluted mAb or TriNKET was added to each well. Rested and/or activated NK cells were harvested from culture, cells were washed, and were resuspended at 10⁵-2.0x10⁶/mL in culture media depending on the desired E:T ratio. 50 µl of NK cells was added to each well of the plate to make a total of 200 µl culture volume. The plate was incubated at 37 °C with 5%CO₂ for 2-3 hours before developing the assay. After culturing for 2-3 hour, the plate was removed from the incubator and the cells were pelleted by centrifugation at 200g for 5 minutes. 20 µl of culture supernatant was transferred to a clean microplate provided from the manufacturer and 200 µl of room temperature europium solution was added to each well. The plate was protected from the light and incubated on a plate shaker at 250 rpm for 15 minutes. The plate was read using either Victor 3 or SpectraMax i3X instruments. % Specific lysis was calculated as follows: % Specific lysis = ((Experimental release - Spontaneous release) / (Maximum release - Spontaneous release)) * 100%.

### F4-TriNKET-mediated NK cytotoxicity

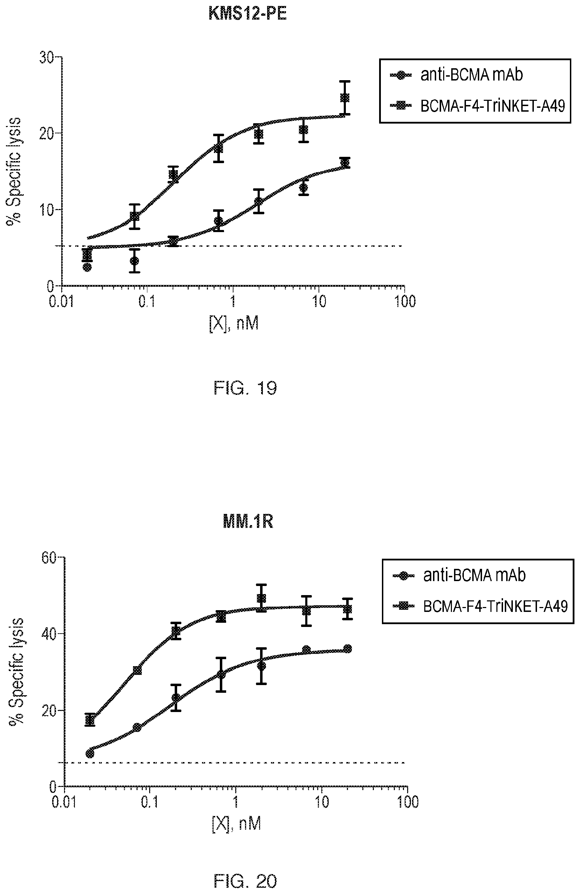

F4-TriNKET-mediated lysis of BCMA-positive myeloma cells was assayed. FIG. 19 shows F4-TriNKET-mediated lysis of BCMA-positive KMS12-PE myeloma cells by rested human NK effector cells. FIG. 20 shows F4-TriNKET-mediated lysis of BCMA-positive MM.1R myeloma cells by rested human NK effector cells. EM-901 monoclonal antibody was used as a control in both experiments. In FIG. 19, KMS12-PE cells (low BCMA expression) were used as target cells; in FIG. 20 MM.1R (high BCMA expression) were used as target cells. Against both high and low BCMA expressing cells, MM.1R and KMS12-PE, respectively, the F4-TriNKET had subnanomolar EC₅₀ values. Compared to a BCMA monoclonal antibody EM-901, the F4-TriNKET provided greater maximum specific lysis and potency against both cell lines.

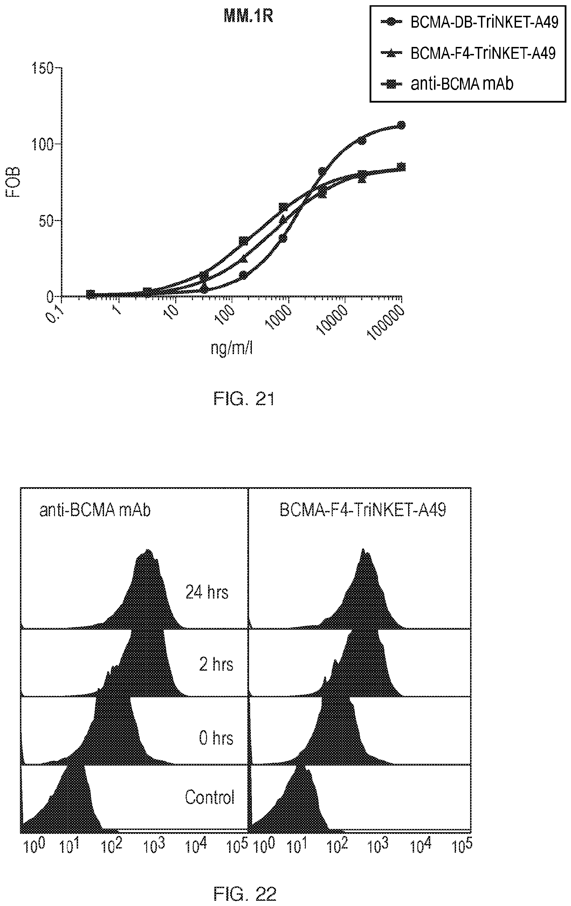

FIG. 21 shows binding of F4-TriNKET (A49-F4-TriNKET-BCMA), duobody-TriNKET (A49-DB-TriNKET-BCMA), or BCMA monoclonal antibody (EM-901) to MM.1R myeloma cells. All three proteins were able to bind to BCMA expressed on MM.1R cells in a dose-responsive manner. Avid binders were able to bind with slightly reduced maximum fold over background compared to monovalent binders, but avid binding provided an improved EC₅₀ binding value.

### F4-TriNKETs stabilize surface BCMA

FIG. 22 shows staining of surface BCMA with F4-TriNKET or BCMA monoclonal antibody (EM-901) after incubation for the indicated time. Both the BCMA mAb (EM-901) and F4-TriNKET were able to stabilize surface BCMA rapidly after incubation. BCMA mAb (EM-901) and F4-TriNKET were able to sustain increased BCMA surface expression over a period of 24 hours.

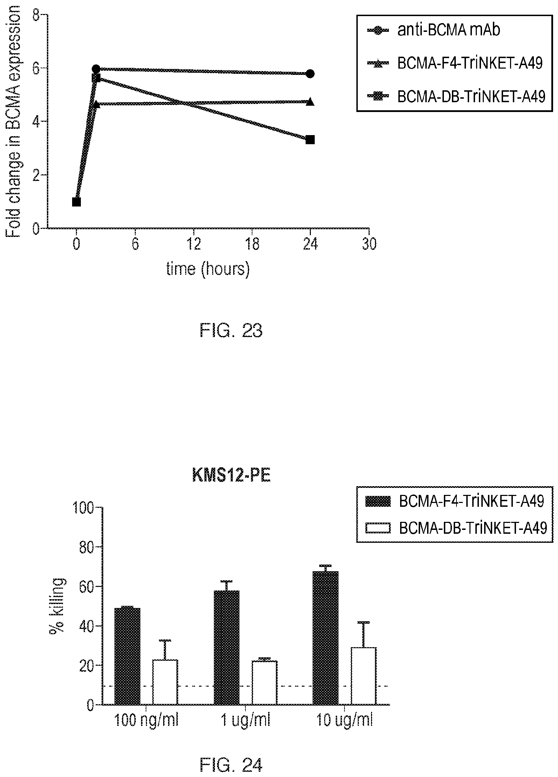

FIG. 23 shows stabilization of surface BCMA on KMS12-PE cells after incubation with F4-TriNKET or BCMA monoclonal antibody (EM-901). Initial stabilization of BCMA on the cell surface happened rapidly after exposure to F4-TriNKET or mAb (EM-901). Avid binding provided by the F4-TriNKET and monoclonal antibody (EM-901) sustained high surface BCMA for longer than monovalent BCMA binding, as indicated by the drop in surface BCMA expression at 24 hours with the duobody-TriNKET.

### F4-TriNKETs mediate substantial long-term cytotoxicity

Flow cytometry cytotoxicity assay: Human cancer cell lines expressing BCMA and transduced to stably express NucLight Green (Essen BioScience 4475) after puromycin selection were harvested from culture, spun down, and resuspended at 10⁵/mL in culture media. 100 µl of target cells was added to each well of a 96-well plate. TriNKETs against BCMA were diluted in culture media and 50 µl of each was added to duplicate wells. Purified human NKs rested overnight were harvested from culture, washed, and resuspended at 4×10⁵/mL in culture media. For a 2:1 E:T ratio, 50 µl of NK cells was added to all wells with the exception of target-only controls, which received 100 µl of culture media. The plate was incubated at 37 °C with 5% CO₂ for 30 hours.

After co-culture, cells were stained, fixed and analyzed by flow cytometry. Remaining target cells were detected with strong shifts in the FITC channel, with dead cells excluded with viability staining. The number of green events was exported and %killing calculated by comparison to target-only control samples. Counting beads were included to ensure recorded volumes were comparable.

### F4-TriNKETs mediate substantial long-term cytotoxicity

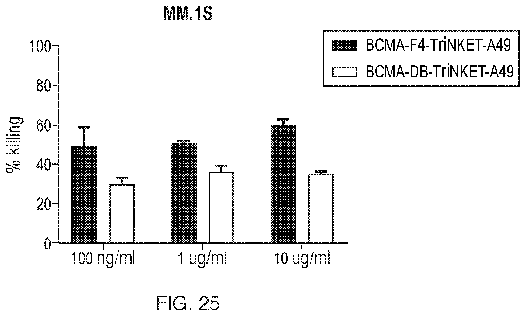

FIG. 24 and FIG. 25 show human NK cell lysis of BCMA positive target cell lines with 2:1 E:T ratio in the presence of F4-or DB-TriNKETs after 30 hours. FIG. 24 shows killing of KMS12-PE cells (low BCMA expression) above no protein control killing, while in FIG. 25 MM.1S cells (higher BCMA expression) were used as target cells. Compared to the monovalent duobody-TriNKET, the F4-TriNKET demonstrated elevated killing of both high and low BCMA expressing cell lines at all tested concentrations.

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

### Embodiments of the invention

1. A polypeptide comprising a single-chain variable fragment (scFv) linked to an antibody constant domain via a hinge comprising Ala-Ser, wherein the scFv comprises a heavy chain variable domain and a light chain variable domain.
2. The polypeptide according to embodiment 1, wherein the heavy chain variable domain forms a disulfide bridge with the light chain variable domain.
3. A polypeptide according to embodiment 2, wherein the disulfide bridge is formed between C44 from the heavy chain variable domain and C100 from the light chain variable domain.
4. A polypeptide according to any one of embodiments 1-3, wherein the heavy chain variable domain is linked to the light chain variable domain via a flexible linker.
5. A polypeptide according to embodiment 4, wherein the flexible linker comprises (G₄S)₄.
6. A polypeptide according to any one of embodiments 1-5, wherein the heavy chain variable domain is positioned at the N-terminus or the C-terminus of the light chain variable domain.
7. A polypeptide according to any one of embodiments 1-6, wherein the hinge further comprises amino acid sequence Thr-Lys-Gly.
8. A polypeptide according to any one of embodiments 1-7, wherein the antibody constant domain comprises an antibody Fc domain or a portion thereof sufficient to bind CD16.
9. A polypeptide according to embodiment 8, wherein the antibody constant domain comprises a CH2 and a CH3 domain of a human IgG1 antibody.
10. A polypeptide according to embodiment 9, wherein the antibody constant domain comprises an amino acid sequence at least 90% identical to amino acids 234-332 of a human IgG1 antibody.
11. A polypeptide according to embodiment 10, wherein the antibody constant domain comprises amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.
12. A polypeptide according to embodiment 11, wherein the antibody constant domain comprises amino acid sequence at least 90% identical to the Fc domain of human IgG1 and differs by one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E.
13. A polypeptide according to any one of embodiments 1-12, wherein the scFv binds NKG2D or a tumor-associated antigen.
14. A polypeptide according to embodiment 13, wherein the scFv binds to NKG2D, and the heavy chain variable domain of the scFv comprises an amino acid sequence at least 90% identical to an amino acid sequence selected from SEQ ID NO:94, SEQ ID NO:1, SEQ ID NO:44, SEQ ID NO:52, SEQ ID NO:60, SEQ ID NO:62, SEQ ID NO:70, SEQ ID NO:78, SEQ ID NO:86, SEQ ID NO:102, SEQ ID NO:322, SEQ ID NO:325, SEQ ID NO:328, SEQ ID NO:331, SEQ ID NO:334, and SEQ ID NO:337.
15. A polypeptide according to embodiment 14, wherein the heavy chain variable domain comprises an amino acid sequence at least 90% identical to SEQ ID NO:94 and the light chain variable domain comprises an amino acid sequence at least 90% identical to SEQ ID NO:98.
16. A polypeptide according to embodiment 14, wherein the heavy chain variable domain comprises an amino acid sequence at least 90% identical to SEQ ID NO:322 and the light chain variable domain comprises an amino acid sequence at least 90% identical to SEQ ID NO:98.
17. A polypeptide according to embodiment 14, wherein the heavy chain variable domain comprises an amino acid sequence at least 90% identical to SEQ ID NO:325 and the light chain variable domain comprises an amino acid sequence at least 90% identical to SEQ ID NO:98.
18. A polypeptide according to embodiment 14, wherein the heavy chain variable domain comprises an amino acid sequence at least 90% identical to SEQ ID NO:328 and the light chain variable domain comprises an amino acid sequence at least 90% identical to SEQ ID NO:98.
19. A polypeptide according to embodiment 14, wherein the heavy chain variable domain comprises an amino acid sequence at least 90% identical to SEQ ID NO:331 and the light chain variable domain comprises an amino acid sequence at least 90% identical to SEQ ID NO:98.
20. A polypeptide according to embodiment 14, wherein the heavy chain variable domain comprises an amino acid sequence at least 90% identical to SEQ ID NO:334 and the light chain variable domain comprises an amino acid sequence at least 90% identical to SEQ ID NO:98.
21. A polypeptide according to embodiment 14, wherein the heavy chain variable domain comprises an amino acid sequence at least 90% identical to SEQ ID NO:337 and the light chain variable domain comprises an amino acid sequence at least 90% identical to SEQ ID NO:98.
22. A polypeptide according to embodiment 14, wherein the heavy chain variable domain comprises an amino acid sequence at least 90% identical to SEQ ID NO:44 and the light chain variable domain comprises an amino acid sequence at least 90% identical to SEQ ID NO:48.
23. A polypeptide according to embodiment 14, wherein the heavy chain variable domain comprises an amino acid sequence at least 90% identical to SEQ ID NO:52 and the light chain variable domain comprises an amino acid sequence at least 90% identical to SEQ ID NO:56.
24. A polypeptide according to embodiment 14, wherein the heavy chain variable domain comprises an amino acid sequence at least 90% identical to SEQ ID NO:60 and the light chain variable domain comprises an amino acid sequence at least 90% identical to SEQ ID NO:61.
25. A polypeptide according to embodiment 14, wherein the heavy chain variable domain comprises an amino acid sequence at least 90% identical to SEQ ID NO:62 and the light chain variable domain comprises an amino acid sequence at least 90% identical to SEQ ID NO:66.
26. A polypeptide according to embodiment 14, wherein the heavy chain variable domain comprises an amino acid sequence at least 90% identical to SEQ ID NO:70 and the light chain variable domain comprises an amino acid sequence at least 90% identical to SEQ ID NO:74.
27. A polypeptide according to embodiment 14, wherein the heavy chain variable domain comprises an amino acid sequence at least 90% identical to SEQ ID NO:78 and the light chain variable domain comprises an amino acid sequence at least 90% identical to SEQ ID NO:82.
28. A polypeptide according to embodiment 14, wherein the heavy chain variable domain comprises an amino acid sequence at least 90% identical to SEQ ID NO:86 and the light chain variable domain comprises an amino acid sequence at least 90% identical to SEQ ID NO:90.
29. A polypeptide according to embodiment 14, wherein the heavy chain variable domain comprises an amino acid sequence at least 90% identical to SEQ ID NO:102 and the light chain variable domain comprises an amino acid sequence at least 90% identical to SEQ ID NO:106.
30. A polypeptide according to embodiment 13, wherein the scFv binds to NKG2D, and wherein the heavy chain variable domain comprises an amino acid sequence at least 90% identical to SEQ ID NO:110 and the light chain variable domain comprises an amino acid sequence at least 90% identical to SEQ ID NO:111.
31. A polypeptide according to embodiment 13, wherein the scFv binds to NKG2D, and wherein the heavy chain variable domain comprises an amino acid sequence at least 90% identical to SEQ ID NO:112 and the light chain variable domain comprises an amino acid sequence at least 90% identical to SEQ ID NO:113.
32. A polypeptide according to embodiment 13, wherein the scFv binds to a tumor-associated antigen, the tumor-associated antigen is selected from the group consisting of ANO1, BCMA, EpCAM, CAIX, CEA, CCR4, CD2, CD123, CD133, CD19, CD20, CD22, CD25, CD30, CD33, CD37, CD38, CD40, CD52, CD70, CLAUDIN-18.2, DLL3, EGFR/ERBB1, GD2, IGF1R, HER2, HER3/ERBB3, HER4/ERBB4, MUC1, cMET, SLAMF7, PSMA, mesothelin, MICA, MICB, TRAILR1, TRAILR2, TROP2, MAGE-A3, B7.1, B7.2, CTLA4, PD1, 5T4, GPNMB, FR-alpha, PAPP-A, FLT3, GPC3, CXCR4, ROR1, ROR2, HLA-E, PD-L1, VLA4, CD44, CD13, CD15, CD47, CLL1, CD81, CD23, CD79a, CD79b, CD80, CRLF2, SLAMF7, CD138, CA125, NaPi2b, Nectin4, ADAM8, ADAM9, SLC44A4, CA19-9, LILRBI, LILRB2, LILRB3, LILRB4, LILRB5, ULRA 1, LILRA2, LILRA3, ULRA4, LILRA5, and ULRA6, CCR8, CD7, CTLA4, CX3CR1, ENTPD1, HAVCR2, IL-1R2, PDCD1LG2, TIGIT, TNFRSF4, TNFRSF8, TNFRSF9, GEM, NT5E, TNFRSF18, MUC1, P-cadherin, Plexin-A1, TNFRSF10B, STEAP1, CDCP1, PTK7, Axl, erbB-3, EDNRB, Tyrp1, CD14, CD163, CSF3R, Siglec-9, ITGAM, VISTA, B7-H4 (VTCN1), CCR1, LRRC25, PTAFR, SIRPB1, TLR2, TLR4, CD300LB, ATP1A3, CCR5, MUC1 (or MUC1-C), Plexin-A1, TNFRSF10B, STEAP1, CDCP1, PTK7, AXL, EDNRB, OLR1, and TYRP1.
33. A protein comprising the scFv according to any one of the previous embodiments.
34. A protein comprising:
   (a) a first antigen-binding site, wherein the first antigen-binding site comprises a polypeptide according to any one of embodiments 1-32;
   (b) a second antigen-binding site; and
   (c) a second antibody constant domain.
35. The protein of embodiment 34, wherein the first antigen-binding site binds NKG2D, and the second antigen-binding site binds an tumor-associated antigen.
36. The protein of embodiment 34, wherein the first antigen-binding site binds an tumor-associated antigen, and the second antigen-binding site binds NKG2D.
37. A protein according to any one of embodiments 34-36, wherein the second antigen binding site comprises an scFv or Fab.
38. A protein according to any one of embodiments 34-37, wherein the second antibody constant domain comprises hinge and CH2 domain of a human IgG1 antibody.
39. A protein according to any one of embodiments 34-38, wherein the second antibody constant domain comprises an amino acid sequence at least 90% identical to amino acids 234-332 of a human IgG1 antibody.
40. A protein according to embodiment 39, wherein the second antibody constant domain comprises an amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.
41. A protein comprising:
   (a) a first antigen-binding site that binds a tumor-associated antigen;
   (b) a second antigen-binding site that binds the same tumor-associated antigen as the first antigen-binding site;
   (c) a third antigen-binding site that binds NKG2D; and
   (d) an antibody constant region or a portion thereof sufficient to bind CD16, or a fourth antigen-binding site that binds CD16.
42. A protein according to embodiment 41, wherein the first antigen-binding site comprises an scFv or Fab.
43. A protein according to embodiment 41 or 42, wherein the second antigen-binding site comprises an scFv or Fab.
44. A protein according to any one of embodiments 41-43, wherein the third antigen-binding site comprises an scFv or Fab.
45. A protein according to any one of embodiments 41-44, wherein the tumor-associated antigen is selected from the group consisting of ANO1, BCMA, EpCAM, CAIX, CEA, CCR4, CD2, CD123, CD133, CD19, CD20, CD22, CD25, CD30, CD33, CD37, CD38, CD40, CD52, CD70, CLAUDIN-18.2, DLL3, EGFR/ERBB1, GD2, IGF1R, HER2, HER3/ERBB3, HER4/ERBB4, MUC1, cMET, SLAMF7, PSMA, mesothelin, MICA, MICB, TRAILR1, TRAILR2, TROP2, MAGE-A3, B7.1, B7.2, CTLA4, PD1, 5T4, GPNMB, FR-alpha, PAPP-A, FLT3, GPC3, CXCR4, ROR1, ROR2, HLA-E, PD-L1, VLA4, CD44, CD13, CD15, CD47, CLL1, CD81, CD23, CD79a, CD79b, CD80, CRLF2, SLAMF7, CD138, CA125, NaPi2b, Nectin4, ADAM8, ADAM9, SLC44A4, CA19-9, LILRBI, LILRB2, LILRB3, LILRB4, LILRB5, ULRA 1, LILRA2, LILRA3, ULRA4, LILRA5, and ULRA6, CCR8, CD7, CTLA4, CX3CR1, ENTPD1, HAVCR2, IL-1R2, PDCD1LG2, TIGIT, TNFRSF4, TNFRSF8, TNFRSF9, GEM, NT5E, TNFRSF18, MUC1, P-cadherin, Plexin-A1, TNFRSF10B, STEAP1, CDCP1, PTK7, Axl, erbB-3, EDNRB, Tyrp1, CD14, CD163, CSF3R, Siglec-9, ITGAM, VISTA, B7-H4 (VTCN1), CCR1, LRRC25, PTAFR, SIRPB1, TLR2, TLR4, CD300LB, ATP1A3, CCR5, MUC1 (or MUC1-C), Plexin-A1, TNFRSF10B, STEAP1, CDCP1, PTK7, AXL, EDNRB, OLR1, and TYRP1.
46. A protein according to any one of embodiments 41-45, wherein the third antigen-binding site comprises a heavy chain variable domain comprising an amino acid sequence at least 90% identical to a sequence selected from SEQ ID NO:94, SEQ ID NO:1, SEQ ID NO:44, SEQ ID NO:52, SEQ ID NO:60, SEQ ID NO:62, SEQ ID NO:70, SEQ ID NO:78, SEQ ID NO:86, SEQ ID NO:102, SEQ ID NO:322, SEQ ID NO:325, SEQ ID NO:328, SEQ ID NO:331, SEQ ID NO:334, and SEQ ID NO:337.
47. A protein according to embodiment 46, wherein the third antigen-binding site comprises a heavy chain variable domain comprising an amino acid sequence at least 90% identical to SEQ ID NO:94 and a light chain variable domain comprising an amino acid sequence at least 90% identical to SEQ ID NO:98.
48. A protein according to embodiment 46, wherein the third antigen-binding site comprises a heavy chain variable domain comprising an amino acid sequence at least 90% identical to SEQ ID NO:322 and a light chain variable domain comprising an amino acid sequence at least 90% identical to SEQ ID NO:98.
49. A protein according to embodiment 46, wherein the third antigen-binding site comprises a heavy chain variable domain comprising an amino acid sequence at least 90% identical to SEQ ID NO:325 and a light chain variable domain comprising an amino acid sequence at least 90% identical to SEQ ID NO:98.
50. A protein according to embodiment 46, wherein the third antigen-binding site comprises a heavy chain variable domain comprising an amino acid sequence at least 90% identical to SEQ ID NO:328 and a light chain variable domain comprising an amino acid sequence at least 90% identical to SEQ ID NO:98.
51. A protein according to embodiment 46, wherein the third antigen-binding site comprises a heavy chain variable domain comprising an amino acid sequence at least 90% identical to SEQ ID NO:331 and a light chain variable domain comprising an amino acid sequence at least 90% identical to SEQ ID NO:98.
52. A protein according to embodiment 46, wherein the third antigen-binding site comprises a heavy chain variable domain comprising an amino acid sequence at least 90% identical to SEQ ID NO:334 and a light chain variable domain comprising an amino acid sequence at least 90% identical to SEQ ID NO:98.
53. A protein according to embodiment 46, wherein the third antigen-binding site comprises a heavy chain variable domain comprising an amino acid sequence at least 90% identical to SEQ ID NO:337 and a light chain variable domain comprising an amino acid sequence at least 90% identical to SEQ ID NO:98.
54. A protein according to embodiment 46, wherein the third antigen-binding site comprises a heavy chain variable domain comprising an amino acid sequence at least 90% identical to SEQ ID NO:44 and a light chain variable domain comprising an amino acid sequence at least 90% identical to SEQ ID NO:48.
55. A protein according to embodiment 46, wherein the third antigen-binding site comprises a heavy chain variable domain comprising an amino acid sequence at least 90% identical to SEQ ID NO:52 and a light chain variable domain comprising an amino acid sequence at least 90% identical to SEQ ID NO:56.
56. A protein according to embodiment 46, wherein the third antigen-binding site comprises a heavy chain variable domain comprising an amino acid sequence at least 90% identical to SEQ ID NO:60 and a light chain variable domain comprising an amino acid sequence at least 90% identical to SEQ ID NO:61.
57. A protein according to embodiment 46, wherein the third antigen-binding site comprises a heavy chain variable domain comprising an amino acid sequence at least 90% identical to SEQ ID NO:62 and a light chain variable domain comprising an amino acid sequence at least 90% identical to SEQ ID NO:66.
58. A protein according to embodiment 46, wherein the third antigen-binding site comprises a heavy chain variable domain comprising an amino acid sequence at least 90% identical to SEQ ID NO:70 and a light chain variable domain comprising an amino acid sequence at least 90% identical to SEQ ID NO:74.
59. A protein according to embodiment 46, wherein the third antigen-binding site comprises a heavy chain variable domain comprising an amino acid sequence at least 90% identical to SEQ ID NO:78 and a light chain variable domain comprising an amino acid sequence at least 90% identical to SEQ ID NO:82.
60. A protein according to embodiment 46, wherein the third antigen-binding site comprises a heavy chain variable domain comprising an amino acid sequence at least 90% identical to SEQ ID NO:86 and a light chain variable domain comprising an amino acid sequence at least 90% identical to SEQ ID NO:90.
61. A protein according to embodiment 46, wherein the third antigen-binding site comprises a heavy chain variable domain comprising an amino acid sequence at least 90% identical to SEQ ID NO:102 and a light chain variable domain comprising an amino acid sequence at least 90% identical to SEQ ID NO:106.
62. A protein according to any one of embodiments 41-45, wherein the third antigen-binding site comprises a heavy chain variable domain comprising an amino acid sequence at least 90% identical to SEQ ID NO:110 and a light chain variable domain comprising an amino acid sequence at least 90% identical to SEQ ID NO:111.
63. A protein according to any one of embodiments 41-45, wherein the third antigen-binding site comprises a heavy chain variable domain comprising an amino acid sequence at least 90% identical to SEQ ID NO:112 and a light chain variable domain comprising an amino acid sequence at least 90% identical to SEQ ID NO:113.
64. A protein according to any one of embodiments 41-63, wherein the antibody constant region or the portion thereof sufficient to bind CD16 comprises hinge and CH2 domain of a human IgG1 antibody.
65. A protein according to embodiment 64, wherein the antibody constant region or the portion thereof sufficient to bind CD16 comprises an amino acid sequence at least 90% identical to amino acids 234-332 of a human IgG1 antibody.
66. A protein according to embodiment 65, wherein the antibody constant region or the portion thereof sufficient to bind CD16 comprises an amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.
67. A protein comprising:
   (a) an antigen-binding site that binds NKG2D;
   (b) an antigen-binding T cell receptor (TCR) fragment; and
   (c) an antibody constant region or a portion thereof sufficient to bind CD16, or an additional antigen-binding site that binds CD16.
68. The protein of embodiment 67, wherein the antigen-binding site is an Fab fragment, and the antigen-binding TCR fragment is a single-chain TCR (scTCR) fragment.
69. The protein of embodiment 68, wherein the scTCR fragment is linked to a polypeptide chain of the antibody constant region via a hinge comprising Ala-Ser.
70. The protein of embodiment 67, wherein the antigen-binding site is an scFv, and the antigen-binding TCR fragment is an extracellular TCR fragment.
71. The protein of embodiment 70, wherein the scFv is linked to a polypeptide chain of the antibody constant region via a hinge comprising Ala-Ser.
72. The protein of embodiment 69 or 71, wherein the hinge further comprises amino acid sequence Thr-Lys-Gly.
73. The protein of embodiment 67, wherein the antigen-binding site is an Fab fragment, and the antigen-binding TCR fragment is an extracellular TCR fragment.
74. The protein of embodiment 67, further comprising an additional antigen-binding TCR fragment that binds the same antigen as the antigen-binding TCR fragment.
75. The protein of embodiment 74, wherein the antigen-binding site is an scFv, and the antigen-binding TCR fragment and the additional antigen-binding TCR fragment are extracellular TCR fragments.
76. The protein of embodiment 74, wherein the antigen-binding site is an scFv, and the antigen-binding TCR fragment and the additional antigen-binding TCR fragment are scTCR fragments.
77. The protein of any one of embodiments 70-72 and 75-76, wherein the scFv comprises a heavy chain variable domain linked to a light chain variable domain via a flexible linker.
78. The protein of embodiment 77, wherein the flexible linker comprises (G₄S)₄.
79. The protein of any one of embodiments 70-72 and 75-78, wherein the scFv comprises a heavy chain variable domain positioned at the N-terminus or the C-terminus of a light chain variable domain.
80. The protein of any one of embodiments 67-79, wherein the antigen-binding site comprises a heavy chain variable domain and a light chain variable domain, and wherein the heavy chain variable domain forms a disulfide bridge with the light chain variable domain.
81. The protein of embodiment 80, wherein the disulfide bridge is formed between Cys at position 44 in the heavy chain variable domain and Cys at position 100 in the light chain variable domain, the positions defined under the Kabat numbering.
82. The protein of any one of embodiments 67-81, wherein the antigen-binding site binds to NKG2D in humans.
83. A protein according to any one of embodiments 67-82, wherein the antigen-binding site comprises a heavy chain variable domain at least 90% identical to an amino acid sequence selected from: SEQ ID NO:94, SEQ ID NO:1, SEQ ID NO:44, SEQ ID NO:52, SEQ ID NO:60, SEQ ID NO:62, SEQ ID NO:70, SEQ ID NO:78, SEQ ID NO:86, SEQ ID NO:102, SEQ ID NO:322, SEQ ID NO:325, SEQ ID NO:328, SEQ ID NO:331, SEQ ID NO:334, and SEQ ID NO:337.
84. A protein according to embodiment 83, wherein the antigen-binding site comprises a heavy chain variable domain comprising an amino acid sequence at least 90% identical to SEQ ID NO:94 and a light chain variable domain comprising an amino acid sequence at least 90% identical to SEQ ID NO:98.
85. A protein according to embodiment 83, wherein the antigen-binding site comprises a heavy chain variable domain comprising an amino acid sequence at least 90% identical to SEQ ID NO:322 and a light chain variable domain comprising an amino acid sequence at least 90% identical to SEQ ID NO:98.
86. A protein according to embodiment 83, wherein the antigen-binding site comprises a heavy chain variable domain comprising an amino acid sequence at least 90% identical to SEQ ID NO:325 and a light chain variable domain comprising an amino acid sequence at least 90% identical to SEQ ID NO:98.
87. A protein according to embodiment 83, wherein the antigen-binding site comprises a heavy chain variable domain comprising an amino acid sequence at least 90% identical to SEQ ID NO:328 and a light chain variable domain comprising an amino acid sequence at least 90% identical to SEQ ID NO:98.
88. A protein according to embodiment 83, wherein the antigen-binding site comprises a heavy chain variable domain comprising an amino acid sequence at least 90% identical to SEQ ID NO:331 and a light chain variable domain comprising an amino acid sequence at least 90% identical to SEQ ID NO:98.
89. A protein according to embodiment 83, wherein the antigen-binding site comprises a heavy chain variable domain comprising an amino acid sequence at least 90% identical to SEQ ID NO:334 and a light chain variable domain comprising an amino acid sequence at least 90% identical to SEQ ID NO:98.
90. A protein according to embodiment 83, wherein the antigen-binding site comprises a heavy chain variable domain comprising an amino acid sequence at least 90% identical to SEQ ID NO:337 and a light chain variable domain comprising an amino acid sequence at least 90% identical to SEQ ID NO:98.
91. A protein according to embodiment 83, wherein the antigen-binding site comprises a heavy chain variable domain comprising an amino acid sequence at least 90% identical to SEQ ID NO:44 and a light chain variable domain comprising an amino acid sequence at least 90% identical to SEQ ID NO:48.
92. A protein according to embodiment 83, wherein the antigen-binding site comprises a heavy chain variable domain comprising an amino acid sequence at least 90% identical to SEQ ID NO:52 and a light chain variable domain comprising an amino acid sequence at least 90% identical to SEQ ID NO:56.
93. A protein according to embodiment 83, wherein the antigen-binding site comprises a heavy chain variable domain comprising an amino acid sequence at least 90% identical to SEQ ID NO:60 and a light chain variable domain comprising an amino acid sequence at least 90% identical to SEQ ID NO:61.
94. A protein according to embodiment 83, wherein the antigen-binding site comprises a heavy chain variable domain comprising an amino acid sequence at least 90% identical to SEQ ID NO:62 and a light chain variable domain comprising an amino acid sequence at least 90% identical to SEQ ID NO:66.
95. A protein according to embodiment 83, wherein the antigen-binding site comprises a heavy chain variable domain comprising an amino acid sequence at least 90% identical to SEQ ID NO:70 and a light chain variable domain comprising an amino acid sequence at least 90% identical to SEQ ID NO:74.
96. A protein according to embodiment 83, wherein the antigen-binding site comprises a heavy chain variable domain comprising an amino acid sequence at least 90% identical to SEQ ID NO:78 and a light chain variable domain comprising an amino acid sequence at least 90% identical to SEQ ID NO:82.
97. A protein according to embodiment 83, wherein the antigen-binding site comprises a heavy chain variable domain comprising an amino acid sequence at least 90% identical to SEQ ID NO:86 and a light chain variable domain comprising an amino acid sequence at least 90% identical to SEQ ID NO:90.
98. A protein according to embodiment 83, wherein the antigen-binding site comprises a heavy chain variable domain comprising an amino acid sequence at least 90% identical to SEQ ID NO:102 and a light chain variable domain comprising an amino acid sequence at least 90% identical to SEQ ID NO:106.
99. A protein according to any one of embodiments 67-82, wherein the antigen-binding site comprises a heavy chain variable domain comprising an amino acid sequence at least 90% identical to SEQ ID NO:110 and a light chain variable domain comprising an amino acid sequence at least 90% identical to SEQ ID NO:111.
100. A protein according to any one of embodiments 67-82, wherein the antigen-binding site comprises a heavy chain variable domain comprising an amino acid sequence at least 90% identical to SEQ ID NO:112 and a light chain variable domain comprising an amino acid sequence at least 90% identical to SEQ ID NO:113.
101. A protein according to any one of embodiments 67-100, wherein the antigen-binding TCR fragment binds a peptide from a tumor-associated antigen presented by a major histocompatibility complex (MHC).
102. The protein of embodiment 101, wherein the antigen-binding TCR fragment binds an ELAVL4 peptide having the amino acid sequence of SEQ ID NO:425 presented by HLA-A*02:01:48, and wherein the antigen-binding TCR fragment comprises an alpha chain variable domain at least 90% identical to SEQ ID NO:351 and a beta chain variable domain at least 90% identical to SEQ ID NO:352.
103. The protein of embodiment 101, wherein the antigen-binding TCR fragment binds an Insulin peptide having the amino acid sequence of SEQ ID NO:426 presented by HLA-A*02:01:48, and wherein the antigen-binding TCR fragment comprises an alpha chain variable domain at least 90% identical to SEQ ID NO:357 and a beta chain variable domain at least 90% identical to SEQ ID NO:358.
104. The protein of embodiment 101, wherein the antigen-binding TCR fragment binds a TERT peptide having the amino acid sequence of SEQ ID NO:340 presented by HLA-A*02:01:48, and wherein the antigen-binding TCR fragment comprises an alpha chain variable domain at least 90% identical to SEQ ID NO:363 and a beta chain variable domain at least 90% identical to SEQ ID NO:364.
105. The protein of embodiment 101, wherein the antigen-binding TCR fragment binds an ERBB2 peptide having the amino acid sequence of SEQ ID NO:341 presented by HLA-A*02, and wherein the antigen-binding TCR fragment comprises an alpha chain variable domain at least 90% identical to SEQ ID NO:430 and a beta chain variable domain at least 90% identical to SEQ ID NO:431.
106. The protein of embodiment 101, wherein the antigen-binding TCR fragment binds a WT1 peptide having the amino acid sequence of SEQ ID NO:342 presented by HLA-A*02, and wherein the antigen-binding TCR fragment comprises an alpha chain variable domain at least 90% identical to SEQ ID NO:434 and a beta chain variable domain at least 90% identical to SEQ ID NO:435.
107. The protein of embodiment 101, wherein the antigen-binding TCR fragment binds a WT1 peptide having the amino acid sequence of SEQ ID NO:342 presented by HLA-A*02, and wherein the antigen-binding TCR fragment comprises an alpha chain variable domain at least 90% identical to SEQ ID NO:438 and a beta chain variable domain at least 90% identical to SEQ ID NO:439.
108. The protein of embodiment 101, wherein the antigen-binding TCR fragment binds a MAGE-A3 peptide having the amino acid sequence of SEQ ID NO:343 presented by HLA-A1, and wherein the antigen-binding TCR fragment comprises an alpha chain variable domain at least 90% identical to SEQ ID NO:375 and a beta chain variable domain at least 90% identical to SEQ ID NO:376.
109. The protein of embodiment 101, wherein the antigen-binding TCR fragment binds a MART1 peptide having the amino acid sequence of SEQ ID NO:344 presented by HLA-A2, and wherein the antigen-binding TCR fragment comprises an alpha chain variable domain at least 90% identical to SEQ ID NO:381 and a beta chain variable domain at least 90% identical to SEQ ID NO:382.
110. The protein of embodiment 101, wherein the antigen-binding TCR fragment binds a BIRC5 peptide having the amino acid sequence of SEQ ID NO:346 presented by HLA-A2, and wherein the antigen-binding TCR fragment comprises an alpha chain variable domain at least 90% identical to SEQ ID NO:442 and a beta chain variable domain at least 90% identical to SEQ ID NO:443.
111. The protein of embodiment 101, wherein the antigen-binding TCR fragment binds a BIRC5 peptide having the amino acid sequence of SEQ ID NO:346 presented by HLA-A2, and wherein the antigen-binding TCR fragment comprises an alpha chain variable domain at least 90% identical to SEQ ID NO:444 and a beta chain variable domain at least 90% identical to SEQ ID NO:445.
112. The protein of embodiment 101, wherein the antigen-binding TCR fragment binds a PRAME peptide having the amino acid sequence of SEQ ID NO:347 presented by HLA-A2, and wherein the antigen-binding TCR fragment comprises an alpha chain variable domain at least 90% identical to SEQ ID NO:395 and a beta chain variable domain at least 90% identical to SEQ ID NO:396.
113. The protein of embodiment 101, wherein the antigen-binding TCR fragment binds a PRAME peptide having the amino acid sequence of SEQ ID NO:347 presented by HLA-A2, and wherein the antigen-binding TCR fragment comprises an alpha chain variable domain at least 90% identical to SEQ ID NO:401 and a beta chain variable domain at least 90% identical to SEQ ID NO:402.
114. The protein of embodiment 101, wherein the antigen-binding TCR fragment binds a PRAME peptide having the amino acid sequence of SEQ ID NO:347 presented by HLA-A2, and wherein the antigen-binding TCR fragment comprises an alpha chain variable domain at least 90% identical to SEQ ID NO:407 and a beta chain variable domain at least 90% identical to SEQ ID NO:408.
115. The protein of embodiment 101, wherein the antigen-binding TCR fragment binds an NY-ESO-1 peptide having the amino acid sequence of SEQ ID NO:348 presented by HLA-A2, and wherein the antigen-binding TCR fragment comprises an alpha chain variable domain at least 90% identical to SEQ ID NO:413 and a beta chain variable domain at least 90% identical to SEQ ID NO:414.
116. The protein of embodiment 101, wherein the antigen-binding TCR fragment binds an NY-ESO-1 peptide having the amino acid sequence of SEQ ID NO:348 presented by HLA-A2, and wherein the antigen-binding TCR fragment comprises an alpha chain variable domain at least 90% identical to SEQ ID NO:418 and a beta chain variable domain at least 90% identical to SEQ ID NO:414.
117. The protein of embodiment 101, wherein the antigen-binding TCR fragment binds an NY-ESO-1 peptide having the amino acid sequence of SEQ ID NO:348 presented by HLA-A2, and wherein the antigen-binding TCR fragment comprises an alpha chain variable domain at least 90% identical to SEQ ID NO:421 and a beta chain variable domain at least 90% identical to SEQ ID NO:422.
118. The protein of embodiment 101, wherein the antigen-binding TCR fragment binds an SSX2 peptide having the amino acid sequence of SEQ ID NO:345 presented by HLA-A2.
119. The protein of any one of embodiments 67-118, wherein the antibody constant region or the portion thereof sufficient to bind CD16 comprises hinge and CH2 domain of a human IgG1 antibody.
120. The protein of embodiment 119, wherein the antibody constant region or the portion thereof sufficient to bind CD16 comprises an amino acid sequence at least 90% identical to amino acids 234-332 of a human IgG1 antibody.
121. The protein of embodiment 120, wherein the antibody constant region or the portion thereof sufficient to bind CD16 comprises an amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.
122. A formulation comprising a protein according to any one of embodiments 33-121 and a pharmaceutically acceptable carrier.
123. A cell comprising one or more nucleic acids encoding a protein according to any one of embodiments 33-121.
124. A method of enhancing tumor cell death, the method comprising exposing a tumor and natural killer cells to the protein according to any one of embodiments 33-121.
125. A method of treating cancer, wherein the method comprises administering the protein according to any one of embodiments 33-121 or the formulation according to embodiment 122 to a patient.
126. The method of embodiment 125, wherein the cancer is selected from the group consisting of acute myeloid leukemia, acute myelomonocytic leukemia, B cell lymphoma, bladder cancer, breast cancer, colorectal cancer, diffuse large B cell lymphoma esophageal cancer, Ewing's sarcoma, follicular lymphoma, gastric cancer, gastrointestinal cancer, gastrointestinal stromal tumors, glioblastoma, head and neck cancer, melanoma, mesothelioma, multiple myeloma, myelodysplastic syndrome, renal cell carcinoma, neuroblastoma, non-small cell lung cancer, neuroendocrine tumors, ovarian cancer, and pancreatic cancer, prostate cancer, sarcomas, small cell lung cancer, T cell lymphoma, testis cancer, thymic carcinoma, thyroid cancer, urothelial cancer, cancers infiltrated by myeloid-derived suppressor cells, cancers with extracellular matrix deposition, cancers with high levels of reactive stroma, and cancers with neoangiogenesis.

## Claims

1. A protein comprising:
(a) a first antigen-binding site that binds a tumor-associated antigen;
(b) a second antigen-binding site that binds the same tumor-associated antigen as the first antigen-binding site;
(c) a third antigen-binding site that binds NKG2D; and
(d) an antibody constant region or a portion thereof sufficient to bind CD16.

2. The protein according to claim 1, wherein:
(i) the first antigen-binding site is an scFv or Fab;
(ii) the second antigen-binding site is an scFv or Fab; and
(iii) the third antigen-binding site is an scFv, wherein the scFv comprises a heavy chain variable domain and a light chain variable domain.

3. The protein according to claim 2, wherein within the scFv of the third antigen-binding site, a disulfide bridge is formed between the heavy chain variable domain and the light chain variable domain, optionally wherein the disulfide bridge forms between C44 from the heavy chain variable domain and C100 from the light chain variable domain, the positions defined under the Kabat numbering.

4. The protein according to claim 2 or 3, wherein within the scFv of the third antigen-binding site, the heavy chain variable domain is linked to the light chain variable domain via a flexible linker, optionally wherein the flexible linker comprises (G₄S)₄.

5. The protein according to any one of claims 2-4, wherein within the scFv of the third antigen-binding site, the heavy chain variable domain is positioned at the C-terminus of the light chain variable domain.

6. The protein according to any one of claims 1-5, wherein the first and second antigen-binding site are each a Fab.

7. The protein according to any one of claims 1-6, wherein the antibody constant region or a portion thereof sufficient to bind CD16 comprises a first antibody constant domain and a second antibody constant domain, wherein the first antibody constant domain and the second antibody constant domain each comprise a hinge and a CH2 domain of a human IgG1 antibody, optionally wherein the first antibody constant domain and the second antibody constant domain each comprise an amino acid sequence at least 90% identical to amino acids 234-332 of a human IgG1 antibody, numbered according to the EU index as in Kabat.

8. The protein according to claim 7, wherein the first antibody constant domain and the second antibody constant domain comprise heterodimerization mutations.

9. The protein according to claim 8, wherein the heterodimerization mutations are located at one or more positions selected from the group consisting of Q347, Y349, L351, S354, E356, E357, K360, Q362, S364, T366, L368, K370, N390, K392, T394, D399, S400, D401, F405, Y407, K409, T411, and K439, numbered according to the EU index as in Kabat, optionally wherein the heterodimerization mutations comprise one or more substitutions selected from the group consisting of Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T3661, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E, numbered according to the EU index as in Kabat.

10. The protein according to claim 9, wherein the heterodimerization mutations comprise Q347R, D399V and F405T substitutions in the first antibody constant domain and K360E and K409W substitutions in the second antibody constant domain, numbered according to the EU index as in Kabat.

11. The protein according to any one of claims 1-10, wherein the third antigen-binding site that binds NKG2D comprises:
(a) a heavy chain variable domain comprising an amino acid sequence at least 90% identical to SEQ ID NO:94 and a light chain variable domain comprising an amino acid sequence at least 90% identical to SEQ ID NO:98;
(b) a heavy chain variable domain comprising an amino acid sequence at least 90% identical to SEQ ID NO:322 and a light chain variable domain comprising an amino acid sequence at least 90% identical to SEQ ID NO:98;
(c) a heavy chain variable domain comprising an amino acid sequence at least 90% identical to SEQ ID NO:325 and a light chain variable domain comprising an amino acid sequence at least 90% identical to SEQ ID NO:98;
(d) a heavy chain variable domain comprising an amino acid sequence at least 90% identical to SEQ ID NO:328 and a light chain variable domain comprising an amino acid sequence at least 90% identical to SEQ ID NO:98;
(e) a heavy chain variable domain comprising an amino acid sequence at least 90% identical to SEQ ID NO:331 and a light chain variable domain comprising an amino acid sequence at least 90% identical to SEQ ID NO:98; or
(f) a heavy chain variable domain comprising an amino acid sequence at least 90% identical to SEQ ID NO:334 and a light chain variable domain comprising an amino acid sequence at least 90% identical to SEQ ID NO:98.

12. The protein according to any one of claims 1-11, wherein the third-antigen binding site that binds NKG2D comprises:
(a) a heavy chain complementarity-determining region 1 (CDR1) sequence comprising the amino acid sequence of SEQ ID NO: 319, a heavy chain complementarity-determining region 2 (CDR2) sequence comprising the amino acid sequence of SEQ ID NO: 96, and a heavy chain complementarity-determining region 3 (CDR3) sequence comprising the amino acid sequence of SEQ ID NO: 320; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO: 101;
(b) a heavy chain CDR1 sequence comprising the amino acid sequence of SEQ ID NO: 319, a heavy chain CDR2 sequence comprising the amino acid sequence of SEQ ID NO: 96, and a heavy chain CDR3 sequence comprising the amino acid sequence of SEQ ID NO: 324; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO: 101;
(c) a heavy chain CDR1 sequence comprising the amino acid sequence of SEQ ID NO: 319, a heavy chain CDR2 sequence comprising the amino acid sequence of SEQ ID NO: 96, and a heavy chain CDR3 sequence comprising the amino acid sequence of SEQ ID NO: 327; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO: 101;
(d) a heavy chain CDR1 sequence comprising the amino acid sequence of SEQ ID NO: 319, a heavy chain CDR2 sequence comprising the amino acid sequence of SEQ ID NO:96, and a heavy chain CDR3 sequence comprising the amino acid sequence of SEQ ID NO: 330; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO 101;
(e) a heavy chain CDR1 sequence comprising the amino acid sequence of SEQ ID NO: 319, a heavy chain CDR2 sequence comprising the amino acid sequence of SEQ ID NO: 96, and a heavy chain CDR3 sequence comprising the amino acid sequence of SEQ ID NO: 333; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO: 101; or
(f) a heavy chain CDR1 sequence comprising the amino acid sequence of SEQ ID NO: 319, a heavy chain CDR2 sequence comprising the amino acid sequence of SEQ ID NO: 96, and a heavy chain CDR3 sequence comprising the amino acid sequence of SEQ ID NO: 336; and a light chain CDR1 sequence comprising the amino acid sequence of SEQ ID NO: 99, a light chain CDR2 sequence comprising the amino acid sequence of SEQ ID NO: 100, and a light chain CDR3 sequence comprising the amino acid sequences of SEQ ID NO: 101.

13. A pharmaceutical composition comprising a protein according to any one of claims 1-12 and a pharmaceutically acceptable carrier.

14. A cell comprising one or more nucleic acids encoding a protein according to any one of claims 1-12.

15. The protein according to any one of claims 1-11 or a pharmaceutical composition according to claim 12 for use in a method of treating cancer, wherein the method comprises administering the protein or pharmaceutical composition to a patient in need thereof, optionally wherein the cancer is selected from the group consisting of acute myeloid leukemia, acute myelomonocytic leukemia, B cell lymphoma, bladder cancer, breast cancer, colorectal cancer, diffuse large B cell lymphoma esophageal cancer, Ewing's sarcoma, follicular lymphoma, gastric cancer, gastrointestinal cancer, gastrointestinal stromal tumor, glioblastoma, head and neck cancer, melanoma, mesothelioma, multiple myeloma, myelodysplastic syndrome, renal cell carcinoma, neuroblastoma, non-small cell lung cancer, neuroendocrine tumors, ovarian cancer, pancreatic cancer, prostate cancer, sarcoma, small cell lung cancer, T cell lymphoma, testis cancer, thymic carcinoma, thyroid cancer, urothelial cancer, cancer infiltrated by myeloid-derived suppressor cells, cancer with extracellular matrix deposition, cancer with a high level of reactive stroma, and cancer with neoangiogenesis.
